# EUROPÄISCHE PATENTSCHRIFT

(11) **EP 0 538 194 B1**
(45) Veröffentlichungstag und Bekanntmachung des Hinweises auf die Patenterteilung: **04.06.1997**
(21) Anmeldenummer: 92810769.7
(22) Anmeldetag: 08.10.1992
(51) Int. Cl.: C07H 21/00, A61K 31/70, C07H 19/04, C07H 19/073, C07H 19/173

(54) **Bicyclische Nukleoside, Oligonukleotide, Verfahren zu deren Herstellung und Zwischenprodukte**
Bicyclic nucleosides, oligonucleotides, their method of preparation and intermediates therein
Nucléosides et oligonucléosides bicycliques, leur procédé de préparation et leurs intermédiaires

(30) Priorität: 17.10.1991 CH 3043/91
(43) Veröffentlichungstag der Anmeldung: 21.04.1993
(73) Patentinhaber: Novartis AG, 4058 Basel (CH)
(72) Erfinder: Leumann, Christian, Dr., CH-8053 Zürich (CH)

(56) Entgegenhaltungen:
- WO-A-90/12014
- WO-A-91/04983
- JOURNAL OF ORGANIC CHEMISTRY. Bd. 32, 1967, EASTON US Seiten 3595 - 3603 J.D.FISSEKIS ET AL. 'Synthesis of 5-Hydroxyalkylpyrimidines from Lactones. III. 5-Dihydroxycyclopentylpyrimidines.'

## Beschreibung

Die Erfindung betrifft Nukleoside mit einem Bicyclo-[3,3,0]-8-oxaoctangerüst, ein Verfahren zu deren Herstellung durch die Substitution einer anomeren Abgangsgruppe mit einer Nukleinbase aus der Thymin-, Adenin-, Purin- oder Cytosinreihe, Bicyclo-[3,3,0]-1,3,5-trihydroxy-8-oxa-octan und dessen geschützte Derivate als Zwischenprodukte, Oligonukleotide mit diesen Nukleosiden und die Verwendung der Nukleoside zur Herstellung von Oligonukleotiden mit gleichen oder verschiedenen Nukleosideinheiten im Molekül.

Nukleoside und Oligonukleotide haben als antivirale Wirkstoffe und wegen ihrer Fähigkeit zur Wechselwirkung mit Nukleinsäuren und der damit verbundenen biologischen Aktivität breites Interesse gefunden, siehe zum Beispiel E. Uhlmann et al., Chemical Reviews, Vol. 90, Seiten 543 bis 584 (1990). Zur Bereitstellung von Nukleosiden mit neuen Eigenschaften oder zur Verbesserung der Wechselwirkung und Stabilität gegenüber Nukleasen sind die Zuckerreste der Nukleoside, zum Beispiel Furanosen, in unterschiedlicher Weise derivatisiert worden, siehe zum Beispiel V. E. Marquez et al., Medicinal Research Reviews, Vol. 6, Seiten 1-40 (1986). Bicyclische Furanosederivate sind für diesen Zweck noch nicht bekannt geworden.

Ein Gegenstand der Erfindung sind Verbindungen der Formel I in Form ihrer Racemate oder Enantiomeren, 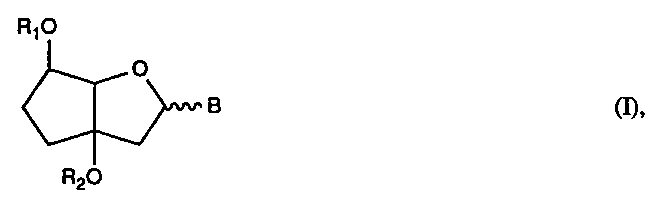 worin R₁ und R₂ unabhängig voneinander für Wasserstoff oder eine Schutzgruppe stehen und B einen Purin- oder Pyrimidinrest oder ein Analoges davon bedeutet.

Schutzgruppen und Verfahren zur Derivatisierung der Hydroxylgruppen mit solchen Schutzgruppen sind in der Zuckerchemie allgemein bekannt. Beispiele für solche Schutzgruppen sind: lineares oder verzweigtes C₁-C₈-, besonders C₁-C₄-Alkyl, zum Beispiel Methyl, Ethyl, n- und i-Propyl, n-, i- und t-Butyl; C₇-C₁₂-Aralkyl, zum Beispiel Benzyl, Methylbenzyl, Dimethylbenzyl, Methoxybenzyl, Dimethoxybenzyl, Brombenzyl; Diphenylmethyl, Di(methylphenyl)methyl, Di(dimethylphenyl)methyl, Di(methoxyphenyl)methyl, Di(dimethoxyphenyl)methyl, Trityl, Tri(methylphenyl)methyl, Tri(dimethylphenyl)methyl, Tri(methoxyphenyl)methyl, Tri(dimethoxyphenyl)methyl; Triphenylsilyl, Alkyldiphenylsilyl, Dialkylphenylsilyl und Trialkylsilyl mit 1 bis 20, bevorzugt 1 bis 12 und besonders bevorzugt 1 bis 8 C-Atomen in den Alkylgruppen, zum Beispiel Trimethylsilyl, Triethylsilyl, Tri-n-propylsilyl, i-Propyl-dimethylsilyl, t-Butyl-dimethylsilyl, t-Butyl-diphenylsilyl, n-Octyl-dimethylsilyl, (1,1,2,2-Tetramethylethyl)-dimethylsilyl; C₂-C₁₂-, besonders C₂-C₈-Acyl, wie zum Beispiel Acetyl, Propanoyl, Butanoyl, Pentanoyl, Hexanoyl, Benzoyl, Methylbenzoyl, Methoxybenzoyl, Chlorbenzoyl und Brombenzoyl; R₃-SO₂-, worin R₃ C₁-C₁₂-Alkyl, besonders C₁-C₆-Alkyl, C₅- oder C₆-Cycloalkyl, Phenyl, Benzyl, C₁-C₁₂- und besonders C₁-C₄-Alkylphenyl, oder C₁-C₁₂- und besonders C₁-C₄-Alkylbenzyl, oder Halogenphenyl oder Halogenbenzyl bedeutet, zum Beispiel Methyl-, Ethyl-, Propyl-, Butyl-, Phenyl-, Benzyl-, p-Brom-, p-Methoxy- und p-Methylphenylsulfonyl; C₁-C₁₂-, bevorzugt C₁-C₈-Alkoxycarbonyl, zum Beispiel Methoxy-, Ethoxy-, n- oder i-Propoxy- oder n-, i- oder t-Butoxycarbonyl, oder Phenyloxycarbonyl, Benzyloxycarbonyl, Methyl- oder Methoxy- oder Chlorphenyloxycarbonyl oder -benzyloxycarbonyl. Bei R₁ und R₂ in Formel I kann es sich um gleiche oder verschiedene Schutzgruppen handeln, wobei im allgemeinen gleiche Schutzgruppen bevorzugt sind.

In einer bevorzugten Ausführungsform sind die Verbindungen der Formel I solche, worin R₁ und R₂ unabhängig voneinander lineares oder verzweigtes C₁-C₄-Alkyl, C₇-C₁₂-Aralkyl, Trialkylsilyl mit 1 bis 12 C-Atomen in den Alkylgruppen, C₂-C₈-Acyl, R₃-SO₂-, worin R₃ C₁-C₆-Alkyl, Phenyl, Benzyl, C₁-C₄-Alkylphenyl, C₁-C₄-Alkylbenzyl, oder Halogenphenyl oder Halogenbenzyl bedeutet, oder C₁-C₈-Alkoxycarbonyl, Phenoxycarbonyl oder Benzyloxycarbonyl darstellen.

In einer besonders bevorzugten Ausführungsform stellen R₁ und R₂ Methyl, Ethyl, n- und i-Propyl, n-, und t-Butyl; Benzyl, Methylbenzyl, Dimethylbenzyl, Methoxybenzyl, Dimethoxybenzyl, Brombenzyl; Diphenylmethyl, Di(methylphenyl)methyl, Di(dimethylphenyl)methyl, Di(methoxyphenyl)methyl, Di(dimethoxyphenyl)methyl, Trityl, Tri(methylphenyl)methyl, Tri(dimethylphenyl)methyl, Tri(methoxyphenyl)methyl, Tri(dimethoxyphenyl)methyl; Trimethylsilyl, Triethylsilyl, Tri-n-propylsilyl, i-Propyl-dimethylsilyl, t-Butyl Butyl-dimethylsilyl, t-Butyldiphenylsilyl, n-Octyl-dimethylsilyl, (1,1,2,2-Tetramethylethyl)-dimethylsilyl; Acetyl, Propanoyl, Butanoyl, Pentanoyl, Hexanoyl, Benzoyl, Methylbenzoyl, Methoxybenzoyl, Chlorbenzoyl und Brombenzoyl; Methyl-, Ethyl-, Propyl-, Butyl-, Phenyl-, Benzyl-, p-Brom-, p-Methoxy- und p-Methylphenylsulfonyl; Methoxy-, Ethoxy-, n- oder i-Propoxy- oder n-, i- oder t-Butoxycarbonyl, oder Phenyloxycarbonyl, Benzyloxycarbonyl, Methyl- oder Methoxy- oder Chlorphenyloxycarbonyl oder -benzyloxycarbonyl dar.

Wenn B einen Purinrest oder ein Analoges davon darstellt, so kann es sich um Reste der Formeln II, IIa, IIb, IIc, IId oder IIe handeln, 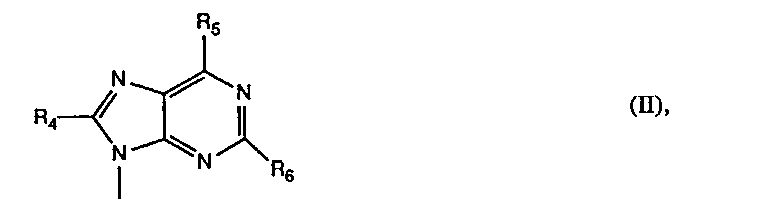 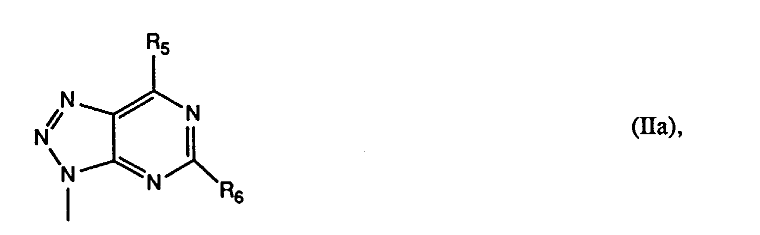 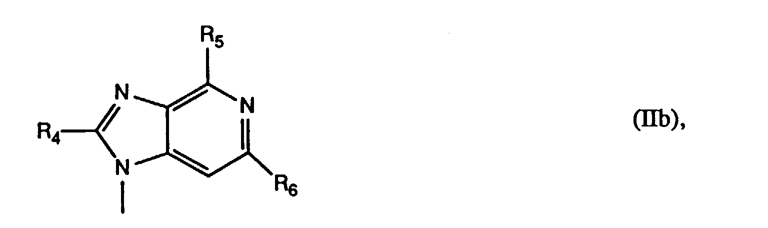 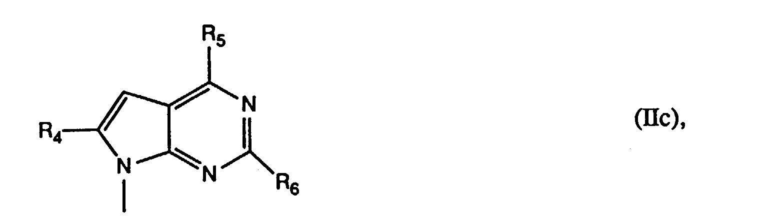 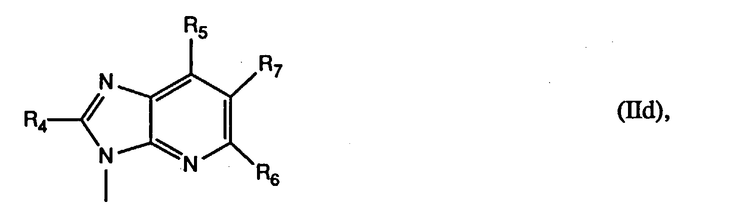 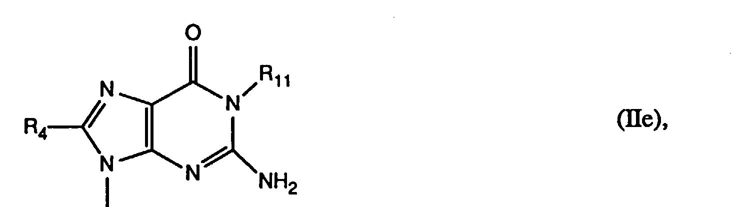 worin R₄ für H, Cl, Br oder OH steht, und R₅, R₆ und R₇ unabhängig voneinander H, OH, SH, NH₂, NHNH₂, NHOH, NHOAlkyl mit 1 bis 12 C-Atomen, F, Cl, Br, Alkyl oder Hydroxyalkyl oder Aminoalkyl oder Alkoxy oder Alkylthio mit 1 bis 12 C-Atomen, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, und R₁₁ H oder C₁-C₄-Alkyl darstellt.

Geeignete Schutzgruppen sind zuvor erwähnt worden. Bevorzugte Schutzgruppen sind C₁-C₈-Acylgruppen, wie zum Beispiel Acetyl, Propionyl, Butyroyl und Benzoyl. R₁₁ steht bevorzugt für H oder Methyl.

Das Primäramino enthält bevorzugt 1 bis 12 und besonders bevorzugt 1 bis 6 C-Atome, und das Sekundäramino bevorzugt 2 bis 12 und besonders bevorzugt 2 bis 6 C-Atome.

Einige Beispiele für Alkyl, Alkoxy, Alkylthio, Hydroxyalkyl und Aminoalkyl, die bevorzugt 1 bis 6 C-Atome enthalten, sind Methyl, Ethyl und die Isomeren von Propyl, Butyl, Pentyl, Hexyl, Heptyl, Octyl, Nonyl, Decyl, Undecyl und Dodecyl, sowie entsprechende Alkoxy-, Alkylthio-, Hydroxyalkyl- und Aminoalkylreste. Das Alkyl, Alkoxy, Alkylthio, Hydroxyalkyl und Aminoalkyl enthält besonders bevorzugt 1 bis 4 C-Atome. Bevorzugte Alkyl-, Alkoxy-, Alkylthio-, Hydroxyalkyl- und Aminoalkylreste sind Methyl, Ethyl, n- und i-Propyl, n-, i- und t-Butyl, Methoxy, Ethoxy, Methylthio und Ethylthio, Aminomethyl, Aminoethyl, Hydroxymethyl und Hydroxyethyl.

Bei dem Primäramino und Sekundäramino kann es sich zum Beispiel um Reste der Formel R₈R₉N handeln, worin R₈ für H steht oder unabhängig die Bedeutung von R₉ hat, und R₉ C₁-C₂₀-, bevorzugt C₁-C₁₂- und besonders bevorzugt C₁-C₆-Alkyl, -Aminoalkyl, -Hydroxyalkyl; Carboxyalkyl oder Carbalkoxyalkyl, wobei die Carbalkoxygruppe 2 bis 8 C-Atome enthält und die Alkylgruppe 1 bis 6, bevorzugt 1 bis 4 C-Atome; C₂-C₂₀-, bevorzugt C₂-C₁₂- und besonders bevorzugt C₂-C₆-Alkenyl; Phenyl, Mono- oder Di-(C₁-C₄-Alkyl- oder -Alkoxy)phenyl, Benzyl, Mono- oder Di-(C₁-C₄-Alkyl- oder -Alkoxy)benzyl; oder 1,2-, 1,3- oder 1,4-Imidazolyl-C₁-C₆-Alkyl darstellt, oder R₈ und R₉ zusammen Tetra- oder Pentamethylen, 3-Oxa-1,5-pentylen, -CH₂-NR₁₀-CH₂CH₂- oder -CH₂CH₂-NR₁₀-CH₂CH₂- darstellen, worin R₁₀ für H oder C₁-C₄-Alkyl steht. Die Aminogruppe im Aminoalkyl kann mit ein oder zwei C₁-C₄-Alkyl oder -Hydroxyalkylgruppen substituiert sein. Die Hydroxylgruppe im Hydroxyalkyl kann mit C₁-C₄-Alkyl verethert sein.

Beispiele für Alkyl sind zuvor angegeben worden. Beispiele für Aminoalkyl sind Aminomethyl, Aminoethyl, 1-Aminoprop-2-yl oder -3-yl, 1-Amino-but-2-yl oder -3-yl oder -4-yl, N-Methyl- oder N,N-Dimethyl- oder N-Ethyl- oder N,N-Diethyl- oder N-2-Hydroxyethyl- oder N,N-Di-2-hydroxyethylaminomethyl oder -aminoethyl oder -aminopropyl oder -aminobutyl. Beispiele für Hydroxyalkyl sind Hydroxymethyl, 1-Hydroxy-eth-2-yl, 1-Hydroxy-prop-2- oder -3-yl, 1-Hydroxy-but-2-yl, -3-yl oder -4-yl. Beispiele für Carboxyalkyl sind Carboxymethyl, Carboxyethyl, Carboxypropyl und Carboxybutyl, und Beispiele für Carbalkoxyalkyl sind diese mit Methyl oder Ethyl veresterten Carboxyalkylgruppen. Beispiele für Alkenyl sind Allyl, But-1-en-3-yl oder -4-yl, Pent-3- oder 4-en-1-yl oder -2-yl, Hex-3- oder -4- oder -5-en-1-yl oder -2-yl. Beispiele für Alkyl- und Alkoxyphenyl beziehungsweise -benzyl sind Methylphenyl, Dimethylphenyl, Ethylphenyl, Diethylphenyl, Methylbenzyl, Dimethylbenzyl, Ethylbenzyl, Diethylbenzyl, Methoxyphenyl, Dimethoxyphenyl, Ethoxyphenyl, Diethoxyphenyl, Methoxybenzyl, Dimethoxybenzyl, Ethoxybenzyl, Diethoxybenzyl. Beispiele für Imidazolylalkyl, in dem die Alkylgruppe bevorzugt 2 bis 4 C-Atome enthält, sind 1,2-, 1,3- oder 1,4-Imidazolylethyl oder -n-propyl oder -n-butyl. R₁₀ stellt bevorzugt H, Methyl oder Ethyl dar.

Bevorzugte Beispiele für Primäramino und Sekundäramino sind Methyl-, Ethyl-, Dimethyl-, Diethyl-, Allyl-, Mono- oder Di-(1-hydroxy-eth-2-yl)-, Phenyl- und Benzylamino, Acetylamino und Benzoylamino.

In einer bevorzugten Ausführungsform stellt R₄ Wasserstoff dar. In einer anderen bevorzugten Ausführungsform stellt R₇ Wasserstoff dar. In einer weiteren bevorzugten Ausführungsform bedeuten R₅ und R₆ unabhängig voneinander H, F, Cl, Br, OH, SH, NH₂, NHOH, NHNH₂, Methylamino, Dimethylamino, Benzoylamino, Methoxy, Ethoxy und Methylthio.

Einige Beispiele für Analoge der Purinreihe sind neben Purin Adenin, N-Methyladenin, N-Benzyladenin, 2-Methyladenin, 2-Methylthioadenin, 2-Aminoadenin, 3-Carbaadenin, 7-Carbaadenin, 1-Carbaadenin, 6-Hydroxypurin, 2-Amino-6-chlorpurin, 2-Amino-6-methylthiopurin, 2-Amino-6-hydroxypurin, 3-Carba-6-chlorpurin, Guanin, 2-Methylguanin. Besonders bevorzugt sind Adenin, 2-Aminoadenin und Guanin.

Wenn B in Formel I einen analogen Pyrimidinrest darstellt, so handelt es sich bevorzugt um Uracil-, Thymin- und Cytosinreste der Formeln III, IIIa und IIIb, 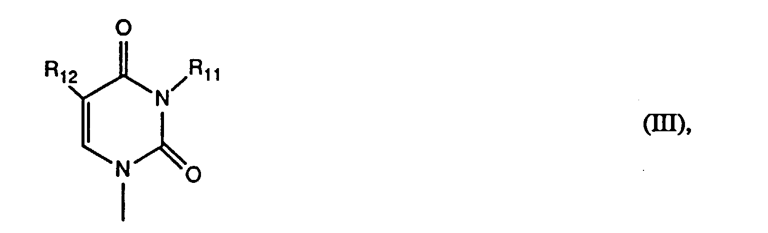 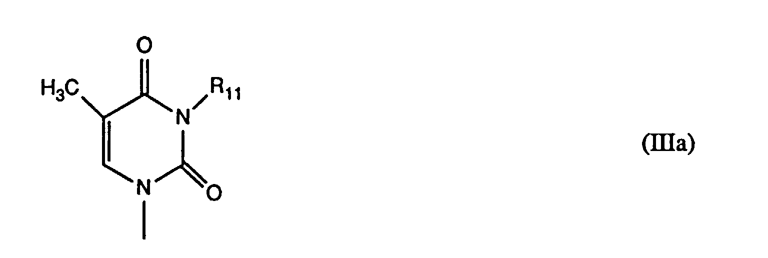 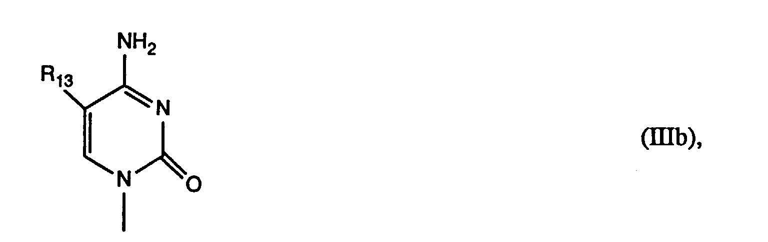 worin R₁₁ H oder C₁-C₄-Alkyl bedeutet, und R₁₂ und R₁₃ unabhängig voneinender die zuvor für R₅ angegebene Bedeutung haben, einschliesslich der Bevorzugungen, und die Wasserstoffatome der NH₂-Gruppe in Formel IIIb mit C₁-C₆-Alkyl oder Benzoyl substituiert sein können, sowie die Dihydroderivate der Reste der Formeln III, IIIa und IIIb. Bevorzugt stellt R₁₂ H, C₁-C₆-Alkyl oder Hydroxyalkyl, F, Cl, Br, NH₂, Benzoylamino, Mono- oder Di-C₁-C₆-alkylamino dar und R₁₃ stellt bevorzugt H, C₁-C₆-Alkyl oder -Alkoxy oder -Hydroxyalkyl, F, Cl, Br, NH₂, Benzoylamino, Mono- oder Di-C₁-C₆-alkylamino dar.

R₁₁ steht bevorzugt für H oder Methyl. R₁₂ bedeutet bevorzugt H, F, Cl, Br, NH₂, NHCH₃, N(CH₃)₂ oder C₁-C₄-Alkyl. R₁₃ stellt bevorzugt H, C₁-C₄-Alkyl, besonders Methyl, oder NH₂, NHCH₃ oder (CH₃)₂N dar.

Einige Beispiele für Pyrimidinanaloge sind Uracil, Thymin, Cytosin, 5-Fluoruracil, 5-Chloruracil, 5-Bromuracil, Dihydrouracil, Pseudouracil, 1-Methylpseudouracil, 5-Methyluracil, 3-Methylcytosin und 5-Methylcytosin.

In einer bevorzugten Ausführungsform entsprechen die Verbindungen der Formel I den α- und β-Anomeren der Formel IV 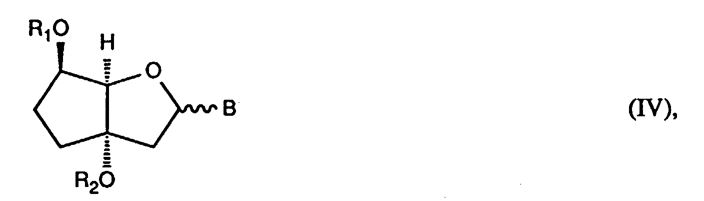 worin R₁, R₂ und B die zuvor angegebenen Bedeutungen haben, einschliesslich der Bevorzugungen.

In einer besonders bevorzugten Ausführungsform entsprechen die Verbindungen der Formel IV den β-Anomeren der Formel (IVa) 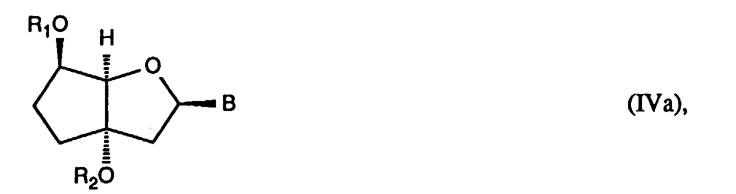 worin R₁, R₂ und B die zuvor angegebenen Bedeutungen haben, einschliesslich der Bevorzugungen.

Ein weiterer Gegenstand der Erfindung ist ein Verfahren zur Herstellung von Verbindungen der Formel I, das dadurch gekennzeichnet ist, dass man eine Verbindung der Formel V in Form ihrer Racemate oder Enantiomeren 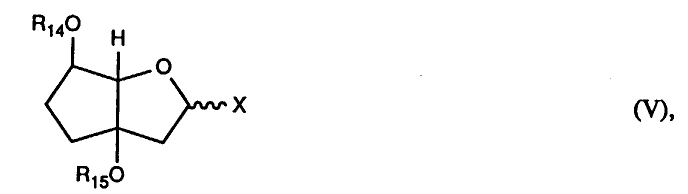 worin R₁₄ und R₁₅ für gleiche oder verschiedene Schutzgruppen stehen und X eine Abgangsgruppe bedeutet, mit einem Purin, Purinanalogen oder Pyrimidinanalogen umsetzt und anschliessend gegebenenfalls die Schutzgruppen entfernt.

Schutzgruppen sind zuvor erwähnt worden. Abgangsgruppen sind in der Zuckerchemie allgemein bekannt. Es kann sich zum Beispiel um Halogen, besonders F, Cl, Br oder I, um C₁-C₆- und bevorzugt C₁-C₄-Alkoxy, besonders Methoxy oder Ethoxy, um C₁-C₈-Acyloxy wie zum Beispiel Acetyloxy, Propionyloxy, Butyroyloxy, Mono- oder Di- oder Trichloracetyloxy oder -fluoracetyloxy, Benzoyloxy und Chlorbenzoyloxy, oder um R₁₆SO₃, worin R₁₆ C₁-C₆-Alkyl oder -Halogenalkyl, oder um unsubstituiertes oder mit ein bis drei Halogen (F, Cl und Br), C₁-C₄-Alkyl oder -Alkoxy substituiertes Phenyl oder Benzyl handeln. Beispiele für Halogenalkyl sind Mono- oder Di- oder Trichlormethyl oder -fluormethyl, 1,1,1-Trichlor- oder -Trifluormethyl und Pentafluorethyl. Beispiele für substituiertes Phenyl und Benzyl sind Methyl-, Dimethyl-, Methoxy-, Dimethoxy-, Mono- und Dichlor- und Mono- und Dibrom- und Mono- und Difluorphenyl und -benzyl. Die Schutz- und Abgangsgruppen können identisch sein, besonders im Fall von Acylresten.

Die Reaktion kann bei Temperaturen von -20 bis 150 °C, bevorzugt 0 bis 100 °C durchgeführt werden.

Im allgemeinen verwendet man ein Lösungsmittel, das bevorzugt aprotisch ist, und noch bevorzugter auch dipolar. Beispiele für Lösungsmittel, die alleine oder als Mischung von mindestens zwei Lösungsmitteln eingesetzt werden können, sind Ether (Dibutylether, Tetrahydrofuran, Dioxan, Diethylenglykoldimethylether, Ethylenglykoldimethyl- oder -di-ethylether, Diethylenglykoldiethylether, Triethylenglykoldimethylether), halogenierte Kohlenwasserstoffe (Methylenchlorid, Chloroform, 1,2-Dichlorethan, 1,1,1-Trichlorethan, 1,1,2,2-Tetrachlorethan), Carbonsäureester und Lactone (Essigsäureethylester, Propionsäuremethylester, Benzoesäureethylester, 2-Methoxyethylacetat, γ-Butyrolacton, δ-Valerolacton, Pivalolacton), Carbonsäureamide und Lactame (N,N-Dimethylformamid, N,N-Diethylformamid, N,N-Dimethylacetamid, Tetramethylharnstoff, Hexamethylphosphorsäuretriamid, γ-Butyrolactam, ε-Caprolactam, N-Methylpyrrolidon, N-Acetylpyrrolidon, N-Methylcaprolactam), Sulfoxide (Dimethylsulfoxid), Sulfone (Dimethylsulfon, Diethylsulfon, Trimethylensulfon, Tetramethylensulfon), tertiäre Amine (Triethylamin, N-Methylpiperidin, N-Methylmorpholin), aromatische Kohlenwasserstoffe wie zum Beispiel Benzol oder substituierte Benzole (Chlorbenzol, o-Dichlorbenzol, 1,2,4-Trichlorbenzol, Nitrobenzol, Toluol, Xylol) und Nitrile (Acetonitril, Propionitril, Benzonitril, Phenylacetonitril), sowie aliphatische oder cycloaliphatische Kohlenwasserstoffe (Pentan, Petrolether, Hexan, Cyclohexan und Methylcyclohexan).

Bevorzugte Lösungsmittel sind halogenierte aliphatische Kohlenwasserstoffe, aromatische Kohlenwasserstoffe, Ether und Nitrile, zum Beispiel Methylenchlorid, Chloroform, Benzol, Toluol, Acetonitril, Diethylether, Dibutylether, Tetrahydrofuran und Dioxan.

Die Reaktion wird bevorzugt in Gegenwart von Mitteln durchgeführt, die die reaktive NH-Gruppe aktivieren. Solche Mittel sind zum Beispiel anorganische Basen (NaOH, KOH, Na₂CO₃, K₂CO₃, NaHCO₃ und KHCO₃ sowie Alkalimetallalkolate wie zum Beispiel NaOCH₃ oder NaOC₄H₉) oder N,O-silylierte Carbonsäureamide [N,O-Bis-(trimethylsilyl)-acetamid], Disilazane (Hexamethyldisilazan), Chlorsilane (Trimethylchlorsilan) und Silyl-trifluormethansulfonate (Trimethylsilyl-trifluormethansulfonat), die alleine oder in Mischung eingesetzt oder zusammen mit Lewissäuren wie zum Beispiel BF₃, SbF₃, SbCl₅, TiCl₄ oder SnCl₄ eingesetzt werden können. Ferner ist es möglich, die reaktive NH-Gruppe mit Hilfe von zum Beispiel Alkalimetallhydriden oder Lithiumallkylverbindungen (zum Beispiel LiH, NaH, KH, LiCH₃ oder LiC₄H₉) zu metallieren.

Im einzelnen kann die Reaktion so durchgeführt werden, dass man eine Base B in einem Lösungsmittel vorlegt, die aktivierenden Mittel zugibt, und dann das Gemisch mit einer Verbindung der Formel V versetzt und danach ausreagieren lässt. Die Aufarbeitung des Reaktionsgemisches zur Isolierung der Verbindungen der Formel I erfolgt dann in an sich bekannter Weise, wobei zur Herstellung von reinen Isomeren, besonders der α- und β-Anomeren, chromatographische Methoden angewendet werden können. Die Enantiomeren, zum Beispiel solche der Formel IV, sind erhältlich durch die Verwendung entsprechender Enantiomerer der Formel V. Bei der Reaktion wird in der Regel ein Gemisch der α- und β-Anomeren erhalten.

Die Verbindungen der Formel V sind neu und stellen zusammen mit den Vorprodukten, worin R₁₄ und R₁₅ für H stehen und X OH bedeutet, einen weiteren Gegenstand der Erfindung dar. Ein weiterer Gegenstand der Erfindung sind somit Verbindungen der Formeln V und Va in Form ihrer Racemate oder Enantiomeren, 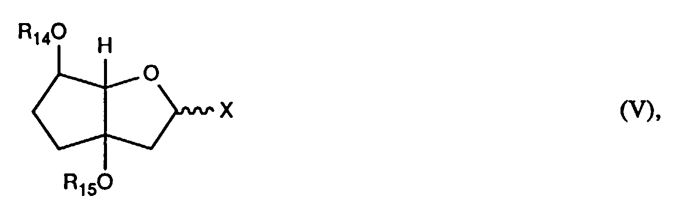 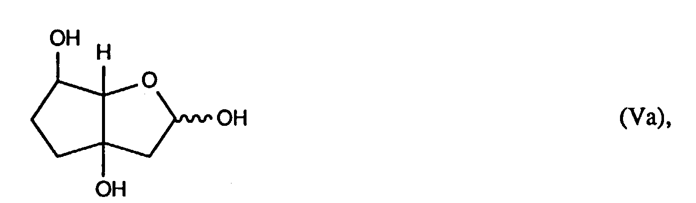 worin R₁₄ und R₁₅ für gleiche oder verschiedene Schutzgruppen stehen und X eine Abgangsgruppe bedeutet. Schutz- und Abgangsgruppen sind zuvor erwähnt worden. Eine bevorzugte Abgangsgruppe ist CH₃C(O)O.

In einer bevorzugten Ausführungsform entsprechen die Verbindungen der Formeln V und Va den Enantiomeren der Formeln VI und VIa 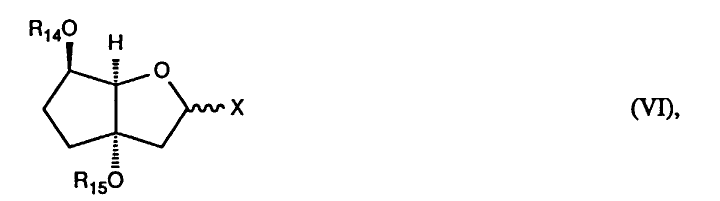 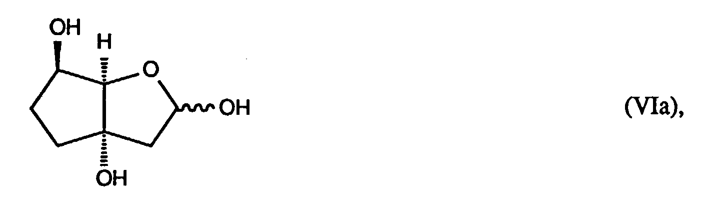 worin R₁₄, R₁₅ und X die zuvor angegebenen Bedeutungen haben.

Die Verbindungen der Formeln V, Va, VI und VIa können zum Beispiel nach dem folgenden neuen Verfahren hergestellt werden, das einen weiteren Gegenstand der Erfindung darstellt.

Man setzt das von F. G. Cocu et al. in Helv. Chim. Acta 55, Seite 2838 (1972) beschriebene (±)- Cis-2,3-(2',2'-lsopropyldioxolyl)-cyclopentan-1-on der Formel A 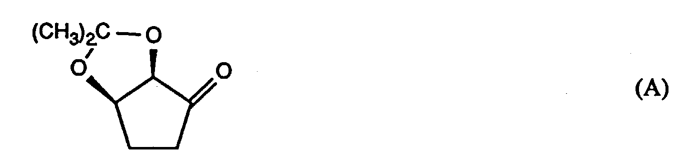 in einem Lösungsmittel wie zum Beispiel Tetrahydrofuran in Gegenwart von etwa 2 Äquivalenten Azabicycloalkenen (zum Beispiel 1,5,7-Triaza-bicyclo-[4,4,0]-dec-5-en) bei Raumtemperatur mit einem β-Alkoxycarbonylethylphosphonsäureester (zum Beispiel β-Ethoxycarbonylethylphosphonsäurediethylester) zu einem (±)-Cis-2,3-(2',2'-Isopropyldioxolyl)-cyclopent-5-en umsetzt, zum Beispiel der Formel B

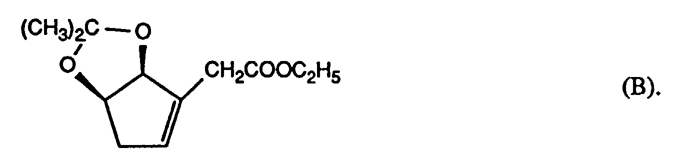

Das Cyclopentenderivat wird dann epoxidiert, zum Beispiel mit Persäuren oder H₂O₂, zweckmässig bei Raumtemperatur oder unter Eiskühlung in einem halogenierten Kohlenwasserstoff als Lösungsmittel. Hierbei wird überwiegend das entsprechende (±)-Exoepoxid des Cyclopentenderivats gebildet, zum Beispiel der Formel C 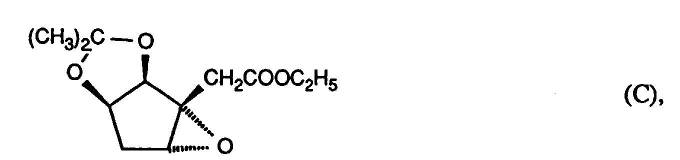 das nur geringe Anteile des Endoepoxids enthält, welches leicht abgetrennt werden kann.

Das (±)-Exoepoxid kann direkt durch Hydrierung der Carbonsäureestergruppe zur primären Alkoholgruppe und gleichzeitiger Hydrierung der Epoxidgruppe weiterverarbeitet werden, wobei man Racemate erhält. Das Racemat des Exoepoxids kann aber auch vor der Hydrierung in die (+)- und (-)-Enantiomeren aufgetrennt werden. So sind neben den Nukleosidracematen auch (-)-Enantiomere der natürlichen und (+)-Enantiomere der unnatürlichen Reihe zugänglich. Die Hydrierung wird hierbei in an sich bekannter Weise durchgeführt, zum Beispiel mit LiH, NaH, AlH₃, BH₃, LiBH₄, LiAlH₄, Di-n-butylaluminiumhydrid oder auch katalytisch mit Wasserstoff.

Die Trennung des Racemats gelingt in einfacher Weise und in hohen Ausbeuten durch eine partielle Hydrolyse der Carbonsäurestergruppe mit Alkalimetallhydroxiden, zum Beispiel NaOH, in Gegenwart von Schweineleberesterase (EC 3.1.1.1) und NaH₂PO₄-Puffer in wässriger Phase. Hierbei wird ein Gemisch aus einer Carbonsäure der Formel D 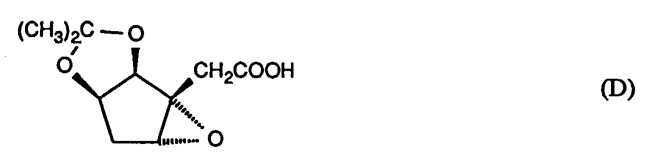 beziehungsweise eines Alkalimetallsalzes davon und eines Carbonsäureesters zum Beispiel der Formel E 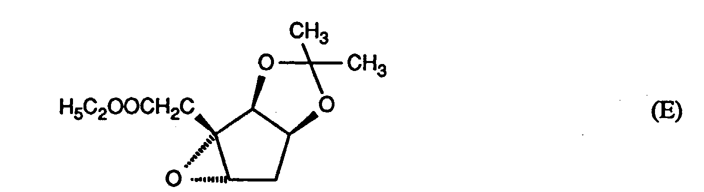 gebildet, aus dem der Ester durch Extraktion mit einem organischen Lösungsmittel, zum Beispiel Diethylether, leicht abgetrennt werden kann.

Die bei der Hydrierung erhaltenen Racemate beziehungsweise die (-)-Enantiomeren (3S,4R,5S-Konfiguration) der Formel F und (+)-Enantiomeren (3R,4S,5S-Konfiguration) der Formel G, 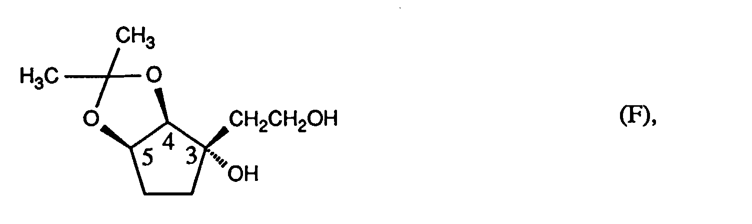 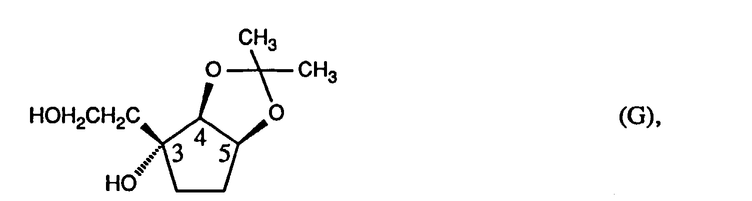 werden mit spezifischen Oxidationsmitteln, zum Beispiel 1,1,1-Triacetyloxy-1,1-dihydro-1,2-benzjodoxol-3(1H)-on (Dess-Martin-Reagenz), zu den Aldehyden der Formeln H oder J oder des Racemats oxidiert, 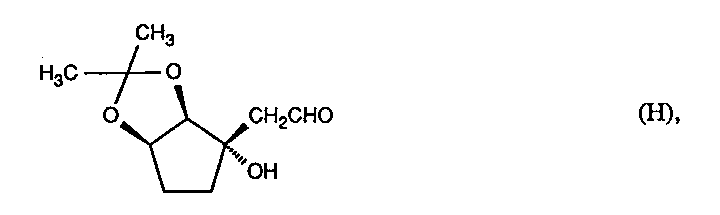 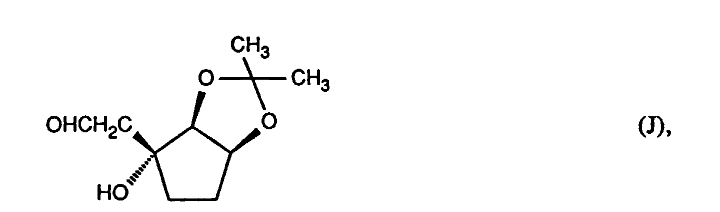 die in Gegenwart von wässrigen Säuren oder sauren Ionenaustauschern in Gegenwart von Wasser zu den Verbindungen der Formeln Va oder VIa hydrolysiert und cyclisiert werden.

Somit ist ein weiterer Gegenstand der vorliegenden Erfindung ein Verfahren zur Herstellung einer Verbindung der Formel Va oder der Formel Vla, dadurch gekennzeichnet, dass eine Verbindung der Formel H oder der Formel J oder das Racemat 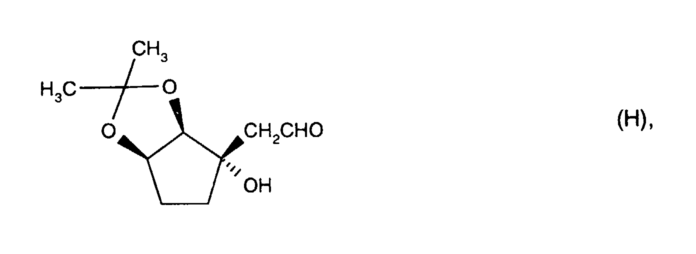 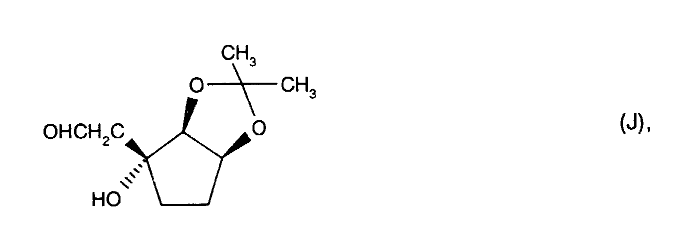 in Gegenwart von wässrigen Säuren oder sauren lonenaustauschern in Gegenwart von Wasser hydrolysiert und cyclisiert wird.

Die Verbindungen der Formeln Va und Vla können nach in der Zuckerchemie allgemein bekannten Methoden in die Verbindungen der Formeln V oder VI übergeführt werden, indem man zunächst die anomere Hydroxylgruppe durch eine Abgangsgruppe X ersetzt und dann die Schutzgruppen R₁₄ und R₁₅ einführt. Wenn die Abgangsgruppe und die Schutzgruppen identisch sind, kann die Einführung der Gruppen in einem Verfahrensschritt erfolgen.

Somit ist ein weiterer Gegenstand der vorliegenden Erfindung ein Verfahren zur Herstellung einer Verbindung der Formel V oder der Formel VI, dadurch gekennzeichnet, dass bei einer Verbindung der Formel Va oder der Formel Vla die anomere Hydroxylgruppe durch eine Abgangsgruppe X ersetzt und die Schutzgruppen R₁₄ und R₁₅ eingeführt werden.

Die Verbindungen der Formeln V und VI können zum Beispiel mittels chromatographischer Methoden in die α- und β-Isomeren getrennt werden. Da bei der Umsetzung mit den Basen B wieder Gemische der α- und β-Formen gebildet werden, ist es zweckmässig, diese Trennung erst mit den Verbindungen der Formel I beziehungsweise IV durchzuführen. Die Trennbarkeit kann durch Entschützen und die Einführung anderer Schutzgruppen verbessert werden.

Die Verbindungen der Formeln I, IV oder IVa werden vor deren Weiterverarbeitung beziehungsweise zu deren Verwendung als pharmazeutische Wirkstoffe in bekannter Weise entschützt, wobei man Verbindungen erhält, in denen R₁ und R₂ Wasserstoff bedeutet. Aus diesen Verbindungen können Oligonukleotide aufgebaut werden, die auf Grund ihrer Wechselwirkung mit Nukleinsäuren wertvolle biologische Aktivitäten aufweisen, und als pharmazeutische Wirkstoffe oder als Diagnostika verwendet werden können.

Ein weiterer Gegenstand der Erfindung ist die Verwendung der Verbindungen der Formel I in Form von Racematen oder Enantiomeren zur Herstellung von Oligonukleotiden, die gleiche oder verschiedene Monomereinheiten von Verbindungen der Formel I oder Mono-. mereinheiten von anderen Nukleosiden enthalten, wobei die Oligonukleotide 2 bis 200 Monomereinheiten enthalten. Bevorzugt enthalten die Oligonukleotide 2 bis 100, besonders bevorzugt 2 bis 50, und insbesondere bevorzugt 2 bis 20 Monomereinheiten. Bevorzugt sind gleiche oder verschiedene und besonders gleiche Monomereinheiten von Verbindungen der Formel I.

Somit ist ein weiterer Gegenstand der vorliegenden Erfindung ein Verfahren zur Herstellung eines erfindungsgemässen Oligonukleotides der Formeln VII, VIIa oder VIIb, dadurch gekennzeichnet, dass eine erfindungsgemässe Verbindung der Formel I, IV oder IVa bei der Oligonukleotidsynthese eingesetzt wird. Bevorzugt weist das Oligonukleotid dabei 2 bis 100 Monomereinheiten, besonders bevorzugt 2 bis 50 Monomereinheiten, und insbesondere 2 bis 20 Monomereinheiten auf.

Ein weiterer Gegenstand der Erfindung sind Oligonukleotide der Formel VII 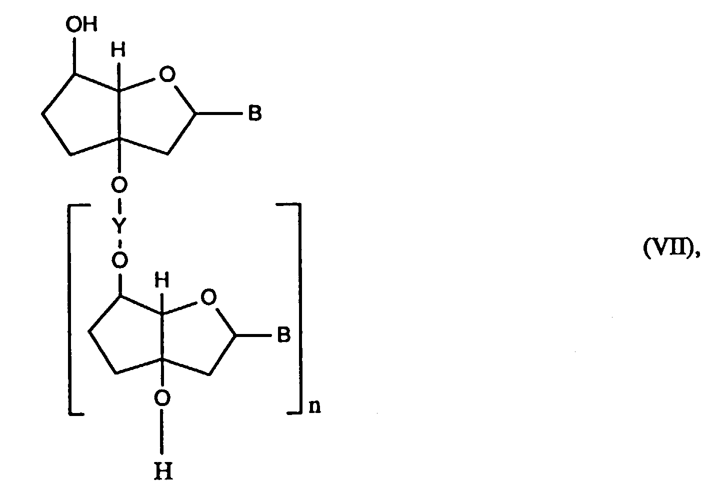 worin B einen Purin- oder Pyrimidinrest oder ein Analoges davon bedeuten, n für eine Zahl von 2 bis 200 steht und Y für eine Nukleotid-Brückengruppe steht. Für B gelten die zuvor für Verbindungen der Formel I angegebenen Bevorzugungen und Beispiele. Eine bevorzugte Brückengruppe ist die in natürlichen Oligonukleotiden vorkommende Gruppe -P(O)O^{⊖}-. Beispiele für weitere Brückengruppen sind -P(O)S^{⊖} -, -P(S)S^{⊖}-, -P(O)R₁₇-, -P(O)OR₁₈-, P(O)NR₁₉R₂₀, -CO- oder -CON(R₁₈)₂-, worin R₁₇ H oder C₁-C₆-Alkyl darstellt und R₁₈ C₁-C₆-Alkyl bedeutet und R₁₉ und R₂₀ unabhängig voneinander die Bedeutung von R₁₇ haben. In Formel VII steht n bevorzugt für eine Zahl von 2 bis 100, besonders bevorzugt für eine Zahl von 2 bis 50 und insbesondere bevorzugt für eine Zahl von 2 bis 20.

In einer bevorzugten Ausführungsform entsprechen die erfindungsgemässen Oligonukleotide der Formel VIIa 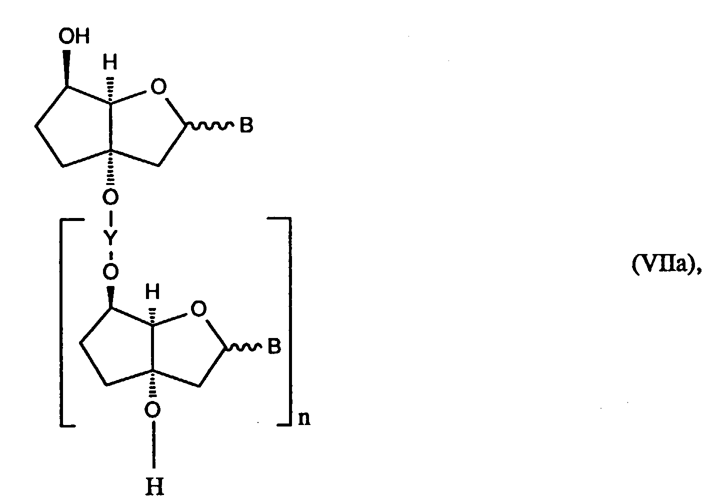 worin B, Y und n die zuvor angegebenen Bedeutungen haben, einschliesslich der Bevorzugungen.
In einer besonders bevorzugten Ausführungsform entsprechen die erfindungsgemässen Oligonukleotide der Formel VIIb 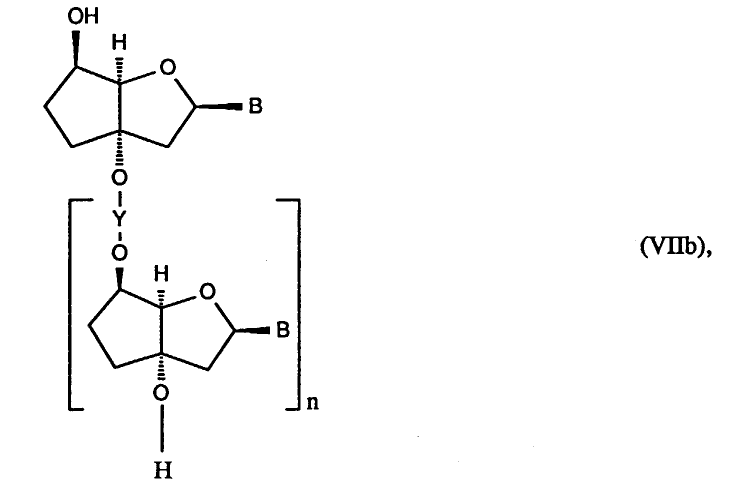 worin B, Y und n die zuvor angegebenen Bedeutungen haben, einschliesslich der Bevorzugungen. Besonders bevorzugt sind solche Oligonukleotide der Formel VIIb, worin B 9-Adenyl und n 10 bedeuten, oder B Cytosyl und n 6 darstellt, oder B für Thymidyl und n für 10 stehen.

Die Herstellung der erfindungsgemässen Oligonukleotide kann in an sich bekannter Weise nach verschiedenen Verfahren in gegebenenfalls automatisierten und zusammen mit Verfahrensvorschriften käuflichen DNA-Synthesizern erfolgen. Im Falle der Brückengruppe -P(O)O^{⊖}- kann zum Beispiel das Phosphortriesterverfahren, das Phosphittriesterverfahren oder das H-Phosphonatverfahren angewendet werden, die dem Fachmann geläufig sind. Beim Phosphittriesterverfahren kann man zum Beispiel so vorgehen, dass man die Nukleoside der Formel I, worin R₁ und R₂ je H bedeuten, in Form Ihrer Racemate oder Enantiomeren mit einem Schutzgruppenreagenz, zum Beispiel 4,4'-Dimethoxytriphenylmethyl-trifluormethylsulfonat (abgekürzt DMT-Triflat) zu einem Nukleosid der Formel K 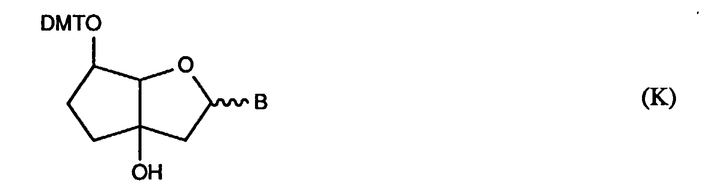 umsetzt, und die Verbindung der Formel K mit Hilfe eines "linkers", zum Beispiel Bernsteinsäureanhydrid, an ein festes Trägermaterial bindet, zum Beispiel an Controlled Pore Glass (CPG), das langkettige Alkylaminogruppen enthält. In einem seperaten Verfahren wird die Hydroxylgruppe der Verbindung der Formel K derivatisiert, zum Beispiel zu einem Phosphoramidit unter Verwendung von [R'O(i-Propyl₂N)]PCl zu einer Verbindung der Formel L 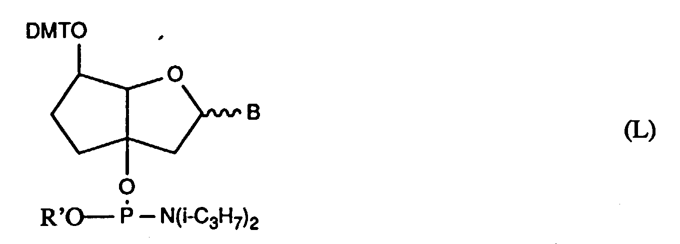 umsetzt, wobei R' zum Beispiel Allyl oder β-Cyanoethyl darstellt.

Nach dem Abspalten der Schutzgruppe des an den Träger gebundenen Materials koppelt man unter Abspaltung von -N(i-C₃H₇) mit der Verbindung der Formel I, blockiert eventuell vorhandene freie Hydroxylgruppen (capping) und oxidiert dann das gebildete Phosphit zum Phosphat. Nach dem Entschützen des Dimeren wiederholt man den Reaktionszyklus mit der Verbindung L, bis man ein Oligomer mit der gewünschten Anzahl an Monomereinheiten synthetisiert hat, und löst das Produkt vom Trägermaterial ab.

Die erfindungsgemässen Verbindungen der Formel I, worin R₁ und R₂ je H bedeuten, weisen antivirale und antiproliferative Eigenschaften auf und können demgemäss als Arzneimittel Verwendung finden. Die erfindungsgemässen Oligonukleotide weisen eine erhöhte Stabilität gegenüber einem Abbau durch Nukleasen (zum Beispiel Enzyme) auf. Ferner wird eine sehr gute Paarung mit Nukleinsäuren, besonders doppelsträngigen Nukleinsäuren unter Ausbildung von stabilen Trippelhelices beobachtet. Die erfindungsgemässen Oligonukleotide eignen sich daher besonders für die Antisense-Technologie zur Inaktivierung von Nukleosidsequenzen in Nukleinsäuren (siehe EP-A-0 266 099, WO 87/07300 und WO 89/08146). Sie können zur Behandlung von Infektionen und Krankheiten zum Beispiel durch die Blockierung von bioaktiven Proteinen auf der Stufe der Nukleinsäuren (zum Beispiel Onkogene) eingesetzt werden. Die erfindungsgemässen Oligonukleotide eignen sich auch als Diagnostika und können als Gensonden zum Nachweis von viralen Infektionen oder genetisch bedingten Krankheiten durch selektive Interaktion auf der Stufe von einzel- oder doppelsträngigen Nukleinsäuren verwendet werden ("gene probes"). Im besonderen - bedingt durch die erhöhte Stabilität gegenüber Nukleasen - ist eine diagnostische Anwendung nicht nur *in vitro* sondern auch *in vivo* (zum Beispiel Gewebeproben, Blutplasma und Blutserum) möglich. Solche Verwendungsmöglichkeiten sind zum Beispiel in der WO 91/06556 beschrieben.

Ein weiterer Gegenstand der Erfindung betrifft die Verwendung der erfindungsgemässen Oligonukleotide als Diagnostika zum Nachweis von viralen Infektionen oder genetisch bedingten Krankheiten.

Ein anderer Gegenstand der Erfindung betrifft auch die erfindungsgemässen Nukleoside der Formeln I, IV oder VIa oder der Oligonukleotide der Formeln VII, VIIa oder VIIb zur Anwendung in einem therapeutischen Verfahren zur Behandlung von Krankheiten bei Warmblütern einschliesslich des Menschen durch Inaktivierung von Nukleotidsequenzen im Körper. Die Dosierung bei Verabreichung an Warmblüter von etwa 70 kg Körpergewicht kann zum Beispiel 0,01 bis 1000 mg pro Tag betragen. Die Verabreichung erfolgt vorzugsweise in Form pharmazeutischer Präparate parenteral, zum Beispiel intravenös oder intraperitoneal.

Ein weiterer Gegenstand der Erfindung betrifft ein pharmazeutisches Präparat, enthaltend eine wirksame Menge eines Nukleosids der Formeln I, IV oder VIa oder eines Oligonukleotids der Formeln VII, VIIa oder VIIb alleine oder zusammen mit anderen Wirkstoffen, ein pharmazeutisches Trägermaterial vorzugsweise in einer signifikanten Menge und gegebenenfalls Hilfsstoffe.

Man kann die pharmakologisch wirksamen erfindungsgemässen Nukleoside und Oligonukleotide in Form von parenteral verabreichbaren Präparaten oder von Infusionslösungen verwenden. Solche Lösungen sind vorzugsweise isotonische wässrige Lösungen oder Suspensionen, wobei diese zum Beispiel bei lyophilisierten Präparaten, welche die Wirksubstanz allein oder zusammen mit einem Trägermaterial, zum Beispiel Mannit, enthalten, vor Gebrauch hergestellt werden können. Die pharmazeutischen Präparate können sterilisiert sein und/oder Hilfsstoffe, zum Beispiel Konservier- Stabilisier-, Netz- und/oder Emulgiermittel, Löslichkeitsvermittler, Salze zur Regulierung des osmotischen Drucks und/oder Puffer enthalten. Die pharmazeutischen Präparate, die gewünschtenfalls weitere pharmakologisch wirksame Stoffe wie zum Beispiel Antibiotka enthalten können, werden in an sich bekannter Weise, zum Beispiel mittels konventioneller Lösungs- oder Lyophilisierungsverfahren, hergestellt, und enthalten etwa 0,1 % bis 90 %, insbesondere von etwa 0,5 % bis etwa 30 %, zum Beispiel 1 % bis 5 % Aktivstoff(e).

Somit ist ein weiterer Gegenstand der vorliegenden Erfindung ein Verfahren zur Herstellung eines erfindungsgemässen pharmazeutischen Präparates, dadurch gekennzeichnet, dass man eine wirksame Menge eines erfindungsgemässen Nukleosids der Formeln I, IV oder IVa oder eines erfindungsgemässen Oligonukleotids der Formeln VII, VIIa oder VIIb, alleine oder zusammen mit anderen Wirkstoffen, mit einem pharmazeutischen Trägermaterial und gegebenenfalls Hilfsstoffen versetzt.

Die nachfolgenden Beispiele erläutern die Erfindung. Den ¹H-NMR-Spektren liegt die Nummerierung der Kohlenstoffatome in den folgenden cyclischen Kohlenstoffgerüsten zu Grunde:

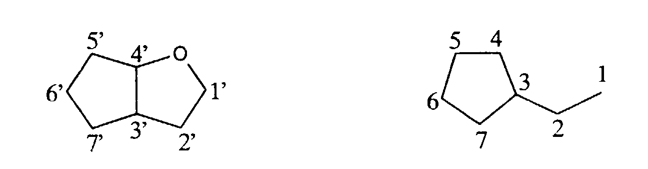

### A) Herstellung von Zwischenprodukten

### Beispiel A1:

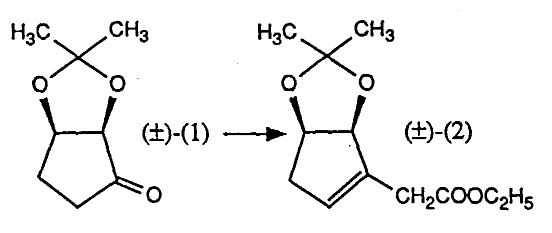

Zu einer Lösung von 1,87 g (13,5 mmol) 1,5,7-Triaza-bicyclo[4.4.0]dec-5-en in 20 ml Methylenchlorid werden bei Raumtemperatur unter Stickstoffatmosphäre 1,4 ml (7 mmol) Triethylphosphonoacetat und 1,0 g (6,4 mmol) der Verbindung (1) gelöst in 20 ml Methylenchlorid, thylenchlorid, gegeben. Nach 3 Stunden wird das Reaktionsgemisch mit 70 ml verdünnt und mit je 100 ml gesättigter Natriumchloridlösung und Wasser gewaschen. Die wässrige Phase wird zweimal mit je 100 ml Methylenchlorid extrahiert. Die vereinigten organischen Phasen werden über MgSO₄ getrocknet, filtriert und im Vakuum eingedampft. Das erhaltene Oel wird an Silikagel chromatographiert (Hexan/Essigsäureethylester 3:1). Danach wird das Lösungsmittel im Vakuum entfernt und das Oel über Nacht im Hochvakuum getrocknet. Man erhält 1,3 g (90 %) der Verbindung (2) als farbloses Oel.

¹H-NMR (400 MHz, CDCl₃): 1,27 [t, J=7,5 H₃C-CH₂]; 1,36 und 1,38 [2s, 2 CH₃C]; 2,48 [dd, J=1,0, J=17,8, HC(6)] und 2,59 [dd, J=5,4, J=17,8, HC(6)]; 3,17 [d, J=16,3, H₂-C(2)]; 3,22 [d, J=16,3, H₂-C(2)]; 4,15 [q, J=7,15, H₃C-CH₂]; 4,75 [t, J=5,5, HC(5)]; 5,07 [d, J=5,7, HC(4)]; 5,61 [s, breit, HC(7)].

### Beispiel A2:

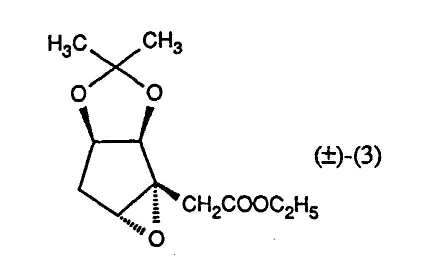

Zu einer Lösung von 4,13 g (18,27 mmol) der Verbindung (2) in 200 ml Methylenchlorid werden bei 0°C 11,5 g (36,55 mmol) 3-Chlorperbenzoesäure, gelöst in 100 ml Methylenchlorid, innerhalb von 10 Minuten zugegeben. Nach 10 Minuten wird das Eisbad entfernt und das Reaktionsgemisch bei Raumtemperatur während 4 Tagen weitergerührt. Die Reaktion wird durch Zugabe von 200 ml 1M wässriger NaHCO₃ gestoppt, die organische Phase abgetrennt und die wässrige Phase zweimal mit je 150 ml Methylenchlorid extrahiert. Die vereinigten organischen Phasen werden über Na₂SO₄ getrocknet, filtriert und im Vakuum eingedampft. Das erhaltene Oel wird an Silikagel chromatographiert (Hexan/Essigsäureethylester 4:1). Nach Entfernung des Lösungsmittels und Trocknen am Hochvakuum erhält man 3,39 g (76 %) der Titelverbindung als farbloses Oel.

¹H-NMR (400 MHz, CDCl₃): 1,28 [t, J=7,14 H₃C-CH₂]; 1,31 und 1,46 [2s, H₃C-CH₂]; 1,99 [dt, Jₜ=2,1, J_{d}=15,4, HC(6)]; 2,28 [dd, J=6,0, J=15,4, HC(6)]; 2,69 [d, J=16,2, HC(2)]; 3,08 [d, J=16,2, HC(2)]; 3,59 [s breit, HC(7)]; 4,18 [q, J=7,14, H₃C-CH₂]; 4,56 [m, (triplettoid), HC(5)]; 4,65 [d, J=6,0, HC(4)].

### Beispiel A3:

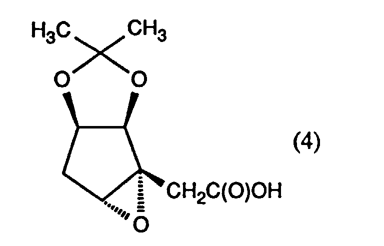

Zu einer stark gerührten Emulsion von 10,1 g (41,3 mmol) der Verbindung (3) in 500 ml 0,1M Phosphat Puffer und 1,1 ml (11 mg Protein) Schweinsleber-Esterase (EC 3.1.1.1; FLUKA AG Buchs) wird 1M NaOH so zugegeben, dass der pH konstant bei 7,75 liegt. Nach Zugabe von 20 ml 1M NaOH (20 mmol) wird mit 2N NaOH auf pH 9,0 eingestellt und der nicht hydrolisierte Ester viermal mit je 250 ml Diethylether extrahiert. Die wässrige Phase wird dann mit 1M HCl auf pH 2,0 gebracht, mit Natriumchlorid gesättigt und sechsmal mit je 250 ml Methylenchlorid extrahiert. Die organische Phase wird über Na₂SO₄ getrocknet, filtriert, im Vakuum eingedampft und am Hochvakuum getrocknet. Man erhält 4,7 g (53 %) der Titelverbindung als farbloses Oel.

¹H-NMR (200 MHz, C₆D₆): u.a. 1,10 und 1,36 [2s, H₃C-C]; 1,71 dt, Jₜ=1, J_{d}=7,5, HC(6)]; 1,89 [dd, J=3, J=7,5, HC(6)]; 2,45 und 3,00 [2d, J=16,5, HC(2)]; 3,07 [s breit, HC(7)]; 4,24 [dt, J=1, J=3 HC(5)]; 4,63 [d, J=3, HC(4)].

### Beispiel A4:

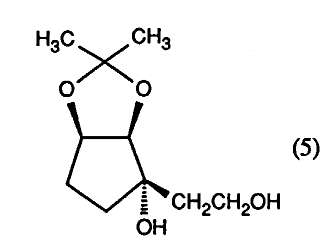

4,2 g (110,7 mmol) LiAlH₄ werden in 60 ml Diethylether unter Stickstoffatmosphäre suspendiert und bei -30°C während 20 Minuten mit 4,7 g (21,9 mmol) der Verbindung 4, gelöst in 80 ml Diethylether und 20 ml Methylenchlorid, versetzt. Nach vollständiger Zugabe wird das Kühlbad entfernt und 6 Stunden bei Rückflusstemperatur gerührt. Bei Raumtemperatur werden dann 20 ml Wasser und 10 ml 2M NaOH zugegeben. Das Reaktionsgemisch wird über Cellite filtriert, der weisse Aluminatrückstand noch mit 400 ml Methylenchlorid gewaschen und die vereinigten Filtrate im Vakuum eingedampft. Der Rückstand wird an Silikagel chromatographiert (Hexan/Essigester 1:2). Nach dem Eindampfen und Trocknen am Hochvakuum erhält man 3,7 g (84 %) der Titelverbindung als erstarrendes Oel (72 % ee). Durch selektive Kristallisation des Racemates aus Hexan (80 ml/1 g Produkt) lassen sich 2,7 g (61 %) der Titelverbindung in der Mutterlauge in praktisch enantiomeren reiner Form anreichern (97 % ee).

¹H-NMR (400 MHz, C₆D₆): 1,17 und 1,39 [2s, H₃C-C]; 1,54-1,59 [m, 1H]; 1.71-1.90 [m, 5H davon 1OH[; 2.01-2.10 [m 1H]; 3,59 und 3.81 [2s breit, HC(1)]; 4,24 [dd, J=1,4, J=5,5, HC(4)]; 4,68 [t, J=5,3, HC(5)]; [α]_{D}²⁵=-47,0 (c=1,0, Methanol).

### Beispiel A5:

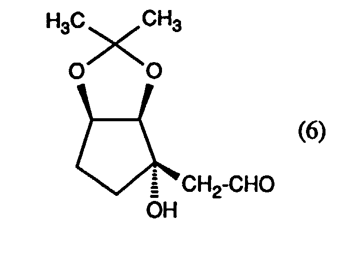

7,18 g (16,93 mmol) 1,1,1-tris(acetyloxy)-1,1-dihydro-1,2-benzjodoxol-3(1H)-on ("Dess-Martin-Reagenz") werden in 68 ml Methylenchlorid (0,25M Lösung) vorgelegt und unter Argonatmosphäre bei Raumtemperatur mit 2,74 g(13,55 mmol) der Verbindung 5 in 54 ml Methylenchlorid (0,25M Lösung) während 5 Minuten versetzt. Nach 2 Stunden Rühren wird die milchigweisse Reaktionslösung in ein Gemisch von 50 ml wässriger Na₂S₂O₃ (20 %), 150 ml gesättigter wässriger NaHCO₃ und 150 ml Diethylether gegossen. Es wird 10 Minuten gerührt und dann die organische Phase abgetrennt. Die wässrige Phase wird dreimal mit je 300 ml Diethylether extrahiert. Die vereinigten organischen Phasen werden über MgSO₄ getrocknet, filtriert und im Vakuum eingedampft. Der Rückstand wird in wenig Essigsäureethylester aufgenommen und über Silikagel filtriert (Essigsäureethylester). Eine Kugelrohrdestillation am Hochvakuum (0,026 bar, 72-75°C) liefert 1,78 g (66 %) der Titelverbindung als farbloses Oel.

¹H-NMR (300 MHz, C₆D₆): 1,09 und 1,33 [2s, H₃C-C]; 1,51-1,62 [m, 2H]; 1,70-1,77 [m, 1H]; 1,85-1,98 [m, 1H]; 2,23 [dd, J=0,9, J=18,0 HC(2)] 2,55 [dd, J=1,0, J=18,0 HC(2)]; 2,70 [s breit, OH]; 4,18 [dd, J=1,3, J=5,5 HC(4)]; 4,52 [t, J=5,2 HC(5)]; 9,33 [t, J=1,0 CHO].

### Beispiel A6:

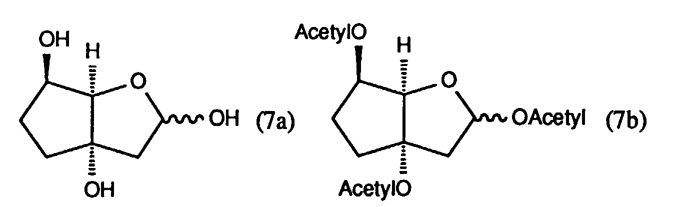

a) 1,64 g (8,19 mmol) der Verbindung 6, gelöst in 40 ml Wasser und 3,3 g Ionentauscherharz Amberlite® IR-120, werden 90 Minuten bei 55°C gerührt. Die Lösung wird filtriert und der Ionentauscher mit wenig Wasser nachgewaschen. Der pH des Filtrates wird mit gesättigter wässriger NaHCO₃ Lösung auf 8,0 eingestellt. Das Lösungsmittel wird im Vakuum bis auf 2 ml eingedampft, der Rückstand in 20 ml Pyridin aufgenommen und bis zur Trockene eingedampft. Die als gelbliches Oel erhaltene Verbindung 7a wird direkt in der Stufe b verwendet.
b) Das gelbliche Oel wird in 25 ml Pyridin aufgenommen, bei 0°C mit 5,4 ml (57,13 mmol) Essigsäureanhydrid und 250 mg (2,05 mmol) 4-Dimethylaminopyridin versetzt. Nach dreistündigem Rühren des Reaktionsgemisches bei Raumtemperatur wird wieder auf 0°C gekühlt und die Reaktion durch Zugabe von 100 ml gesättigter wässriger NaHCO₃ gestoppt. Es wird dreimal mit je 150 ml Methylenchlorid extrhiert, die organische Phase abgetrennt, über MgSO₄ getrocknet, filtriert und im Vakuum zur Trockene eingedampft. Der Rückstand wird zweimal mit je 100 ml Toluol aufgenommen und zur Trokkene eingedampft. Das gelbbraune Oel wird an Silikagel chromatographiert (Hexan/Essigester 2:1) und man erhält nach Entfernung des Lösungsmittels und Trocknen am Hochvakuum (2 Tage) 2,19 g (93 %) der Titelverbindung (7b) als leicht gelbliches Oel, das noch etwas Essigsäureethylester enthält.

¹H-NMR (400 MHz, CDCl₃): 6,40 [d, J=5,0, 0,5H HC(1)]; 6,34 [dd, J=2,2, J=5,3, 0,5H, HC(1)]; 5,10-5,05 [m, 0,5H, HC(5)]; 5,03-4,97 [m, 0,5H, HC(5)]; 4,79 [d, J=5,3, 0,5H, HC(4)]; 4,73 [d, J=5,8, 0,5H, HC(4)]; 2,67-1,73 [Signalhaufen 15H, darunter 2,09, 2,08, 2,063, 2,061, 2,05, 2,04 6s, H₃C-CO].

### B) Herstellung von Nukleosiden

### Beispiel B1:

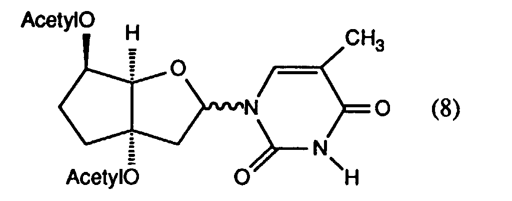

1,25 g (9,91 mmol) Thymin werden in 100 ml Acetonitril unter Argonatmosphäre suspendiert und nacheinander bei 0°C mit 1,65 ml (7,91 mmol) Hexamethyldisilazan, 1,0 ml (7,89 mmol) Trimethylchlorsilan und 1,39 ml (11,83 mmol) SnCl₄ versetzt. Nun werden 2,83 g (9,89 mmol) der Verbindung 7(b), gelöst in 20 ml Acetonitril, innerhalb von 5 Minuten zur Suspension gegeben, das Eisbad entfernt und die nun klare Lösung während 35 Minuten bei Raumtemperatur und 35 Minuten bei 50°C gerührt. Die auf Raumtemperatur abgekühlte Lösung wird in 300 ml gesättigte wässrige NaHCO₃ gegossen und zweimal mit je 300 ml Essigsäureethylester extrahiert. Die organische Phase wird über Watte filtriert und das Lösungsmittel im Vakuum entfertn. Das Trocknen am Hochvakuum liefert einen bräunlichen Schaum, der an Silikagel chromatographiert wird (Hexan/Essigsäureethylester 1:3). Nach Entfernung des Lösungsmittels im Vakuum und Trocknen im Hochvakuum erhält man 2,55 g (73 %) der Titelverbindung als farblosen Schaum (Anomerengemisch α:β = 1:2).

¹H-NMR (200 MHz, CDCl₃): 8,57 [s, breit, 1H, HN]; 7,31, 7,28 [2s, 1H, HC(6)]; 6,30-6,18 [m, 1H, HC(1')]; 5,19-5,00 [m, 1H, HC(5')]; 4,91 [d, J=5,0, 0,4H, HC(4')]; 4,63 [d, J=6, 0,6H, HC(4')]; 2,96 [dd, J=15,0, 0,6H, HC(2')]; 2,79 [dd, J=7, J=15, 0,4H, HC(2')]; 2,55-1,50 [Signalhaufen 14H, darunter 2,13, 2,09, 2,05, 1,95 4s, H₃C].

### Beispiel B2:

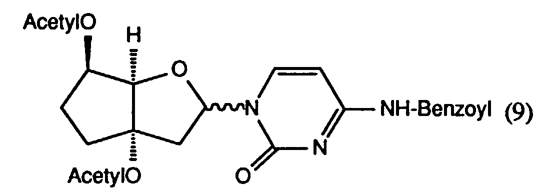

Zu einer Suspension von 1,345 g (6,25 mmol) N-4-Benzoylcytosin in 50 mol Acetonitril werden bei Raumtemperatur (RT) 3,1 ml (12,5 mmol) N,O-bis-Trimethylsilylacetamid zugefügt. Nach 45 Minuten wird zum nunmehr homogenen Reaktionsgemisch der Reihe nach 1,431 g (5 mmol) der Verbindung 7b, gelöst in 25 ml Acetonitril, und 1,91 ml (16,2 mmol) Zinntetrachlorid gegeben. Nach 50 Minuten Rühren bei Raumtemperatur wird das Reaktionsgemisch in 200 ml Methylenchlorid aufgenommen und mit zweimal 200 ml gesättigter NaHCO₃-Lösung und zweimal 200 ml gesättigter NaCl-Lösung extrahiert, wobei die wässrigen Phasen noch je zweimal mit 200 ml Methylenchlorid extrahiert werden. Die vereinigten organischen Phasen werden über MgSO₄ getrocknet, eingeengt und der Rückstand an Silikagel mit Methylenchlorid/Methanol 30:1 chromatographiert. Auf diese Weise erhält man nach Fällen aus 400 ml Pentan und Trocknen am Hochvakuum (HV) über Nacht 1,99 g (90 %) der Titelverbindung als ca 1:1 Anomerengemisch in Form eines leicht bräunlichen Pulvers.

¹H-NMR (400 MHz, CDCl₃): u.a. 8,71 (s, breit,/1H/NH); 8,14, 7,97 (2d, J=7,5,/1H/H-C6); 6,26-6,21 (m/1H/H-C1'); 5,16 (dt, J_{d}=6,9, Jₜ=5,9/0,5H/H-C5'); 5,08 (dt, J_{d}=9,8, Jₜ=5,8/0,5H/H-C(5'); 5,07 (d, J=5,8/0,5H/H-C4'); 4,78 (d, J=5,7/0,5H/H-C4'); 3,33 (dd, J=5,5, 14,9/0,5H/H-C2'); 2,89 (dd, J=6,6, 15,2/0,5H/H-C2'); 2,72 (dd, J=3,8, 15,2/0,5H/H-C2'); 2,13, 2,12, 2,08, 1,95 (4s/6H/CH₃CO).

### Beispiel B3:

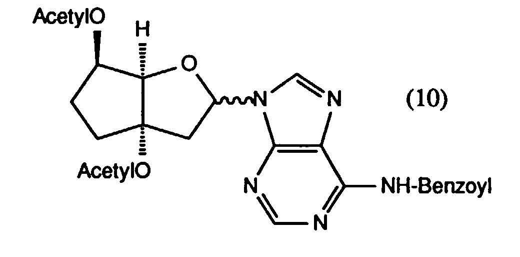

3,86 g (16,1 mmol) N-6-Benzoyladenin werden in 20 ml Acetonitril suspendiert und mit 5,86 ml (32,3 mmol) N,O-bis-Trimethylsilylacetamid versetzt. Nach 30 Minuten Rühren bei Raumtemperatur wird zum nunmehr homogenen Reaktionsgemisch eine Lösung von 2,31 g (8,1 mmol) der Verbindung 7b) in 15 ml Acetonitril gegeben, gefolgt von 200 µl Trifluormethansulfonsäure-triethylsilylester. Nach 2 Stunden Reaktionszeit bei Rückflusstemperatur lässt man auf Raumtemperatur abkühlen und rührt noch 3 Stunden nach. Anschliessen wird die tiefbraune Reaktionslösung auf ein Gemisch von 100 ml gesättigter NaCl-Lösung und 100 ml gesättigter NaHCO₃-Lösung gegossen und mit zweimal 200 ml Methylenchlorid extrahiert. Die vereinigten organischen Phasen weden über MgSO₄ getrocknet und eingedampft. Der Rückstand wird in wenig Essigester gelöst, filtriert und das Filtrat an Silikagel mit Essigester chromatographiert. Die produkthaltigen Fraktionen werden vereinigt, vom Lösungsmittel befreit und der resultierende Rückstand am Hochvakuum (1h) zu einem leicht gelblichen Schaum getrocknet. Es verbleiben 2,94 g (78 %) der Titelverbindung als Anomerengemisch im Verhältnis von α:β = 3:2.

¹H-NMR (400 MHz, CDCl₃): u.a. 8,79, 8,78, 8,25, 8,24 (4s/2H/H-C(2,8); 6,54 (dd, J=3,5, 6,7/0,6H/H-C1'), 6,48 (dd, J=5,8, 9,0/0,4H/H-C(1'); 5,17 (dt, J_{d}=8,4, Jₜ=5,8/0,6H/H-C5'), 5,05 (dt, J_{d}=9,7, Jₜ=6,2/0,4H/H-C5'); 4,98 (d, J=5,3/0,6H/H-C4'); 4,77 (d, J=5,4/0,4H/H-C4'); 3,32 (dd, J=3,5, 15,0/0,6H/H-C2'), 3,14 (dd J=5,8, 14,6/0,4H/H-C2'); 2,91 (dd, J=6,8, 15,1/0,6H/H-C2'); 2,84 (dd, J=8,9, 14,6/0,4H/H-C2'); 2,13, 2,11, 2,04, 1,94 (4s/6H/CH₃-CO).

### Beispiel B3a:

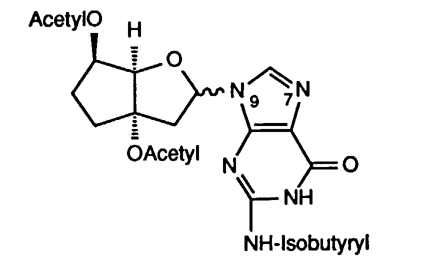

4,69 g (21,2 mmol) N₂-Isobutyroylguanin werden unter Argon in 150 ml absolutem Acetonitril suspendiert und mit 19 ml (76,3 mmol) N,O-Bis(trimethylsilyl)acetamid versetzt. Nach 1 h Rühren bei RT und 30 Minuten bei 40°C wird zum homogenen Gemisch eine Lösung von 3,03 g (10,6 mmol) der Verbindung 7b in 15 ml absolutem Acetonitril gefolgt von 4,2 ml (5,2 g, 23,3 mmol) Trifluormethylsulfonsäuretrimethylsilylester gegeben. Nach 16 Stunden Reaktionstzeit bei RT und 6 Stunden bei 40°C wird die Reaktionslösung eingeengt, das resultierende Oel in 250 ml Methylenchlorid aufgenommen und mit 2 x 250 ml gesättigter NaHCO₃-Lösung extrahiert. Die wässrigen Phasen werden mit 3 x 250 ml Methylenchlorid rückextrahiert. Das Einengen der über MgSO₄ getrockneten, vereinigten organischen Phasen ergibt einen braunen Schaum, der zur Trennung in die diastereomeren Reaktionsprodukte erst an 300 g, dann noch 2 mal an 250 g Kieselgel 60 (Methylenchlorid:MeOH 39:1) chromatografiert wird. Man erhält 2,13 g (45 %) der Titelverbindung als Anomerengemisch im Verhältnis α:β 8:5 und 1,75 g (37 %) der zur Titelverbindung isomeren N7-Nukleoside ebenfalls als Anomerengemisch im Verhältnis α:β 3:2.

¹H-NMR (400 MHz, CDCl₃) der Titelverbindung: 1,23-1,28 (m, 6H, H₃C(isobut)); 1,82-2,03, 2,18-2,28, 2,31-2,41 (m, 4H, H-C(6'), H-C(7')); 1,98, 2,06 (2s, 2,3H, 2 H₃C-CO); 2,04, 2,10 (2s, 2,7H, 2 H₃C-CO); 2,45 (dd, J = 9,1, 14,6, 0,55H, H-C(2')); 2,70, 2,71 (heptett, J = 6,9, 1H, H-C(isobut)); 2,81 (dd, J = 7,0, 15,2, 0,45H, H-C(2')); 3,03 (dd, J = 5,7, 14,6, 0,55H, H-C(2')); 3,10 (dd, J = 3,2, 15,2, 0,45H, H-C(2')); 4,68 (d, J = 5,6, 0,45H, H-C(4')); 4,90 (d, J = 5,2, 0,55H, H-C(4')); 4,98-5,03 (m, 0,45H, H-C(5')); 4,10-5,15 (m, 0,55H, H-C(5')); 6,20 (dd, J = 5,7. 9,0, 0,55H, H-C(1')); 6,26 (dd, J = 3,2, 6,9, 0,45H, H-C(1')); 7,86 (s, 0,55H, H-C(8)); 7,90 (s, 0,45H, H-C(8)); 8,87 (s, br, 0,45H, H-N(1)); 8,93 (s, br, 0,55H, H-N(1)); 2,15, 2,20 (2s, br, 1H, H-N(2)).

### Beispiel B4:

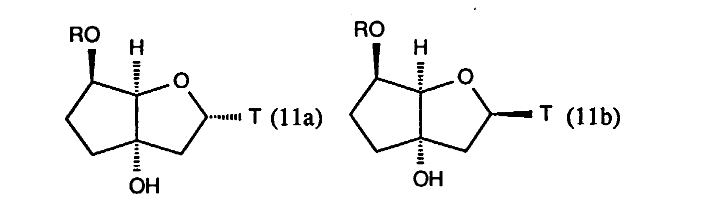

R = t-Butyldimethylsilyl
T = Thymidyl

2,30 g (6,53 mmol) der Verbindung 8 weden in 260 ml Lösungsmittelgemisch (Tetrahydrofuran(THF)/Methanol/H₂O 5:4:1) gelöst und bei 0°C mit 26 ml (52 mmol) 2N NaOH versetzt. Nach 75 Minuten Rühren bei derselben Temperatur werden 3,5 g (65,4 mmol) NH₄Cl zugegeben und solange weitergerührt bis eine klare Lösung entsteht. Das Lösungsmittel wird im Vakuum entfernt, der Rückstand in 200 ml Methanol aufgenommen, an 8 g Silikagel adsorbiert und an Silikagel chromatographiert (Methylenchlorid/Methanol 5:1). Das Lösungsmittel wird im Vakuum entfernt. Der farblose Schaum wird in wenig Methanol gelöst mit 100 ml Pentan versetzt, im Vakuum bis zur Trockne eingedampft. Nach Trocknen über Nacht im Hochvakuum werden 1,65 g (94 %) des deacetylierten Produktes als farbloses Pulver erhalten. Dieses Pulver wird in 33 ml Pyridin unter leichtem Erwärmen gelöst und dann bei 0°C unter Argonatmosphäre mit 1,84 ml (8,01 mmol) t-Butyldimethylsilyl-triflat versetzt. Bei dieser Temperatur wird während 45 Minuten weitergerührt. Zur klaren Reaktionslösung werden 100 ml gesättigte wässrige NaHCO₃ zugegeben und das Gemisch dreimal mit je 150 ml Essigsäureethylester extrahiert. Das Lösungsmittel wird im Vakuum entfernt und der farblose Schaum kurz am Hochvakuum getrocknet. Chromatographie an Silikagel (5 % Aceton in Diethylether) liefert die beiden Anomeren der Titelverbindung: 11a, 655 mg (28 %) und 11b, 1,47 g (62 %).

¹H-NMR von 11a (400 MHz, CDCl₃): u.a. 7,37 [s, 1H, HC(6)]; 6,05 [dd, J=2,5, J=7,8, 1H,HC(1')]; 4,45 [d, J=4,6, 1H,HC(4')]; 4,15 [dd, J=4,2, J=8,6, 1H,HC(5')]; 2,61 [dd, J=7,9, J=14,7, 1H,HC(2')]; 2,49 [d, J=14,5 [d, J=14,5, 1H,HC(2')]; 1,89 [s, 3H, H₃C-C(5)]; 0,91 [s, 9H, H₃C-C]; 0,10, 0,09 [2s, 6H, H₃C-Si].

¹H-NMR von 11b (400 MHz, CDCl₃): u.a. 7,66 [s, 1H, HC(6)]; 6,39 [dd, J=4,9, J=9,3, 1H,HC(1')]; 4,19 [m, 1H,HC(5')]; 4,08 [d, J=6,0, 1H,HC(4')]; 3,18 [s breit, 1H,OH]; 2,67 [dd, J=5,0, J=13,6, 1H,HC(2')]; 1,92 [s, 3H, H₃C-C(5)]; 0,91 [s, 9H, H₃C-C]; 0,11, 0,09 [2s, 6H, H₃C-Si].

### Beispiel B4a:

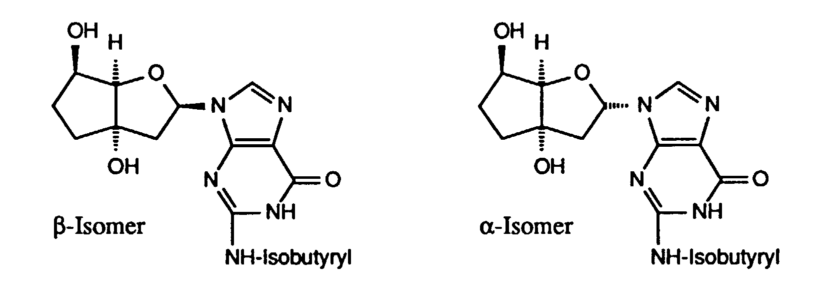

1,13 g (2,53 mmol) der Titelverbindung aus Beispiel B3a werden in 250 ml Lösungsmittelgemisch (THF:MeOH:H₂O 5:4:1) gelöst und bei 2°C mit 25 ml (50 mmol) 2N NaOH versetzt. Nach 25 Minuten Rühren bei derselben Temperatur werden 4 g (75 mmol) Ammoniumchlorid zugegeben und solange weitergerührt bis eine klare Lösung entsteht. Nach Abziehen des Lösungsmittels wird der weisse Rückstand kurz am HV getrocknet und anschliessend zur Trennung der Anomeren an 150 g Kieselgel 60 (Methylenchlorid:Methanol 10:1) chromatographiert. Reine Fraktionen werden vereinigt, Mischfraktionen können durch wiederholtes Chromatographieren an Kieselgel 60 getrennt werden. Man erhält so 427 mg (47 %) des apolareren β-Isomers und 254 mg (28 %) des polareren α-Isomers. Zusammen 681 mg (75 %).

¹H-NMR (400 MHz, CD₃OD) des α-Anomers: 1,25 (d, J = 6,8, 6H, H₃C(isobut)); 1,69-1,80, 2,02-2,11 (2m, 4H, H-C(6'), H-C(7')); 2,65 (dd, J = 7,2, 14,4, 1H, H-C(2')); 2,73-3,17 (m, 2H, H-C(2'), H-C(isobut)); 4,11-4,16 (m, 1H, H-C(5')); 4,34 (d, J = 5,2, H-C(4')); 6,42 (dd, J = 2,8, 7,1, 1H, H-C(1')); 8,38 (s, 1H, H-C(8)).

¹H-NMR (400 MHz, CD₃OD) des β-Anomers: 1,22 (d, J = 6,9, 6H, H₃-C(isobut)); 1,65-1,72, 1,86-1,97, 2,06-2,15 (3m, 4H, H-C(6'), H-C(7')); 2,50 (dd, J = 9,7, 13,2, 1H, H-C(2')); 2,57 (dd, J = 5,4, 13,3, 1H, H-C(2')); 2,72 (heptett, J = 6,9, 1H, H-C(isobut)); 4,09-4,13 (m, 2H, H-C(4'), H-C(5')); 6,32 (dd, J = 5,4, 9,6, 1H, H-C(1')); 8,3 (s, 1H, H-C(8)).

### Beispiel B5:

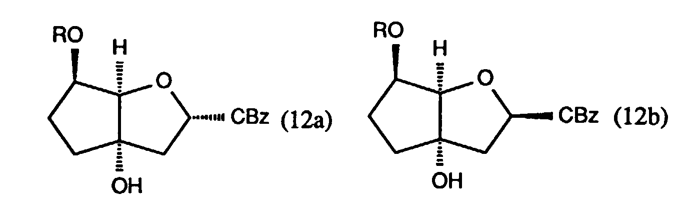

R = t-Butyldimethylsilyl
CBz = Benzoylcytosyl

1,99 g (4,5 mmol) der Verbindung 9 werden in 400 ml THF/Methanol/Wasser 5:4:1 gelöst und bei 0°C mit 40 ml 2N wässriger NaOH versetzt. Nach 20 Minuten Rühren bei 0°C werden 6 g (112 mmol) Ammoniumchlorid zugegeben, das Reaktionsgemisch anschliessend eingedampft und der Rückstand an Silikagel mit Methylenchlorid/Methanol 10:1 chromatographiert. Die produkthaltigen Fraktionen werden vereinigt, eingeengt, der resultierende Rückstand (1,35 g) in 26 ml Pyridin gelöst und bei 0°C mit 1,11 ml (4,86 mmol) t-Butyldimethylsilyl-trifluormethansulfonat versetzt. Nach 30 Minuten wird das Reaktionsgemisch in 200 ml gesättigter wässriger NaHCO₃ aufgenommen und dreimal mit 200 ml Essigester extrahiert. Die vereinigten organischen Phasen werden über MgSO₄ getrocknet, eingeengt und der Rückstand an Silikagel mit Essigester chromatographiert. Der Elutionsreihenfolge nach erhält man auf diese Weise nach Trocknen über Nacht 865 mg (41 %) der Verbindung 12b als farbloses Kristallisat sowie 677 mg (32 %) 12a als farblosen Schaum.

¹H-NMR von 12a (400 MHz, CDCl₃): u.a. 7,95 (d, J=7,4/1H/H-C6); 6,06 (dd, J=3,0, 5.7/1H/H-C1'); 4,53 (d, J=4,6/1H/H-C4'); 4,16 (dd, J=4,0, 8,5/1H/H-C5'); 2,67-2,65 (m/2H/H-C2').

¹H-NMR von 12b (400 MHz, CDCl₃): u.a. 8,59 (d, J=7,5/1H/H-C6); 6,47 (dd, J=5,4, 8.5/1H/H-C1'); 4,24 (d, J=5,4/1H/H-C4'); 4,24-4,16 (m/1H/H-C5'); 3,10 (dd, J=5,4, 14,0/1H/H-C2'); 1,77 (dd, J=8,5, 14,0/1H/H-C2').

### Beispiel B6:

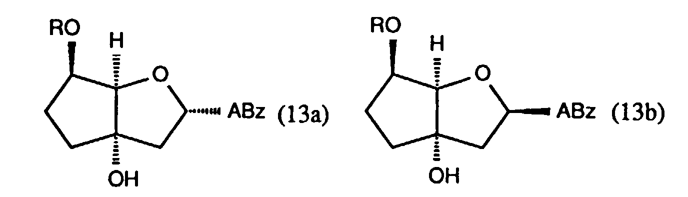

R = t-Butyldimethylsilyl
ABz = Benzoyladenyl

3,36 g (7,24 mmol) der Verbindung 10 werden in 289 ml eines Gemisches von THF/Methanol/Wasser 5:4:1 gelöst und bei 0°C mit 28,9 ml 2N NaOH-Lösung versetzt. Nach 30 Minuten Rühren werden dem Reaktionsgemisch 3,6 g (67 mmol) Ammoniumchlorid zugesetzt und das Eisbad entfernt. Nach dem Entstehen einer homogenen Lösung wird das Reaktionsgemisch eingeengt, der Rückstand in 50 ml Methanol gelöst und an 5 g Silikagel adsorbiert. Chromatographie an Silikagel mit Methylenchlorid/Methanol 9:1, liefert nach kurzem Trocknen 1,98 g des deacetylierten Produktes, welches anschliessend in 20 ml Pyridin gelöst und bei 0°C mit 1,43 ml (6,24 mmol) t-Butyldimethylsilyl-trifluormethansulfonat versetz wird. Nach 30 Minuten Reaktionszeit wird das Gemisch auf 100 ml wässrige NaHCO₃-Lösung gegossen und zweimal mit 200 ml Ether extrahiert. Die vereinigten organischen Phasen werden über MgSO₄ getrocknet und eingedampft. Das Rohprodukt (2,3 g) wird an Silikagel mit Diethylether (wassergesättigt)/Aceton 83:17 chromatographiert, wobei zuerst das Isomer 13a und dann das Isomer 13b eluiert wird. Reine Fraktionen werden vereinigt und eingedampft, und Mischfraktionen noch ein zweitesmal analog chromatographiert. Auf diese Weise erhält man nach dem Trocknen über Nacht 1,452 g (57 %) 13a und 0,864 g (33 %) 13b, je in Form eines weissen Schaumes.

¹H-NMR von 13a (400 MHz, CDCl₃): u.a. 8,79, 8,07 (2s/2H/H-C2,8); 6,34 (dd, J=3,8, 7,3/1H/H-C1'); 4,39 (d, J=4,8/1H/H-C4'); 4,18 (dt, J_{d}=6,1, Jₜ=4,8(1H/H-C5'); 2,92-2,82 (m/2H/H-C2'), 0,92 (s/9H/3CH₃-C); 0,107, 0,102 (2s/6H/2CH₃-Si).

¹H-NMR von 13b (400 MHz, CDCl₃): u.a. 8,79, 8,47 (2s/2H/H-C2,8); 6,57 (dd, J=5,3, 9,0/1H/H-C1'); 4,23-4,18 (m/2H/H-C4',5'); 3,07 (s,breit/1H/OH); 2,84 (dd, J=5,4, 13,5/1H/H-C2'); 2,37 (dd, J=9,1, 13,6/1H/H-C2'); 0,88 (s/9H/3CH₃-C); 0,10, 0,06 (2s/6H/2CH₃-Si).

### Beispiel B7:

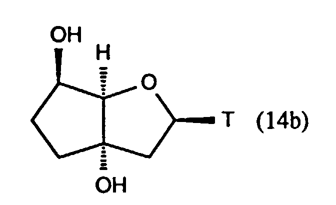

Eine Lösung von 1,07 g (2,80 mmol) der Verbindung 11b in 20 ml Tetrahydrofuran wird bei Raumtemperatur mit 1,76 g (5,58 mmol) Tetrabutylammoniumfluorid (TBAF)·3H₂O versetzt und während 4 Tagen stehengelassen. Das Reaktionsgemisch wird an 10 g Silikagel adsorbiert und an Silikagel chromatographiert (Methylenchlorid/Methanol 6:1). Kristallisation des Produktes (Methanol/Diethylether 4:1/Pentan:isotherme Destillation) liefert 521 mg (69 %) der Titelverbindung als farblose Kristalle.

¹H-NMR (400 MHz, D₂O): u.a. 7,67, [s, 1H,HC(6)]; 6,24 [dd, J=5,2, J=10,1, 1H, HC(1')]; 4,19 [dt, Jt=5,6, Jd=9,4, 1H, HC(5')]; 4,09 [d, J=5,3, 1H,HC(4')]; 2,49 [dd, J=5,2, J=1,41, 1H,HC(2')]; 2,15 [dd, 10,2, J=14,1, 1H,HC(2')]; 1,89 [s, 3H,H₃C-C(5)].

### Beispiel B8:

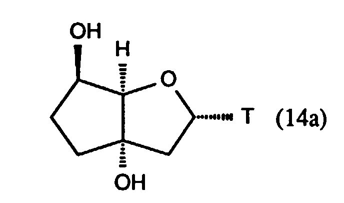

In Analogie zu Beispiel B7 erhält man aus 638 mg (1,67 mmol) der Verbindung 11a nach Chromatographie an Silikagel (Methylenchlorid/Methanol 6:1) und Kristallisation aus (Methanol/Diethylether 4:1/Pentan:isotherme Destillation) 345 mg (77 %) der Titelverbindung als farblose Kristalle.

¹H-NMR (300 MHz, D₂O): u.a. 7,69 [s, 1H,HC(6)]; 6,15 [dd, J=4,3, J=7,0, 1H,HC(1')]; 4,37 [d, J=5,3, 1H,HC(4')]; 4,17-4,10 [m, 1H,HC(5')]; 2,57 [dd, J=7,0, J=14,7, 1H,HC(2')]; 2,38 [dd, J=4,3, J=14,7, 1H,HC(2')]; 1,84 [s, 3H,H₃C-C(5)].

### Beispiel B9:

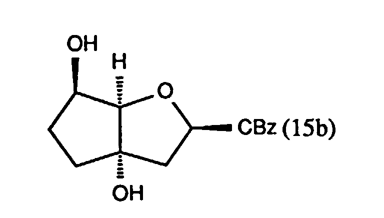

992 mg (2,77) mmol der Verbindung 12b werden in 27 ml THF gelöst und mit 1,75 g (5,5 mmol) Tetrabutylammoniumfluorid-trihydrat versetzt. Nach 84 Stunden Reaktionszeit bei Raumtemperatur wird eingeengt und der Rückstand an Silikagel mit Methylenchlorid/Ethanol 10:1 chromatographiert. Die vereinigten produkthaltigen Fraktionen werden eingeengt und der Rückstand (670 mg) in 6 ml Wasser bei 60°C aufgeschlämmt. Nach dem Stehenlassen bei Raumtemperatur für einige Stunden wird filtriert und die feinen weissen Nadeln der Titelverbindung im Hochvakuum bei 40°C einige Stunden getrocknet. Man erhält 488 mg (66 %) der Titelverbindung.

¹H-NMR (400 MHz, d6-DMSO):u.a. 8,59 (d, J=7,5/1H/H-C6); 6,18 (dd, J=5,3, 9,2/1H/H-C1'); 4,01-3,95 (m/2H/H-C4',5'), 2,54-2,49 (m/H-C2'+ Lsm.); 1,82 (dd, J=9,2, 13,3/1H/H-C2').

### Beispiel B10:

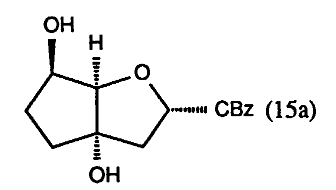

Mit 806 mg (2,25 mmol) der Verbindung 12a verfährt man analog Beispiel B9. Nach der Chromatographie an Silikagel mit Methylenchlorid/Methanol 10:1 und anschliessender Kristallisation aus Methanol/Ether/Pentan erhält man 398 mg (65 %) der Titelverbindung in Form von weissen, sternförmig angeordneten Nadeln.

¹H-NMR (400 MHz, d6-DMSO):u.a. 8,26 (d, J=7,4/1H/H-C6); 6,14 (dd, J=2,9, 7,0/1H/H-C1'); 4,37 (d, J=5,2/1H/H-C4'); 3,96 (dt, J_{d}=11,2, Jₜ=5,9/1H/H-C5'); 251-2,46 (m/H-C‴ + Lösungsmittel); 2,23 (dd, J=2,9, 14,1/1H/H-C2').

### Beispiel B11:

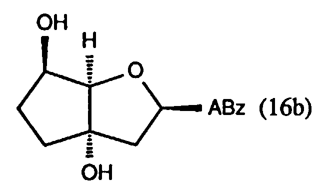

852 mg (1,72 mmol) der Verbindung 13b werden in 20 ml THF gelöst und mit 651 mg (2,1 mmol) Tetrabutylammoniumfluorid-trihydrat versetzt. Nach 12 Stunden Reaktionszeit bei 50°C werden 500 mg (9,3 mmol) Ammoniumchlorid zugefügt und das Reaktionsgemisch nach weiteren 30 Minuten eingeengt. Der Rückstand wird an Silikagel mit Methylenchlorid/Methanol 9:1 chromatographiert und die Produkfraktionen (noch verunreinigt mit Tetrabutylammoniumsalzen) eingedampft. Kristallisation aus 10 ml Wasser liefert nach Trocknen am Hochvakuum während 15 Stunden 530 mg (81 %) der Titelverbindung in Form von farblosen Nadeln.

¹H-NMR (300 MHz, CD₃OD):8,73, 8,71 (2s/2H/H-C2,8); 8,09 (d breit/2H/H-aromatisch); 7,70-7,54 (m/3H/H-aromatisch); 6,58 (dd, J=5,0, 9,0/1H/H-C1'); 4,22-4,10 (m/2H/HC-4',5'); 2,72 (dd, J=9,0, 13,0/1H/H-C2'); 2,66 (dd, J=5,0, 13,0/1H/H-C2'); 2,20-1,65 (m/4H/H-C6',7').

### Beispiel B12:

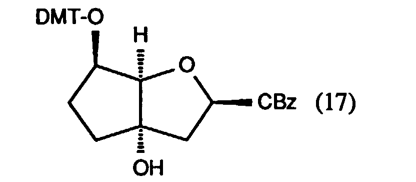

Zu einer Suspension aus 1, 17 g (3,27 mmol) der Verbindung 15b in 24 ml Lutidin und 24 ml Methylenchlorid wird 2,22 g (4,91 mmol) 4,4'-Dimethoxytriphenylmethyl-trifluormethansulfonat (DMT-triflat) hinzugegeben. Nach 4 Stunden Rühren wird die Lösung in 250 ml gesättigte wässrige NaHCO₃ gegossen und dreimal mit je 250 ml Methylenchlorid extrahiert. Die organischen Phasen werden über MgSO₄ getrocknet, filtriert und das Lösungsmittel im Vakuum entfernt. Lutidin wird im Hochvakuum (HV) entfernt und der sultierende braune Schaum anschliessend an Silikagel chromatographiert (Triethylamin/Essigsäureethylester 1:99). Es werden 1,96 g (91 %) der Titelverbindung als praktisch farbloser Schaum erhalten.

¹H-NMR (400 MHz, CDCl₃):u.a. 8,75 [d, J=7,5, 1H,HC(6)]; 6,85 [dd, J=2,0, J=8,9, 4H, H aromatisch (Trityl)]; 6,33 [dd, J=5,6, J=8,1, 1H,HC(1')]; 3,98-3,91 [m, 1H,HC(5')]; 3,80 [s, 6H,OCH₃]; 3,69 [d, J=6,4, 1H,HC(4')]; 3,04 [dd, J=5,6, J=14,1, 1H,HC(2')].

### Beispiel B13:

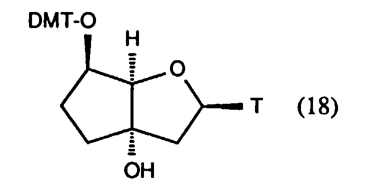

Analog zu Beispiel B12 werden aus 442 mg (4,65 mmol) der Verbindung 14b nach Chromatographie an Silikagel (Hexan/Essigsäureethylester 1:9, 2 % Triethylamin) und Kristallisation (Essigsäureethylester/Pentan) 736 mg (78 %) der Titelverbindung erhalten.

¹H-NMR (200 MHz, CDCl₃):u.a. 7,78 [d, J=1,0, 1H,HC(6)]; 6,83 [d, J=9,0, 4H, H aromarisch (Trityl)]; 6,29 [dd, J=5,0, J=10,0, 1H,HC(1')]; 3,99-3,87 [m, 1H,HC(5')]; 3,76 [s, 6H,OCH₃]; 3,72 [d, J=6,0, HC(4')]; 2,58 [dd, J=5,0, J=13,0, 1H,HC(2')]; 1,75 [d, J=1,0, 3H,H₃C-C(5)].

### Beispiel B14:

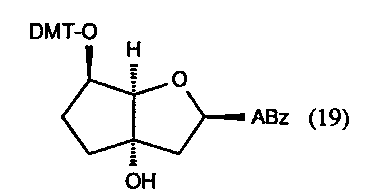

505 mg (1,32 mmol) der Verbindung 16b werden in 10 ml Pyridin gelöst und mit 1,198 g (2,64 mmol) DMT-Triflat versetzt. Nach 30 Minuten werden weiter 2,0 g (4,4 mmol) DMT-Triflat zugegeben. Nach insgesamt 3 Stunden Reaktionszeit wird das Rohgemisch in 100 ml Essigester aufgenommen und mit dreimal 100 ml gesättigter NaHCO₃-Lösung extrahiert. Die wässrigen Phasen werden noch einmal mit je 100 ml Essigester extrahiert. Die vereinigten organischen Phasen werden eingedampft und der Rückstand an Silikagel chromatographiert. Zuerst werden mit Essigester apolare Nebenprodukte eluiert, dann mit Methylenchlorid/Methanol 9:1 die gewünschte Titelverbindung, welche nach Einengen und Trocknen am Hochvakuum über Nacht in einer Menge von 770 mg (85 %) als leicht gelblicher Schaum anfällt.

¹H-NMR (200 MHz, CDCl₃):u.a. 8,80,8,51 (2s/2H/HC-2,8); 6,81 (d br./4H/Haromatisch (Trityl)); 6,41 [dd, J=4,0,9,0/1H/H-C1'); 4,92 (dt, J_{d}=10,0, Jₜ=10,0, Jₜ=5,0/1H/H-C5'); 3,76 (s/6H/O-CH₃); 3,69 (d, J=5,0/1H/H-C4'); 2,79 (dd, J=4,0, 14,0/1H/H-C2'); 2,35 (dd, J=9,0, 14,0/1H/H-C2').

### Beispiel B14a:

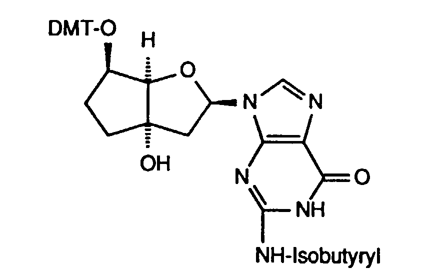

221 mg (608 µmol) der Verbindung aus Beispiel 4a (β-Isomer) werden in 2 ml absolutem Pyridin gelöst und unter Rühren mit 413 mg (912 µmol) DMT-triflat versetzt. Nach 2 und nach 4 Stunden versetzt man je mit weiteren 137 mg (304 µmol) DMT-triflat. Nach 6,5 Stunden nimmt man in 20 ml Methylenchlorid auf, extrahiert der Reihe nach mit 15 ml gesättigter NaHCO₃-Lösung, 15 ml wässriger Zitronensäurelösung (10 %) und 15 ml gesättigter NaHCO₃-Lösung. Die wässrigen Phasen werden mit jeweils 10 ml Methylenchlorid rückextrahiert. Die vereinten organischen Phasen werden über MgSO₄ getrocknet, eingeent und der erhaltene gelbe Schaum an 40 g Kieselgel 60 (Methylenchlorid:Methanol 20:1, 2 % Triethylamin) chromatographiert. Anschliessendes Fällen aus 120 ml Diethylether bei RT ergibt 313 mg (77 %) der Titelverbindung als weisses Pulver.

¹H-NMR (400 MHz, CDCl₃): 1,10-1,25, 1,68-1,70 (2m, 9H, H₃C(isobut), H-C(6'), H-C(7')); 2,02 (dd, J = 8,5, 13,0, 1H, H-C(2')); 2,81-2,88 (m, 1H, H-C(isobut)); 3,69, 3,70 (2s, 6H, H₃C-O(DMT)); 3,88-3,90 (m, 2H, H-C(4'), H-C(5')); 4,40 (s, br, 1H, HO-C(3')); 6,02-6,06 (m, 1H,H-C(1')); 6,73 (dd, J = 7,6, 8,9, 4H, H-C(ar)); 7,11 (t, J = 7,3, 1H, H-C(ar)); 7,16-7,20 (m, 2H, H-C(ar)); 7,33-7,36 (m, 4H, H-C(ar)); 7,46 (d, J = 7,3, 2H, H-C(ar)); 8,16 (s, 1H, H-C(8)); 10,9 (s, br, 1H, H-N(2)); 12,3 (s, br, 1H, H-N(1)).

### Beispiel B15:

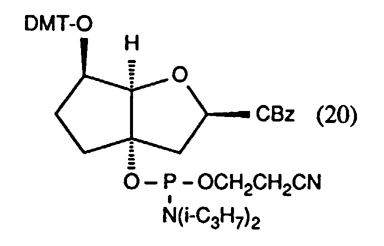

395 mg (0,6 mmol) der Verbindung 17 werden unter Argon in 5 ml THF gelöst und mit 410 µl (2,4 mmol) N,N-diisopropylethylamin, sowie 270 µl (1,2 mmol) 2-cyanethyl-N,N-diisopropylchlorophosphoramidit versetzt. Nach 2 Stunden Rühren wird das Reaktionsgemisch mit 50 ml Essigsäureethylester versetzt und zweimal mit 20 ml gesättigter wässriger NaHCO₃ extrahiert. Die organische Phase wird über MgSO₄ getrocknet, filtriert und das Lösungsmittel im Vakuum entfernt. Der gelbe am Hochvakuum getrocknete Schaum wird an Silikagel chromatographiert (Hexan/Essigsäureethylester 1:2, 1,5 % Triethylamin). Der erhaltene gelbliche Schaum wird zweimal umgefällt (Methylenchlorid/Pentan) und es werden 374 mg (73 %) der Titelverbindung als farbloses Pulver isoliert.

¹H-NMR (400 MHz, CDCl₃);u.a. 8,75 [d, J=7,4, 1H,HC(6)]; 6,86 (dd, J=1,9, J=8,6, 4H,H aromatisch (Trityl)]; 6,21-6,15 [m, 1H,HC(1')]; 3,82, 3,81 [2s, 6H, OCH₃]; 2,63 [t, J=6,4, 1H, -H₂C-CN]; 2,55 [q, J=6,2, 1H, -H₂C-CN]; 1,15-1,11 [m, 12H, CH(CH₃)₂].

### Beispiel B16:

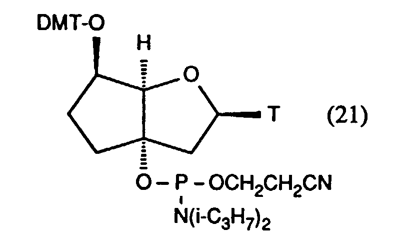

Analog zu Beispiel B15 erhält man aus 108 mg (0,189 mmol) der Verbindung 18 nach Chromatographie an Silikagel (Hexan/Essigsäureethylester/Triethylamin 20:10:4) 91 mg (62 %) der Titelverbindung als weissen Schaum.

¹H-NMR (200 MHz, CDCl₃):u.a. 7,79, 7,78 [2d, J=1,0, 1H,HC(6)]; 6,82 (d breit, J=8,0, H aromatisch (Trityl)]; 6,33-6,20 [m, 1H,HC(1')]; 3,78, 3,77 [2s, 6H, OCH₃]; 2,62, 2,56 [2t, J=7,0, 2H, -H₂C-CN]; 1,73, 1,68 [2d, J=1,0, H₃C-C(5)]; 1,19-1,09 [m, 12H, CH(CH₃)₂].

### Beispiel B17:

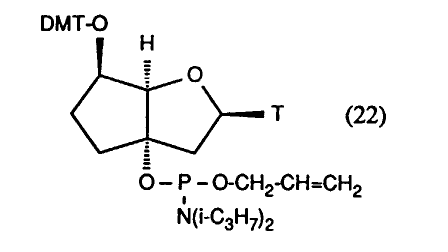

Analog zu Beispiel B15 erhält man aus 200 mg (0,35 mmol) der Verbindung 18 durch Umsetzung mit Allyloxy-chloro-N,N-diisopropylamino-phosphit in CH₂Cl₂ nach Chromatographie an Silikagel (Hexan/Essigsäureethylester 1:2) 236 mg (89 %) der Titelverbindung als farblosen Schaum.

¹H-NMR (200 MHz, CDCl₃):u.a. 7,82 [s breit, 1H,HC(6)]; 6,83 [d, J=9,0, 4H, H aromatisch (Trityl)]; 6,43-6,22 [m, 1H,HC(1')]; 5,99-5,75, 5,31-5,05 [2m, 3H, H allyl]; 3,79, 3,78 [2s, 6H, OCH₃]; 1,73, 1,71 [2d, J=1,0, H₃C-C(5)]; 1,25-1,05 [m, 12H, CH(CH₃)₂].

### Beispiel B18:

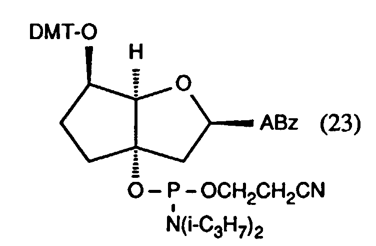

341 mg (0,5 mmol) der Verbindung 19 werden in 4 ml Methylenchlorid gelöst und bei Raumtemperatur der Reihe nach mit 342 µl (2,0 mmol) Ethyldiisopropylamin und 223 µl (1,0 mmol) 2-Cyanethyl-N,N-diisopropylchlorophosphoramidit versetzt. Nach 1 Stunde Reaktionszeit wird das Rohgemisch in 50 ml Essigester aufgenommen und zweimal mit 50 ml NaHCO₃-Lösung extrahiert. Die organische Phase wird über Watte filtriert, eingeengt und der Rückstand mit Essigester an Silikagel chromatographiert. Nach Trocknen am Hochvakuum über Nacht resultieren 348 mg (79 %) der Titelverbindung in Form eines blassgelben Schaumes.

¹H-NMR (200 MHz, CDCl₃):u.a. 8,82, 8,81, 8,51, 8,50 [4s/2H/H-C2',8'); 6,82 (m(dublettoid/4H/Haromatisch (Trityl)); 6,49-6,35 (m/ 1H/H-C1'); 3,75, 3,74, (2s/6H/2O-CH₃); 2,61, 2,58 (2t, J=6,0/CH₂-CN); 1,23-1,10 (m/12H/CH(CH₃)₂].

### Beispiel B18a:

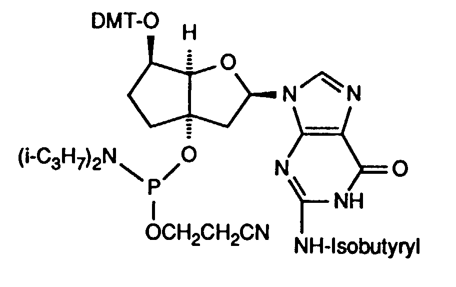

719 mg (1,08 mmol) der Titelverbindung aus B14a werden unter Argon in 10 ml absolutem THF gelöst und der Reihe nach mit 750 µl (4,32 mmol) Diisopropylethylamin und 490 µl (2,16 mmol) 2-Cyanethyl-N,N-Diisopropylchlorophosphoramidit versetzt. Nach 3 Stunden Rühren bei Raumtemperatur wird das Reaktionsgemisch in 25 ml Essigester aufgenommen, mit 20 ml gesättigter NaHCO₃-Lösung extrahiert und die wässrige Phase noch mit 15 ml Essigester rückextrahiert wird. Die vereinigten organischen Phasen werden über MgSO₄ getrocknet, eingeengt und der braune Rückstand über 20 g Kieselgel (Essigester) filiert. Man erhält 880 mg (94 %) der Titelverbindung in Form eines praktisch farblosen Schaumes.

¹H-NMR (400 MHz, CDCl₃): 1,09-1,15 (m, 12H, H₃C(isorpop)); 1,21-1,24 (m, 6H, H₃C(isobut)); 1,42-1,58, 1,82-1,95 (2m, 4H, H-C(6'), H-C(7')); 2,04 (dd, J = 8,8, 13,8, o,4H, H-C(2')); 2,12 (dd, J = 9,1, 14,14, 0,6H, H-C(2')); 2,52-2,59 (m, 1H, H-C(isobut)); 2,62-2,71 (m, 2H, H₂C-CN)); 3,08 (dd, J = 5,3, 15,2, 0,6H, H-C(2')); 3,17-3,21 (m, 0,4H, H-C(2')); 3,48 (d, J = 6,1, 0,4H, H-C(4')); 3,50-3,61 (m, 2H, N-CH(CH₃)₂); 3,62-3,69 (m, 1H, H-C(5')); 3,79, 3,79 (2s; H₃C-O(DMT)); 3,81-3,89 (m, 2H, H₂C-O); 3,92 (dd, J = 2,9, 6,0, 0,6H, H-C(4')); 6,05 (dd, J = 5,2, 8,7, 0,4H, H-C(1')); 6,17 (dd, J = 5,4, 9,0, 0,6H, H-C(1')); 6,80-7,00 (m, 4H, H-C(ar)); 7,18-7,23 (m, 1H, H-C(ar)); 7,27-7,31 (m, 2H, H-C(ar)); 7,39-7,44 (m, 4H, H-C(ar)); 7,51-7,55 (5m, 13H, H-C(ar)); 8,14 (s, 0,6H, H-C(8)); 8,18 (s, 0,4H, H-C(8)); 8,97 (s, 0,6H, H-N(2)); 9,20 (s, 0,4H, H-N(2)); 11,95 (s, br, 1H, H-N(1)).

### C) Herstellung von Oligonukleotiden

### Beispiele C1-C10:

### Herstellung der Festphasengebundenen Nukleoside:

Die tritylierten Nukleoside 17, 18 und 19 werden nach den üblichen Methoden ["Oligonucleotide synthesis, a practical approach" M.J Gait; IRL Press 1984 (Oxford-Washington DC)] mittels einem Bernsteinsäure-linker an long-chain alkylamino-CPG SIGMA® geknüpft. Es werden Beladungsdichten von 20-30 µmol/g Träger erzielt (Tritylassay).

### Oligonukleotid Synthese:

Die Oligonukleotid-Synthese wird auf einem DNA-Synthesizer (Pharmacia LKB Gene Assembler® Plus) gemäss den Standard Protokollen des Herstellers durchgeführt (Owners Manual Gene Assembler® Plus). Folgende Schritte im Synthesezyklus werden modifiziert: Detritylierungsschritt: 45-60 Sekunden

| | |
|---|---|
| Detritylierungsschritt: | 45-60 Sekunden |
| Kopplungsschritt: | 15-20 eq. Phosophoramidit (0.1M in CH₃CN), |
| | 180 eq. Tetrazol 6 min. |

Gemäss Tritylassay werden generell Kopplungsausbeuten von mehr als 97 % pro Schritt erzielt. Die Sequenzen werden im Falle der cyanoethoxy-Phosphoramidite nach üblichen Methoden entschützt und vom Träger gelöst ["Oligonucleotide synthesis, a practical approach" M.J Gait; IRL Press 1984 (Oxford-Washington DC)]. Im Falle der allyloxy-Phosphoramidite erfolgte Entschützung und Loslösung vom Trägermaterial nach (Y. Hayakawa, S. Wakabayashi, H. Kato and R. Noyori J. Am. Chem. Soc. 1990, 112, 1691).

### Reinigung der Oligonukleotidsequenzen:

Die Oligonukleotide werden mittels HPLC auf dem System 1 getrennt und nach Entsalzung auf System 2 zur Kontrolle injiziert.
- System 1:: Vorsäule: Nucleogen guard column, 30x4.0 mm, ID (Macherey Nagel);
Säule: Nucelogen DEAE 60-7, 125x4.0 mm (Macherey Nagel)
A: 20 mM KH₂PO₄ in H₂O/CH₃CN 4. 1, pH 6.0
B: 1M KCl in Puffer A, pH 6.0
Fluss 1 ml/min.
- System 2:: Vorsäule: Rp-8 Newguard, 15x3.2 mm, 7 µm (Brownlee Labs);
Säule: Aquapore Rp-300, 220x4.6 mm, 7 µm (Brownlee Labs)
A: 0.1M Triethylammoniumacetat (TEAOAc) in H₂O, pH 7.0
B: 0.1M TEAOAc in H₂O/CH₃CN 1:4, pH 7.0
Fluss 1 ml/min.
Die Detektion erfolgt UV-spektrometrich bei 260 nm.

Beispiel C1: Aus den Verbindungen (17) und (20) wird ein Oligonukleotid mit 6 Monomereinheiten hergestellt.

Beispiel C2: Aus den Verbindungen (18) und (22) wird ein Oligonukleotid mit 10 Monomereinheiten hergestellt.

Beispiel C3: Aus den Verbindungen (19) und (23) wird ein Oligonukleotid mit 10 Monomereinheiten hergestellt.

Beispiel C4: Aus den Verbindungen aus Beispiel B14a und B18a wird ein Oligonukleotid mit 6 Monomereinheiten hergestellt.

Beispiel C5: Aus den Verbindungen aus Beispiel B14a und B18a sowie den Verbindungen 22 und 23 wird ein Oligonukleotid mit 9 Monomereinheiten der Sequend 5'-GGA TGG GAG-3' hergestellt.

Beispiel C6: Aus den Verbindungen 17, 20, 22 und 23 wird ein Oligonukleotid mit 9 Monomereinheiten der Sequenz 5'-CTC CCA TCC-3' hergestellt.

Beispiel C7: Aus der Verbindung 20 und konventionellen 2'-Cyanoethyldiisopropylaminophosphoramidit-Bausteinen der natürlichen 2'-Deoxyribonucleoside sowie entsprechend Nucleosid-modifizierter Festphase der Firma Pharmacia® wird ein chimäres Oligonucleotid mit 18 Monomereinheiten der Sequenz 5'-CTC GTA CC*C TTC CGG TCC-3' hergestellt, wobei *C die Position des Nucleosids gemäss Verbindung 20 angibt.

Beispiel C8: Aus der Verbindung 23 und konventionellen 2'-Cyanoethyldiisopropylaminophosphoramidit-Bausteinen der natürlichen 2'-Deoxyribonucleoside sowie entsprechend Nucleosid-modifizierter Festphase der Firma Pharmacia® wird ein chimäres Oligonucleotid mit 18 Monomereinheiten der Sequenz 5'-CTC GTA CC*A TTC CGG TCC-3' hergestellt, wobei *A die Position des Nucleosids gemäss Verbindung 23 angibt.

Beispiel C9: Aus der Verbindung 22 und konventionellen 2'-Cyanoethyldiisopropylaminophosphoramidit-Bausteinen der natürlichen 2'-Deoxyribonucleoside sowie entsprechend Nucleosid-modifizierter Festphase der Firma Pharmacia® wird ein chimäres Oligonucleotid mit 18 Monomereinheiten der Sequenz 5'-CTC GTA CC*T TTC CGG TCC-3' hergestellt, wobei *T die Position des Nucleosids gemäss Verbindung 22 angibt.

Beispiel C10: Aus der Verbindung 20 und konventionellen 2'-Cyanoethyldiisopropylaminophosphoramidit-Bausteinen der natürlichen 2'-Deoxyribonucleoside sowie entsprechend Nucleosid-modifizierter Festphase der Firma Pharmacia® wird ein chimäres Oligonucleotid mit 18 Monomereinheiten der Sequenz 5'-CGA CTA TGC AA*C *C*C*C-3' hergestellt, wobei *C die Positonen der Nucleoside gemäss Verbindung 20 angibt.

### Trennung der Oligonukleotide:

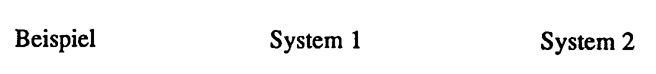

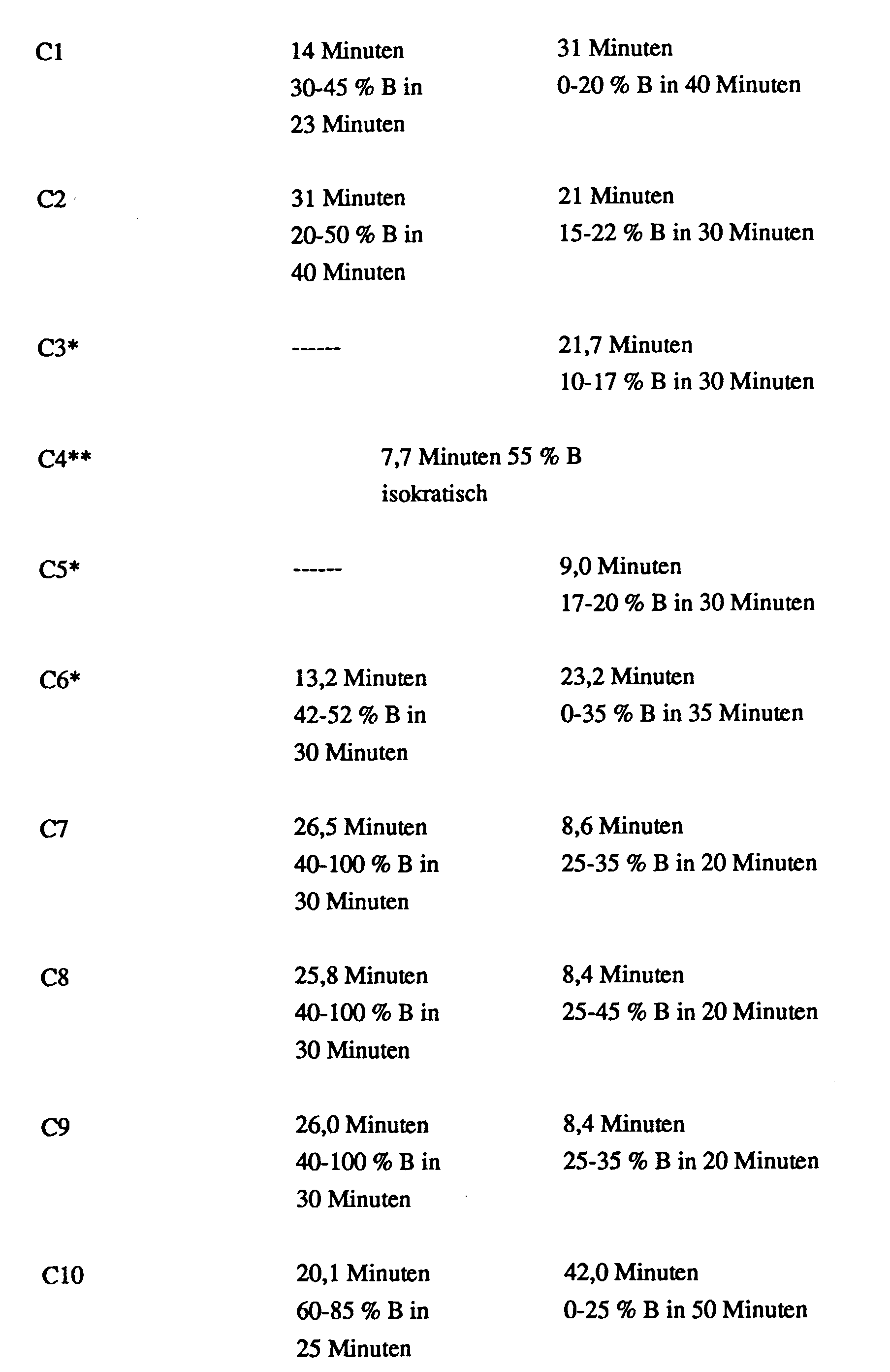

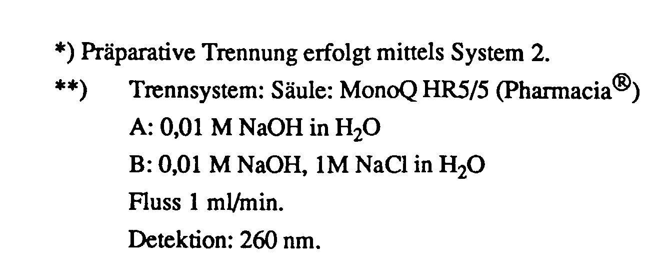

### D) Anwendungsbeisiele

### Beispiel D1: Wechselwirkung der Oligonukleotide gemäss den Beispielen C2 und C3 mit Polynukleotiden.

Die Wechselwirkung der Oligonukleotide gemäss den Beispielen C2 und C3 mit den entsprechenden basenkomplemetären Oligo- und Polymeren der natürlichen Desoxy- und Ribonukleoside werden durch das Aufzeichnen von UV-Schmelzkurven und den daraus ermittelten Tₘ-Werten charakterisiert. Diese Standardmethode ist zum Beispiel von L.A. Marky et al. in Biopolymers, Band 26, Seiten 1601 ff (1987) beschrieben. Es wird eine Lösung der Oligonukleotide gemäss den Beispielen C2 und C3 und der entsprechenden basenkomplementären natürlichen Oligo- beziehungsweise Polynukleotide (siehe Tabelle 1, c = 45 µM) in 10 mM Tris.HCl, 0,15 M NaCl, pH = 7,0 hergestellt, und die Änderung der Extinktion bei 260 nm in Abhängigkeit von der Temperatur (0 bis 70 °C) aufgezeichnet. Aus den erhaltenen Schmelzkurven wird der Tₘ-Wert ermittelt (siehe Tabelle 1).

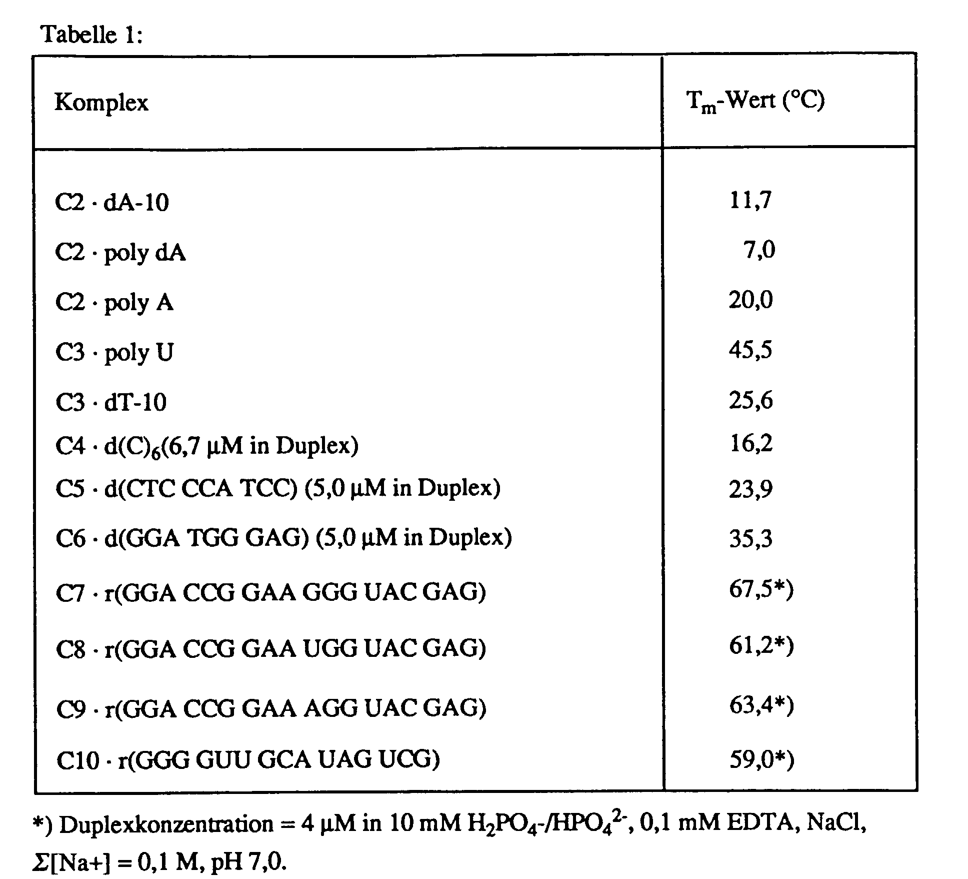

### Beispiel D2: Enzymatische Hydrolyse des Oligonukleotids gemäss Beispiel C2.

Je 0,15 O.D.²⁶⁰-Einheiten des Decanukleotids gemäss Beispiel C2 beziehungsweise des natürlichen Oligomeren dT-10 als Standard in 100 µl des entsprechenden Puffers A, B oder C werden bei 37 °C mit der jeweils angegebenen Menge des entsprechenden Enzyms inkubiert. Nach der angegebenen Zeit wird die Reaktion abgebrochen, das Reaktionsgemisch durch HPLC chromatographisch aufgetrennt und die Spaltprodukte durch Peakflächenintegration bei 260 nm quantifiziert. In Tabelle 2 ist jeweils der prozentuale Anteil der Summe aller Spaltprodukte relativ zur gesamten Menge an Oligonukleotiden angegeben.

Puffer A (Schlangengift PDE): 0,1 M Tris·HCl, pH 8,5.

Puffer B (Nuklease S1): 33 mM Na-Acetat, 50 mM NaCl, 0,03 mM ZnSO₄, ph 4,5.

Puffer C (Kalbsmilz PDE): 0,1 M Ammoniumacetat, pH 6,5.

Die Ergebnisse sind in nachfolgender Tabelle 2 aufgeführt:

**Tabelle 2:**

| Enzym | Standard-Decamer (dT-10) | Beispiel C2 | Verhältnis dT-10:C2 |
|---|---|---|---|
| Schlangengift PDE* (EC 3.1.15.1) 1,5 x 10⁻⁴ U (3'-Exonuklease) | 95 % (5 Minuten) | 28 % (5 Minuten) | 3,4 |
| Nuklease S1 (EC 3.1.30.1) 10 µ (Exo/Endonuklease) | 100 % (5 Minuten) | 0 (5 Minuten) 20 % (60 Minuten) | ≥ 100 |
| Kalbsmilz PDE (EC 3.1.16.1) a) 0,024 U b) 0,24 U (5'-Exonuklease) | a) 91 % (5 Minuten) | b) 12 % (16 Stunden) | 2 x 10³ |

| | | | |
|---|---|---|---|
| * Zusätzlich 5 µ alkalische Phosphatase (EC 3.1.3.1) | | | |

## Patentansprüche

1. Verbindungen der Formel I in Form ihrer Racemate oder Enantiomeren, 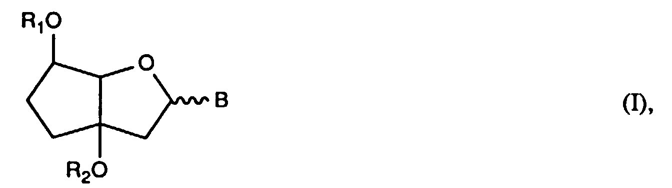 worin R₁ und R₂ unabhängig voneinander für Wasserstoff oder eine Schutzgruppe stehen und B einen Purin- oder Pyrimidinrest oder ein Analoges davon bedeutet.

2. Verbindungen der Formel I gemäss Anspruch 1, worin R₁ und R₂ je für Wasserstoff stehen.

3. Verbindungen der Formel I gemäss Anspruch 1, worin R₁ und R₂ unabhängig voneinander lineares oder verzweigtes C₁-C₈-Alkyl, C₇-C₁₂-Aralkyl, Triphenylsilyl, Alkyldiphenylsilyl, Dialkylphenylsilyl und Trialkylsilyl mit 1 bis 20 C-Atomen in den Alkylgruppen, C₂-C₁₂-Acyl, R₃-SO₂-, worin R₂ C₁-C₁₂-Alkyl, C₅- oder C₆-Cycloalkyl, Phenyl, Benzyl, C₁-C₁₂-Alkylphenyl, C₁-C₁₂-Alkylbenzyl, oder Halogenphenyl oder Halogenbenzyl bedeutet, oder C₁-C₁₂-Alkoxycarbonyl, Phenyloxycarbonyl, Benzyloxycarbonyl, Methyl- oder Methoxy- oder Chlorphenyloxycarbonyl oder -benzyloxycarbonyl darstellen.

4. Verbindungen der Formel I gemäss Anspruch 3, worin R₁ und R₂ unabhängig voneinander lineares oder verzweigtes C₁-C₄-Alkyl, C₇-C₁₂-Aralkyl, Trialkylsilyl mit 1 bis 12 C-Atomen in den Alkylgruppen, C₂-C₈-Acyl, R₃-SO₂-, worin R₃ C₁-C₆-Alkyl, Phenyl, Benzyl, C₁-C₄-Alkylphenyl, C₁-C₄-Alkylbenzyl, oder Halogenphenyl oder Halogenbenzyl bedeutet, oder C₁-C₈-Alkoxycarbonyl, Phenoxycarbonyl oder Benzyloxycarbonyl darstellen.

5. Verbindungen der Formel I gemäss Anspruch 1, worin R₁ und R₂ gleiche Schutzgruppen bedeuten.

6. Verbindungen der Formel I gemäss Anspruch 1, worin R₁ und R₂ Methyl, Ethyl, n- und i-Propyl, n-, i- und t-Butyl; Benzyl, Methylbenzyl, Dimethylbenzyl, Methoxybenzyl, Dimethoxybenzyl, Brombenzyl; Diphenylmethyl, Di(methylphenyl)methyl, Di(dimethylphenyl)methyl, Di(methoxyphenyl)methyl, Di(dimethoxyphenyl)methyl, Trityl, Tri(methylphenyl)methyl, Tri(dimethylphenyl)methyl, Tri(methoxyphenyl)methyl, Tri(dimethoxyphenyl)methyl; Trimethylsilyl, Triethylsilyl, Tri-n-propylsilyl, i-Propyl-dimethylsilyl, t-Butyl-dimethylsilyl, t-Butyl-diphenylsilyl, n-Octyl-dimethylsilyl, (1,1,2,2-Tetramethylethyl)-dimethylsilyl; Acetyl, Propanoyl, Butanoyl, Pentanoyl, Hexanoyl, Benzoyl, Methylbenzoyl, Methoxybenzoyl, Chlorbenzoyl und Brombenzoyl; Methyl-, Ethyl-, Propyl-, Butyl-, Phenyl-, Benzyl-, p-Brom-, p-Methoxy- und p-Methylphenylsulfonyl; Methoxy-, Ethoxy-, n- oder i-Propoxy- oder n-, i- oder t-Butoxycarbonyl, oder Phenyloxycarbonyl, Benzyloxycarbonyl, Methyl- oder Methoxy- oder Chlorphenyloxycarbonyl oder -benzyloxycarbonyl darstellen.

7. Verbindungen der Formel I gemäss Anspruch 1, worin B als Purinrest oder einem Analogen davon einen Rest der Formeln II, IIa, IIb, IIc, IId oder IIe darstellt, 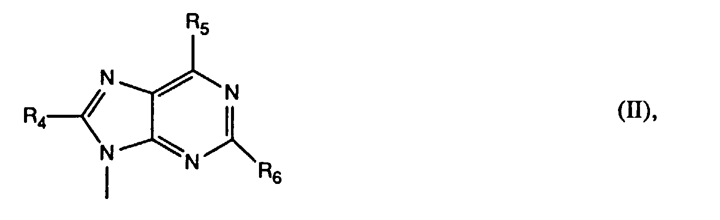 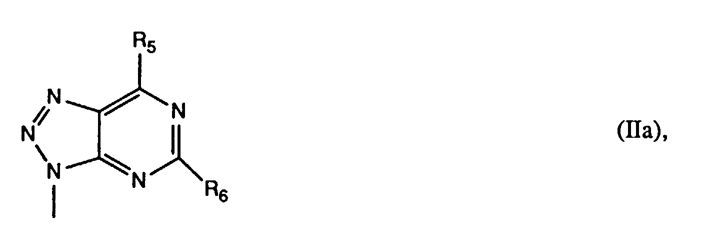 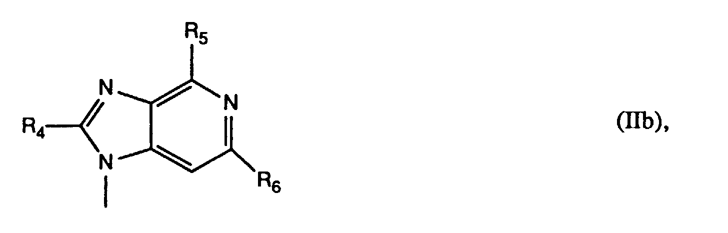 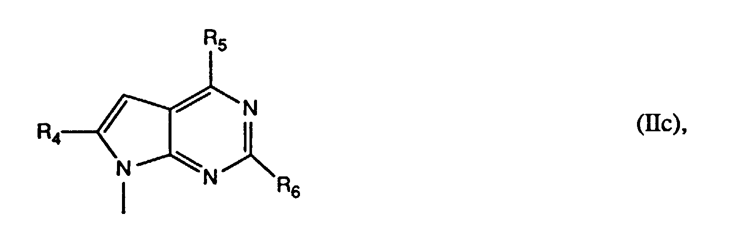 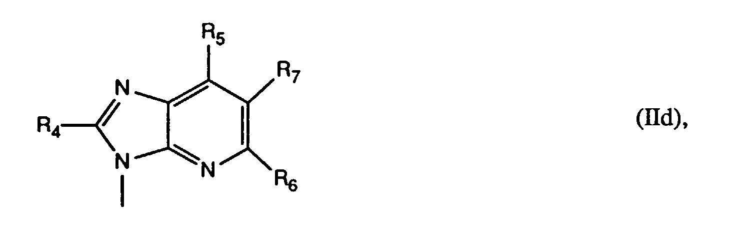 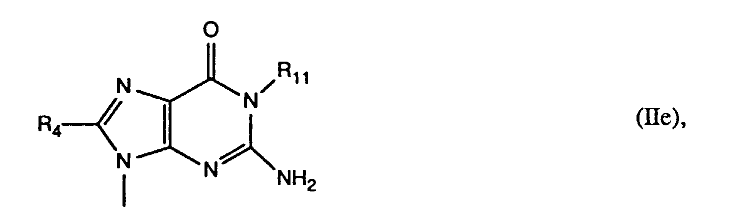 worin R₄ für H, Cl, Br oder OH steht, und R₅, R₆ und R₇ unabhängig voneinander H, OH, SH, NH₂, NHNH₂, NHOH, NHOAlkyl mit 1 bis 12 C-Atomen, F, Cl, Br, Alkyl oder Hydroxyalkyl oder Aminoalkyl oder Alkoxy oder Alkylthio mit 1 bis 12 C-Atomen, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, und R₁₁ H oder C₁-C₄-Alkyl darstellt.

8. Verbindungen der Formel I gemäss Anspruch 7, worin die Schutzgruppe für Hydroxyl- und Aminogruppen C₁-C₈-Acyl darstellt.

9. Verbindungen der Formel I gemäss Anspruch 7, worin Primäramino 1 bis 12 C-Atome und das Sekundäramino 2 bis 12 C-Atome enthält.

10. Verbindungen der Formel I gemäss Anspruch 7, worin es sich bei dem Primäramino und Sekundäramino um Reste der Formel R₈R₉N handelt, worin R₈ für H steht oder unabhängig die Bedeutung von R₉ hat, und R₉ C₁-C₂₀-Alkyl, -Aminoalkyl, -Hydroxyalkyl; Carboxyalkyl oder Carbalkoxyalkyl, wobei die Carbalkoxygruppe 2 bis 8 C-Atome enthält und die Alkylgruppe 1 bis 6, bevorzugt 1 bis 4 C-Atome; C₂-C₂₀-Alkenyl; Phenyl, Mono- oder Di-(C₁-C₄-Alkyl- oder Alkoxy)phenyl, Benzyl, Mono- oder Di-(C₁-C₄-Alkyl- oder -Alkoxy)benzyl; oder 1,2-, 1,3- oder 1,4-Imidazolyl-C₁-C₆-Alkyl darstellt, oder R₈ und R₉ zusammen Tetra- oder Pentamethylen, 3-Oxa-1,5-pentylen, -CH₂-NR₁₀-CH₂CH₂- oder -CH₂CH₂-NR₁₀-CH₂CH₂- darstellen, worin R₁₀ für H oder C₁-C₄-Alkyl steht, wobei die Aminogruppe im Aminoalkyl unsubstituiert oder mit ein oder zwei C₁-C₄-Alkyl oder -Hydroxyalkylgruppen substituiert ist, und die Hydroxylgruppe im Hydroxyalkyl gegebenenfalls mit C₁-C₄-Alkyl verethert ist.

11. Verbindungen der Formel I gemäss Anspruch 9, worin es sich bei dem Primäramino und Sekundäramino um Methyl-, Ethyl-, Dimethyl-, Diethyl-, Allyl-, Mono- oder Di-(hydroxy-eth-2-yl)-, Phenyl- und Benzyl-, Acetyl- und Benzoylamino.

12. Verbindungen der Formel I gemäss Anspruch 7, worin R₄ in den Formeln II, IIb, IIc, IId und IIe für Wasserstoff steht.

13. Verbindungen der Formel I gemäss Anspruch 7, worin R₇ in Formel IId für Wässerstoff steht.

14. Verbindungen der Formel I gemäss Anspruch 7, worin R₅ und R₆ in den Formeln II, IIa, IIb, IIc, IId und IIe unabhängig voneinander H, F, Cl, Br, OH, SH, NH₂, NHOH, NHNH₂, Methylamino, Dimethylamino, Benzoylamino, Methoxy, Ethoxy und Methylthio darstellen.

15. Verbindungen der Formel I gemäss Anspruch 1, worin B ein Purinrest oder ein Rest eines Purinanalogen aus der Reihe Adenin, N-Methyladenin, N-Benzoyladenin, 2-Methyladenin, 2-Methylthioadenin, 2-Aminoadenin, 3-Carbaadenin, 7-Carbaadenin, 1-Carbaadenin, 6-Hydroxypurin, 2-Amino-6-chlorpurin, 2-Amino-6-methylthiopurin, 2-Amino-6-hydroxypurin, 3-Carba-6-chlorpurin, Guanin, 2-Methylguanin.

16. Verbindungen der Formel I gemäss Anspruch 1, worin B in Formel I als analoger Pyrimidinrest ein Uracil-, Thymin- oder Cytosinrest der Formeln III, IIIa und IIIb darstellt, 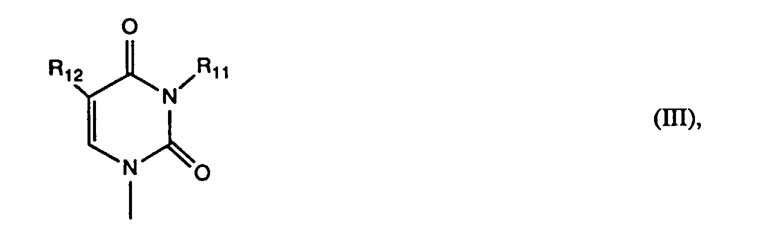 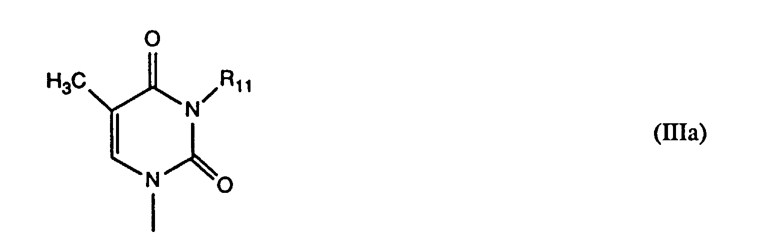 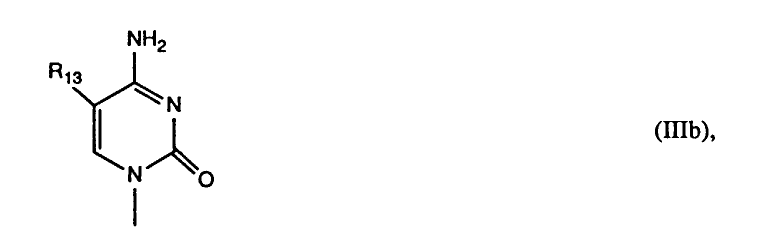 worin R₁₁ H oder C₁-C₄-Alkyl bedeutet, und R₁₂ und R₁₃ unabhängig voneinander H, OH, SH, NH₂, NHNH₂, NHOH, NHOAlkyl mit 1 bis 12 C-Atomen, F, Cl, Br, Alkyl oder Hydroxyalkyl oder Aminoalkyl oder Alkoxy oder Alkylthio mit 1 bis 12 C-Atomen, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, und die Wasserstoffatome der NH₂-Gruppe in Formel IIIb unsubstituiert oder mit C₁-C₆-Alkyl, Benzoyl oder Benzyl substituiert sind, sowie die Dihydroderivate der Reste der Formeln III, IIIa und IIIb.

17. Verbindungen der Formel I gemäss Anspruch 16, worin R₁₂ H, C₁-C₆-Alkyl oder -Hydroxyalkyl, F, Cl, Br, NH₂, Benzoylamino, Mono- oder Di-C₁-C₆-alkylamino darstellt.

18. Verbindungen der Formel I gemäss Anspruch 16, worin R₁₃ H, C₁-C₆-Alkyl oder -Alkoxy oder -Hydroxyalkyl, F, Cl, Br, NH₂, Benzoylamino, Mono- oder Di-C₁-C₆-alkylamino darstellt.

19. Verbindungen der Formel I gemäss Anspruch 17, worin R₁₂ H, F, Cl, Br, NH₂, NHCH₃, N(CH₃)₂ oder C₁-C₄-Alkyl bedeutet.

20. Verbindungen der Formel I gemäss Anspruch 18, worin R₁₃ H, C₁-C₄-Alkyl, NH₂, NHCH₃ oder (CH₃)₂N dar bedeutet.

21. Verbindungen der Formel I gemäss Anspruch 1, worin B als Rest eines Pyrimidinanalogen sich von Uracil, Thymin, Cytosin, 5-Fluoruracil, 5-Chloruracil, 5-Bromuracil, Dihydrouracil, Pseudouracil, 1-Methylpseudouracil, 5-Methyluracil, 3-Methylcytosin und 5-Methylcytosin ableitet.

22. Verbindungen der Formel I gemäss Anspruch 1, dadurch gekennzeichnet, dass sie den *α-* und β-Anomeren der Formel IV entsprechen, 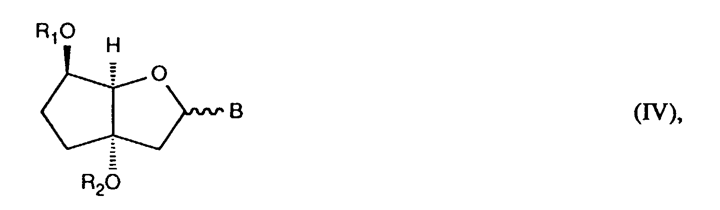 worin R₁, R₂ und B die in Anspruch 1 angegebenen Bedeutungen haben.

23. Verbindungen der Formel I gemäss Anspruch 22, dadurch gekennzeichnet, dass sie den β-Anomeren der Formel IVa entsprechen, 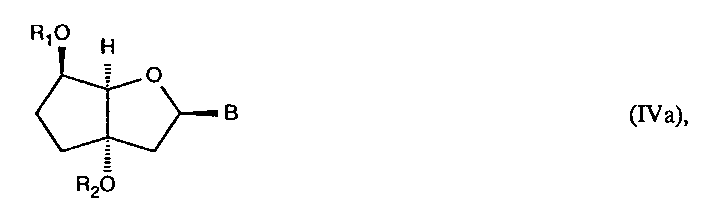 worin R₁, R₂ und B die in Anspruch 1 angegebenen Bedeutungen haben.

24. Verfahren zur Herstellung von Verbindungen der Formeln I, IV oder IVa gemäss einem der Ansprüche 1 bis 23, dadurch gekennzeichnet, dass man eine Verbindung der Formel V in Form ihrer Racemate oder Enantiomeren, 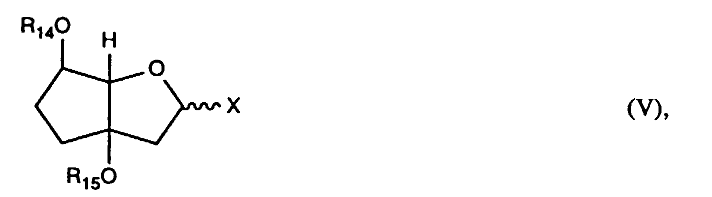 worin R₁₄ und R₁₅ für gleiche oder verschiedene Schutzgruppen stehen und X eine Abgangsgruppe bedeutet, mit einem Purin, Purinanalogen oder Pyrimidinanalogen umsetzt und anschliessend gegebenenfalls die Schutzgruppen entfernt.

25. Verfahren gemäss Anspruch 24, dadurch gekennzeichnet, dass es sich bei der Abgangsgruppe um Halogen, C₁-C₆-Alkoxy, C₁-C₈-Acyloxy oder um R₁₆SO₃ handelt, worin R₁₆ C₁-C₆-Alkyl oder -Halogenalkyl, unsubstituiertes oder mit ein bis drei Halogen, C₁-C₄-Alkyl oder -Alkoxy substituiertes Phenyl oder Benzyl bedeutet.

26. Verbindungen der Formeln V und Va in Form ihrer Racemate oder Enantiomeren, 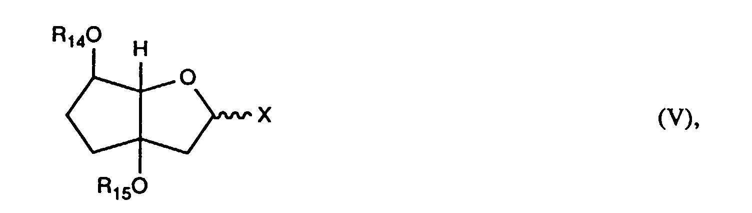 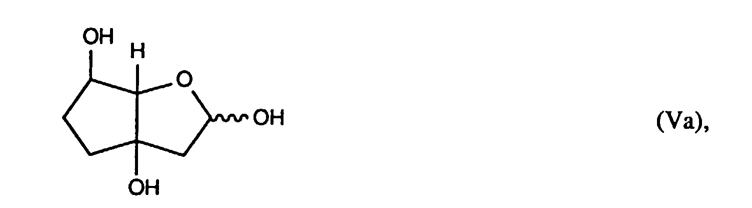 worin R₁₄ und R₁₅ für gleiche oder verschiedene Schutzgruppen stehen und X eine Abgangsgruppe bedeutet.

27. Verbindungen der Formeln V und Va gemäss Anspruch 26, worin es sich bei der Abgangsgruppe um Halogen, C₁-C₆-Alkoxy, C₁-C₈-Acyloxy oder um R₁₆SO₃ handelt, worin R₁₆ C₁-C₆-Alkyl oder -Halogenalkyl, unsubstituiertes oder mit ein bis drei Halogen, C₁-C₄-Alkyl oder -Alkoxy substituiertes Phenyl oder Benzyl bedeutet.

28. Verbindungen der Formeln V und Va gemäss Anspruch 27, worin es sich bei der Abgangsgruppe um CH₃C(O)O handelt.

29. Verbindungen gemäss Anspruch 26, dadurch gekennzeichnet, dass sie den Enantiomeren der Formeln VI und VIa entsprechen, 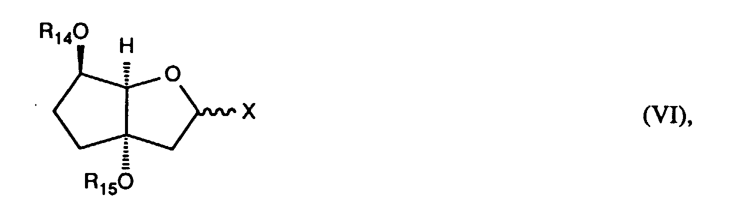 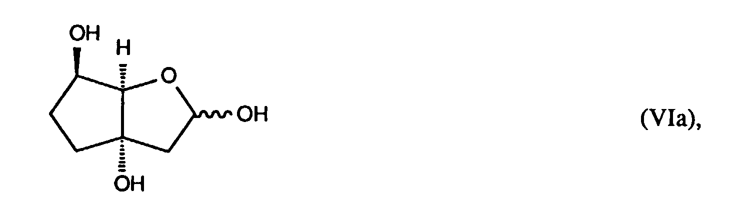 worin R₁₄, R₁₅ und X die in Anspruch 25 angegebenen Bedeutungen haben.

30. Verwendung der Verbindungen der Formel I gemäss Anspruch 1 in Form von Racematen oder Enantiomeren zur Herstellung von Oligonukleotiden, die gleiche oder verschiedene Monomereinheiten von Verbindungen der Formel I oder Monomereinheiten von anderen Nukleosiden enthalten, wobei die Oligonukleotide 2 bis 200 Monomereinheiten enthalten.

31. Verwendung gemäss Anspruch 30 zur Herstellung von Oligonukleotiden mit 2 bis 100 Monomereinheiten.

32. Verwendung gemäss Anspruch 30 zur Herstellung von Oligonukleotiden mit 2 bis 50 Monomereinheiten.

33. Verwendung gemäss Anspruch 30 zur Herstellung von Oligonukleotiden mit 2 bis 20 Monomereinheiten.

34. Verwendung gemäss Anspruch 30 zur Herstellung von Oligonukleotiden mit gleichen Monomereinheiten von Verbindungen der Formel I.

35. Oligonukleotide der Formel VII 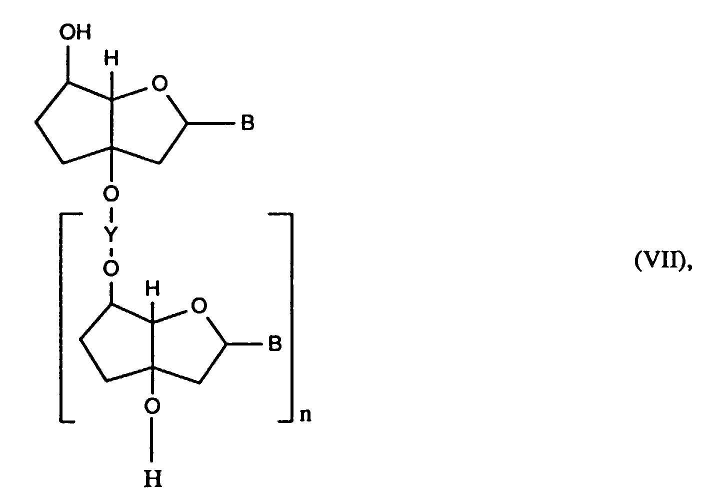 worin B einen Purin- oder Pyrimidinrest oder ein Analoges davon bedeutet, n für eine Zahl von 2 bis 200 steht und Y für eine Nukleotid-Brückengruppe steht.

36. Oligonukleotide der Formel VII gemäss Anspruch 35, dadurch gekennzeichnet, dass es sich bei der Brückengruppe um -P(O)O^{⊖}-, -P(O)S^{⊖}-, -P(S)S^{⊖}-, -P(O)R₁₇-, -P(O)OR₁₈-, -P(O)NR₁₉R₂₀-, -CO- oder -CON(R₁₈)₂- handelt, worin R₁₇ H oder C₁-C₆-Alkyl darstellt und R₁₈ C₁-C₆-Alkyl bedeutet und R₁₉ und R₂₀ unabhängig voneinander die Bedeutung von R₁₇ haben.

37. Oligonukleotide der Formel VII gemäss Anspruch 35, dadurch gekennzeichnet, dass es sich bei der Brückengruppe um -P(O)O^{⊖}- handelt.

38. Oligonukleotide der Formel VII gemäss Anspruch 35, dadurch gekennzeichnet, dass n für eine Zahl von 2 bis 100 steht.

39. Oligonukleotide der Formel VII gemäss Anspruch 35, dadurch gekennzeichnet, dass n für eine Zahl von 2 bis 50 steht.

40. Oligonukleotide der Formel VII gemäss Anspruch 35, dadurch gekennzeichnet, dass n für eine Zahl von 2 bis 20 steht.

41. Oligonukleotide der Formel VII gemäss Anspruch 35, worin B als Purinrest oder einem Analogen davon einen Rest der Formeln II, IIa, IIb, IIc, IId oder IIe darstellt, 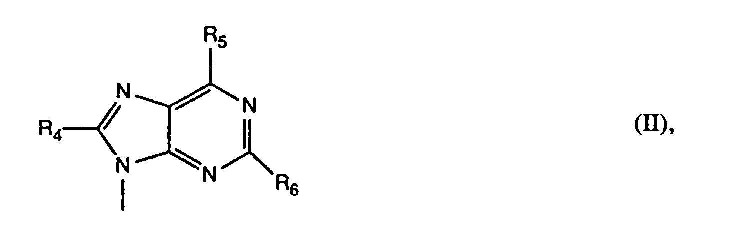 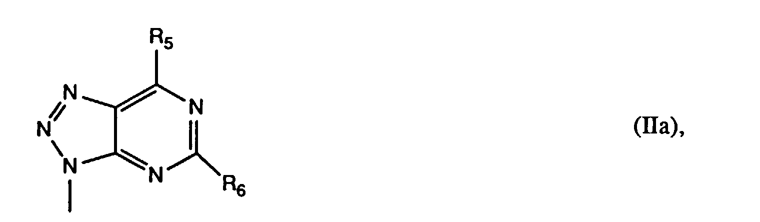 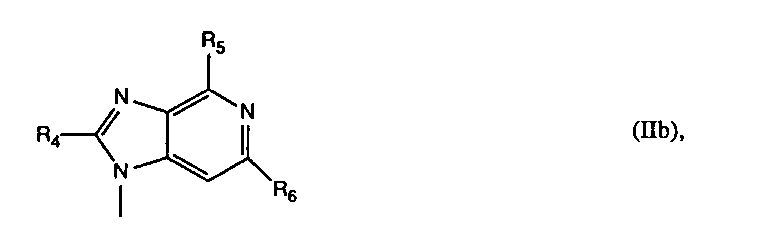 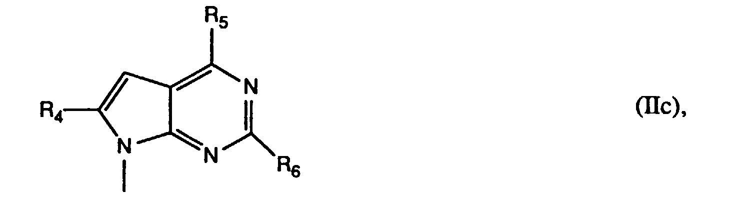 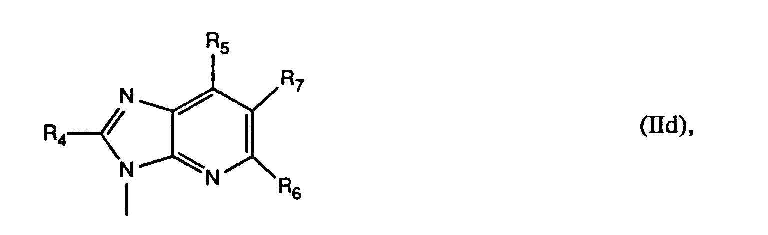 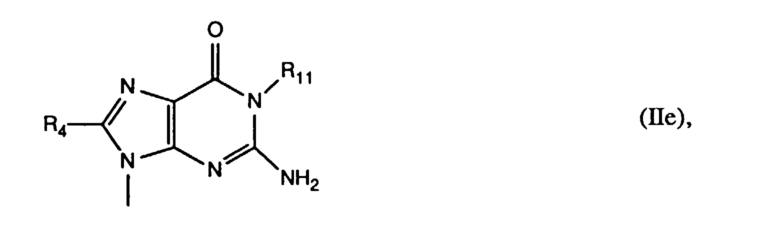 worin R₄ für H, Cl, Br oder OH steht, und R₅, R₆ und R₇ unabhängig voneinander H, OH, SH, NH₂, NHNH₂, NHOH, NHOAlkyl mit 1 bis 12 C-Atomen, F, Cl, Br, Alkyl oder Hydroxyalkyl oder Aminoalkyl oder Alkoxy oder Alkylthio mit 1 bis 12 C-Atomen, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, und R₁₁ H oder C₁-C₄-Alkyl darstellt.

42. Oligonukleotide der Formel VII gemäss Anspruch 41, worin die Schutzgruppe für Hydroxyl- und Aminogruppen C₁-C₈-Acyl darstellt.

43. Oligonukleotide der Formel VII gemäss Anspruch 41, worin das Primäramino 1 bis 12 C-Atome und das Sekundäramino 2 bis 12 C-Atome enthält.

44. Oligonukleotide der Formel VII gemäss Anspruch 41, worin es sich bei dem Primäramino und Sekundäramino um Reste der Formel R₈R₉N handelt, worin R₈ für H steht oder unabhängig die Bedeutung von R₉ hat, und R₉ C₁-C₂₀-Alkyl, -Aminoalkyl, -Hydroxyalkyl; Carboxyalkyl oder Carbalkoxyalkyl, wobei die Carbalkoxygruppe 2 bis 8 C-Atome enthält und die Alkylgruppe 1 bis 6, bevorzugt 1 bis 4 C-Atome; C₂-C₂₀-Alkenyl; Phenyl, Mono- oder Di-(C₁-C₄-Alkyl- oder Alkoxy)phenyl, Benzyl, Mono- oder Di-(C₁-C₄-Alkyl- oder -Alkoxy)benzyl; oder 1,2-, 1,3- oder 1,4-Imidazolyl-C₁-C₆-Alkyl darstellt, oder R₈ und R₉ zusammen Tetra- oder Pentamethylen, 3-Oxa-1,5-pentylen, -CH₂-NR₁₀-CH₂CH₂- oder -CH₂CH₂-NR₁₀-CH₂CH₂- darstellen, worin R₁₀ für H oder C₁-C₄-Alkyl steht, wobei die Aminogruppe im Aminoalkyl unsubstituiert oder mit ein oder zwei C₁-C₄-Alkyl oder -Hydroxyalkylgruppen substituiert ist, und die Hydroxylgruppe im Hydroxyalkyl gegebenenfalls mit C₁-C₄-Alkyl verethert ist.

45. Oligonukleotide der Formel VII gemäss Anspruch 44, worin es sich bei dem Primäramino und Sekundäramino um Methyl-, Ethyl-, Dimethyl-, Diethyl-, Allyl-, Mono- oder Di-(hydroxy-eth-2-yl)-, Phenyl- und Benzyl-, Acetyl- und Benzoylamino handelt.

46. Oligonukleotide der Formel VII gemäss Anspruch 41, worin R₄ in den Formeln II, IIb, IIc, IId und IIe für Wasserstoff steht.

47. Oligonukleotide der Formel VII gemäss Anspruch 41, worin R₇ in Formel IId für Wasserstoff steht.

48. Oligonukleotide der Formel VII gemäss Anspruch 41, worin R₅ und R₆ in den Formeln II, IIa, IIb, IIc, IId und IIe unabhängig voneinander H, F, Cl, Br, OH, SH, NH₂, NHOH, NHNH₂, Methylamino, Dimethylamino, Benzoylamino, Methoxy, Ethoxy und Methylthio darstellen.

49. Oligonukleotide der Formel VII gemäss Anspruch 41, worin B ein Purinrest oder ein Rest eines Purinanalogen Reihe Adenin, N-Methyladenin, N-Benzoyladenin, 2-Methyladenin, 2-Methylthioadenin, 2-Aminoadenin, 3-Carbaadenin, 7-Carbaadenin, 1-Carbaadenin, 6-Hydroxypurin, 2-Amino-6-chlorpurin, 2-Amino-6-methylthiopurin, 2-Amino-6-hydroxypurin, 3-Carba-6-chlorpurin, Guanin, 2-Methylguanin.

50. Oligonukleotide der Formel VII gemäss Anspruch 35, worin B in Formel VII als analoger Pyrimidinrest ein Uracil-, Thymin- oder Cytosinrest der Formeln III, IIIa und IIIb darstellt, 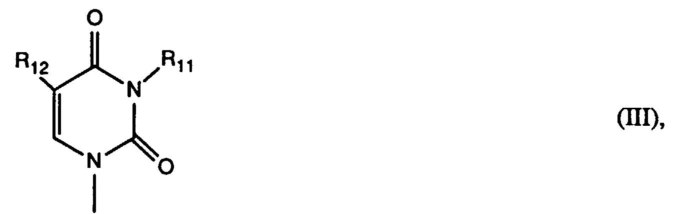 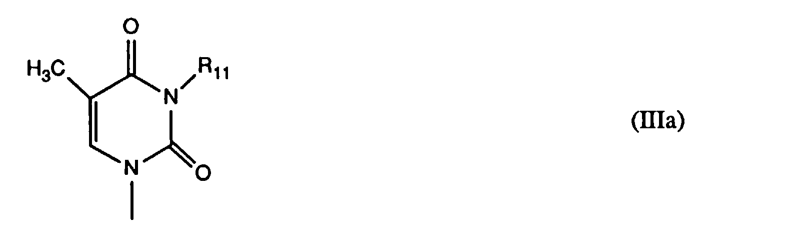 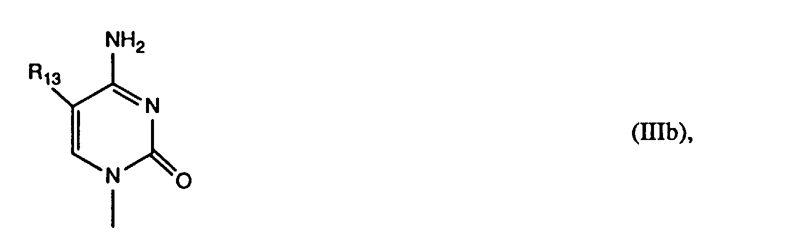 worin R₁₁ H oder C₁-C₄-Alkyl bedeutet, und R₁₂ und R₁₃ unabhängig voneinander H, OH, SH, NH₂, NHNH₂, NHOH, NHOAlkyl mit 1 bis 12 C-Atomen, F, Cl, Br, Alkyl oder Hydroxyalkyl oder Aminoalkyl oder Alkoxy oder Alkylthio mit 1 bis 12 C-Atomen, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, und die Wasserstoffatome der NH₂-Gruppe in Formel IIIb unsubstituiert oder mit C₁-C₆-Alkyl, Benzoyl oder Benzyl substituiert sind, sowie die Dihydroderivate der Reste der Formeln III, IIIa und IIIb.

51. Oligonukleotide der Formel VII gemäss Anspruch 50, worin R₁₂ H, C₁-C₆-Alkyl oder -Hydroxyalkyl, F, Cl, Br, NH₂, Benzoylamino, Mono- oder Di-C₁-C₆-alkylamino darstellt.

52. Oligonukleotide der Formel VII gemäss Anspruch 50, worin R₁₃ H, C₁-C₆-Alkyl oder -Alkoxy oder -Hydroxyalkyl, F, Cl, Br, NH₂, Benzoylamino, Mono- oder Di-C₁-C₆-alkylamino darstellt.

53. Oligonukleotide der Formel VII gemäss Anspruch 51, worin R₁₂ H, F, Cl, Br, NH₂, NHCH₃, N(CH₃)₂ oder C₁-C₄-Alkyl bedeutet.

54. Oligonukleotide der Formel VII gemäss Anspruch 52, worin R₁₃ H, C₁-C₄-Alkyl, NH₂, NHCH₃ oder (CH₃)₂N dar bedeutet.

55. Oligonukleotide der Formel VII gemäss Anspruch 50, worin B als Rest eines Pyrimidinanalogen sich von Uracil, Thymin, Cytosin, 5-Fluoruracil, 5-Cloruracil, 5-Bromuracil, Dihydrouracil, Pseudouracil, 1-Methylpseudouracil, 5-Methyluracil, 3-Methylcytosin und 5-Methylcytosin ableitet.

56. Oligonukleotide der Formel VII gemäss Anspruch 35, dadurch gekennzeichnet, dass sie der Formel VIIa entsprechen, 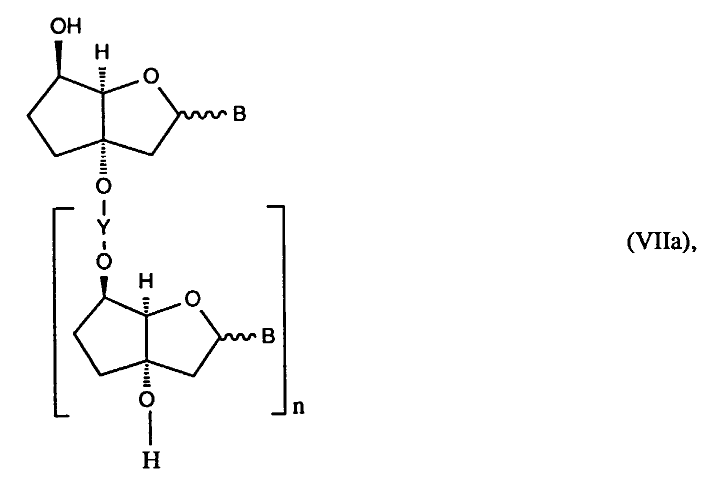 worin B, Y und n die in Anspruch 35 angegebenen Bedeutungen haben.

57. Oligonukleotide der Formel VII gemäss Anspruch 35, dadurch gekennzeichnet, dass sie der Formel VIIb entsprechen, 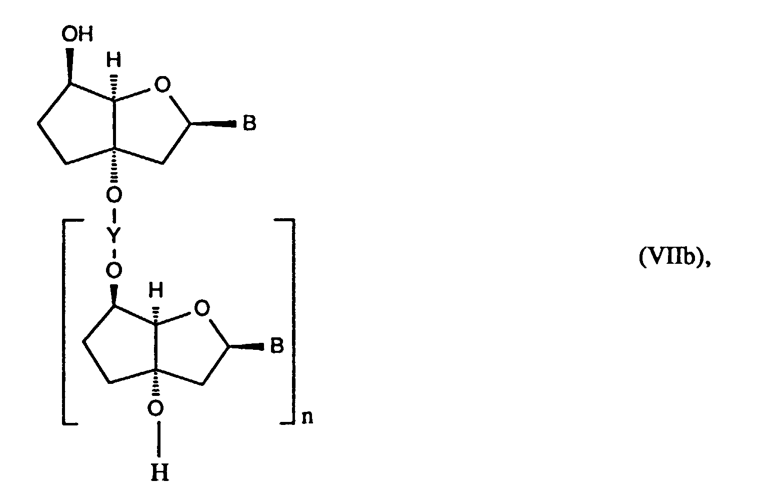 worin B, Y und n die in Anspruch 35 angegebenen Bedeutungen haben.

58. Oligonukleotide der Formel VIIb gemäss Anspruch 57, worin B für 9-Adenyl und n für 10 stehen.

59. Oligonukleotide der Formel VIIb gemäss Anspruch 57, worin B für Cytosyl und n für 6 stehen.

60. Oligonukleotide der Formel VIIb gemäss Anspruch 57, worin B für Thymidyl und n für 10 stehen.

61. Verwendung der Oligonukleotide der Formeln VII, VIIa oder VIIb als Diagnostika zum Nachweis von viralen Infektionen oder genetisch bedingten Krankheiten.

62. Nukleoside der Formeln I, IV oder VIa oder der Oligonukleotide der Formeln VII, VIIa oder VIIb zur Anwendung in einem therapeutischen Verfahren zur Behandlung von Krankheiten bei Warmblütern einschliesslich des Menschen durch Inaktivierung von Nukleotidsequenzen im Körper.

63. Pharmazeutisches Präparat, enthaltend eine wirksame Menge eines Nukleosids der Formeln I, IV oder VIa oder eines Oligonukleotids der Formeln VII, VIIa oder VIIb alleine oder zusammen mit anderen Wirkstoffen, ein pharmazeutisches Trägermaterial und gegebenenfalls Hilfstoffe.

64. Verfahren zur Herstellung eines pharmazeutischen Präparates gemäss Anspruch 63, dadurch gekennzeichnet, dass man eine wirksame Menge eines Nukleosids der Formeln I, IV oder IVa gemäss einem der Ansprüche 1 bis 23 oder eines Oligonukleotids der Formeln VII, VIIa oder VIIb gemäss einem der Ansprüche 35 bis 60, alleine oder zusammen mit anderen Wirkstoffen, mit einem pharmazeutischen Trägermaterial und gegebenenfalls Hilfsstoffen versetzt.

65. Verfahren zur Herstellung eines Oligonukleotides der Formeln VII, VIIa oder VIIb gemäss einem der Ansprüche 35 bis 60, dadurch gekennzeichnet, dass eine Verbindung der Formel I, IV oder IVa gemäss einem der Ansprüche 1 bis 23 bei der Oligonukleotidsynthese eingesetzt wird.

66. Verfahren gemäss Anspruch 65, dadurch gekennzeichnet, dass das Oligonukleotid 2 bis 100 Monomereinheiten aufweist.

67. Verfahren gemäss Anspruch 65, dadurch gekennzeichnet, dass das Oligonukleotid 2 bis 50 Monomereinheiten aufweist.

68. Verfahren gemäss Anspruch 65, dadurch gekennzeichnet, dass das Oligonukleotid 2 bis 20 Monomereinheiten aufweist.

69. Verfahren zur Herstellung einer Verbindung der Formel Va gemäss Anspruch 26 oder der Formel Vla gemäss Anspruch 29, dadurch gekennzeichnet, dass eine Verbindung der Formel H oder der Formel J oder das Racemat 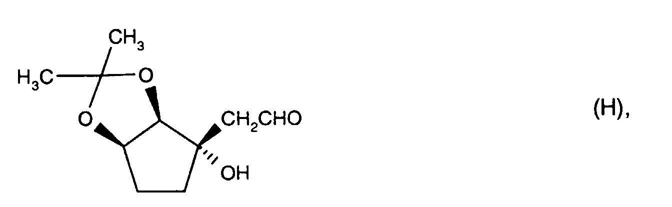 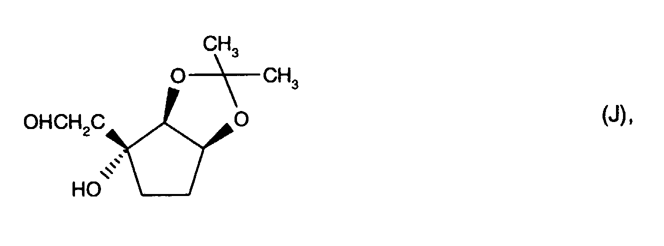 in Gegenwart von wässrigen Säuren oder sauren lonenaustauschern in Gegenwart von Wasser hydrolysiert und cyclisiert wird.

70. Verfahren zur Herstellung einer Verbindung der Formel V gemäss einem der Ansprüche 26 bis 28 oder der Formel VI gemäss Anspruch 29, dadurch gekennzeichnet, dass bei einer Verbindung der Formel Va gemäss Anspruch 26 oder der Formel Vla gemäss Anspruch 29 die anomere Hydroxylgruppe durch eine Abgangsgruppe X ersetzt und die Schutzgruppen R₁₄ und R₁₅ eingeführt werden.

## Claims

1. A compound of the formula I in the form of a racemate or enantiomer thereof, 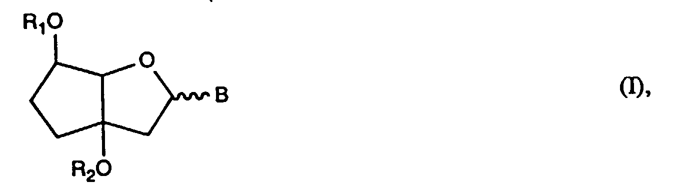 in which R₁ and R₂ independently of one another are hydrogen or a protective group, and B is a purine or pyrimidine radical or an analogue thereof.

2. A compound of the formula I according to claim 1, in which R₁ and R₂ are in each case hydrogen.

3. A compound of the formula I according to claim 1, in which R₁ and R₂ independently of one another are linear or branched C₁-C₈alkyl, C₇-C₁₂aralkyl, triphenylsilyl, alkyldiphenylsilyl, dialkylphenylsilyl and trialkylsilyl, having 1 to 20 C atoms in the alkyl groups, C₂-C₁₂acyl, R₃-SO₂-, in which R₃ is C₁-C₁₂alkyl, C₅cycloalkyl or C₆cycloalkyl, phenyl, benzyl, C₁-C₁₂alkylphenyl, C₁-C₁₂alkylbenzyl, or halophenyl or halobenzyl, or R₁ and R₂ are C₁-C₁₂alkoxycarbonyl, phenyloxycarbonyl, benzyloxycarbonyl, methyl- or methoxy- or chlorophenyloxycarbonyl or -benzyloxycarbonyl.

4. A compound of the formula I according to claim 3, in which R₁ and R₂ independently of one another are linear or branched C₁-C₄alkyl, C₇-C₁₂aralkyl, trialkylsilyl having 1 to 12 C atoms in the alkyl groups, C₂-C₈acyl, R₃-SO₂-, in which R₃ is C₁-C₆alkyl, phenyl, benzyl, C₁-C₄alkylphenyl, C₁-C₄alkylbenzyl, or halophenyl or halobenzyl, or R₁ and R₂ are C₁-C₈alkoxycarbonyl, phenoxycarbonyl or benzyloxycarbonyl.

5. A compound of the formula I according to claim 1, in which R₁ and R₂ are identical protective groups.

6. A compound of the formula I according to claim 1, in which R₁ and R₂ are methyl, ethyl, n- and i-propyl or n-, i- and t-butyl; benzyl, methylbenzyl, dimethylbenzyl, methoxybenzyl, dimethoxybenzyl, bromobenzyl; diphenylmethyl, di(methylphenyl)methyl, di(dimethylphenyl)methyl, di(methoxyphenyl)methyl, di(dimethoxyphenyl)methyl, trityl, tri(methylphenyl)methyl, tri(dimethylphenyl)methyl, tri(methoxyphenyl)methyl, tri(dimethoxyphenyl)methyl; trimethylsilyl, triethylsilyl, tri-n-propylsilyl, i-propyldimethylsilyl, t-butyldimethylsilyl, t-butyldiphenylsilyl, n-octyldimethylsilyl, (1,1,2,2-tetramethylethyl)dimethylsilyl; acetyl, propanoyl, butanoyl, pentanoyl, hexanoyl, benzoyl, methylbenzoyl, methoxybenzoyl, chlorobenzoyl and bromobenzoyl; methyl-, ethyl-, propyl-, butyl-, phenyl-, benzyl-, p-bromo-, p-methoxy- and p-methylphenylsulfonyl; methoxy-, ethoxy-, n- or i-propoxy- or n-, i- or t-butoxycarbonyl, or phenyloxycarbonyl, benzyloxycarbonyl, methyl- or methoxy- or chlorophenyloxycarbonyl or -benzyloxycarbonyl.

7. A compound of the formula I according to claim 1, in which B, as a purine radical or an analogue thereof, is a radical of the formulae II, IIa, IIb, IIc, IId or IIe 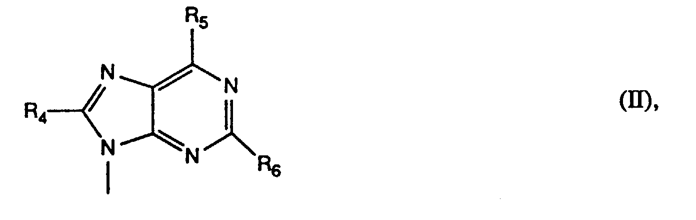 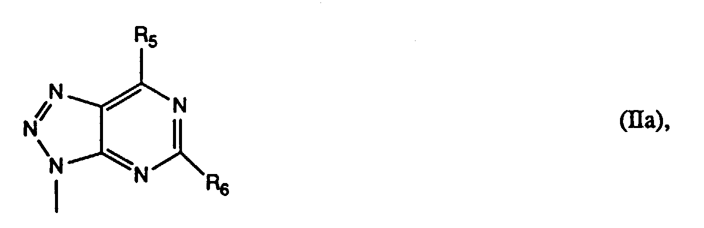 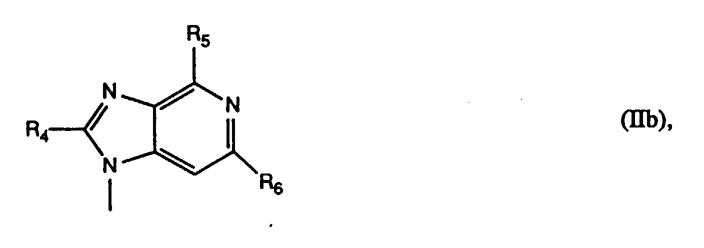 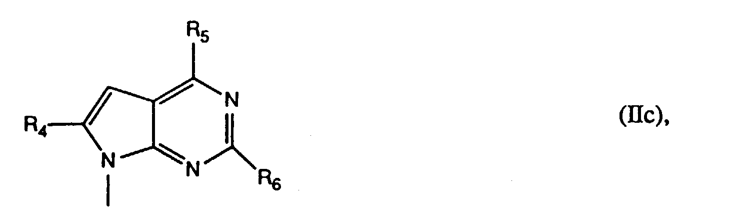 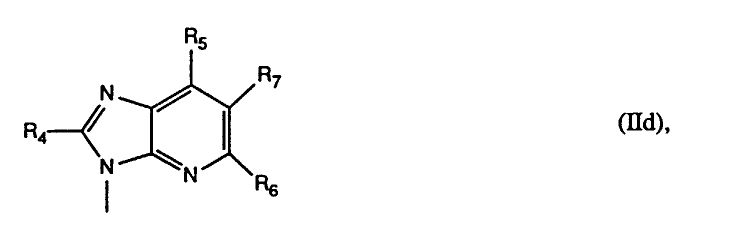 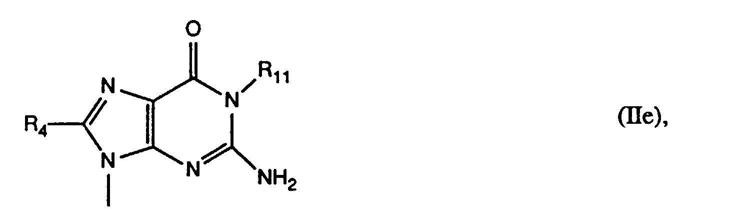 in which R₄ is H, Cl, Br or OH, and R₅, R₆ and R₇ independently of one another are H, OH, SH, NH₂, NHNH₂, NHOH, NHOalkyl having 1 to 12 C atoms, F, Cl, Br, alkyl or hydroxyalkyl or aminoalkyl or alkoxy or alkylthio, having 1 to 12 C atoms, the hydroxyl and amino groups being unsubstituted or substituted by a protective group, or are phenyl, benzyl, primary amino having 1 to 20 C atoms or secondary amino having 2 to 30 C atoms, and R₁₁ is H or C₁-C₄alkyl.

8. A compound of the formula I according to claim 7, in which the protective group for hydroxyl and amino groups is C₁-C₈acyl.

9. A compound of the formula I according to claim 7, in which the primary amino has 1 to 12 C atoms and the secondary amino has 2 to 12 C atoms.

10. A compound of the formula I according to claim 7, in which the primary amino and secondary amino are radicals of the formula R₈R₉N in which R₈ is H or independently has the meaning of R₉, and R₉ is C₁-C₂₀alkyl, C₁-C₂₀aminoalkyl, C₁-C₂₀hydroxyalkyl; carboxyalkyl or carbalkoxyalkyl, the carbalkoxy group having 2 to 8 C atoms and the alkyl group having 1 to 6, preferably 1 to 4, C atoms; C₂-C₂₀alkenyl; phenyl, mono- or di(C₁-C₄alkyl- or C₁-C₄alkoxy)phenyl, benzyl, mono- or di(C₁-C₄alkyl- or C₁-C₄alkoxy)benzyl; or 1,2-, 1,3- or 1,4-imidazolylC₁-C₆alkyl, or R₈ and R₉ together are tetra- or pentamethylene, 3-oxa-1,5-pentylene, -CH₂-NR₁₀-CH₂CH₂- or -CH₂CH₂-NR₁₀-CH₂CH₂-, in which R₁₀ is H or C₁-C₄alkyl, the amino group in the aminoalkyl being unsubstituted or substituted by one or two C₁-C₄alkyl or C₁-C₄hydroxyalkyl groups and the hydroxyl group in the hydroxyalkyl is free or etherified with C₁-C₄alkyl.

11. A compound of the formula I according to claim 9, in which the primary amino and secondary amino are methyl-, ethyl-, dimethyl-, diethyl-, allyl-, mono- or di-(hydroxyeth-2-yl)-, phenyl- and benzyl-, acetyl- and benzoyl-amino.

12. A compound of the formula I according to claim 7, in which R₄ in formulae II, IIb, IIc, IId and IIe is hydrogen.

13. A compound of the formula I according to claim 7, in which R₇ in formula IId is hydrogen.

14. A compound of the formula I according to claim 7, in which R₅ and R₆ in formulae II, IIa, IIb, IIc, IId and IIe independently of one another are H, F, Cl, Br, OH, SH, NH₂, NHOH, NHNH₂, methylamino, dimethylamino, benzoylamino, methoxy, ethoxy and methylthio.

15. A compound of the formula I according to claim 1, in which B is a purine radical or a radical of a purine analogue from the series comprising adenine, N-methyladenine, N-benzoyladenine, 2-methyladenine, 2-methylthioadenine, 2-aminoadenine, 3-carbaadenine, 7-carbaadenine, 1-carbaadenine, 6-hydroxypurine, 2-amino-6-chloropurine, 2-amino-6-methylthiopurine, 2-amino-6-hydroxypurine, 3-carba-6-chloropurine, guanine, 2-methylguanine.

16. A compound of the formula I according to claim 1, in which B in formula I as an analogue pyrimidine radical is a uracil, thymine or cytosine radical of the formulae III, IIIa and IIIb, 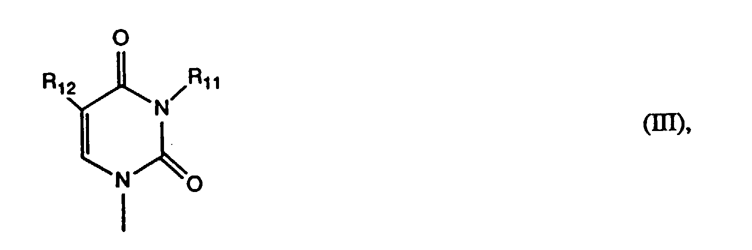 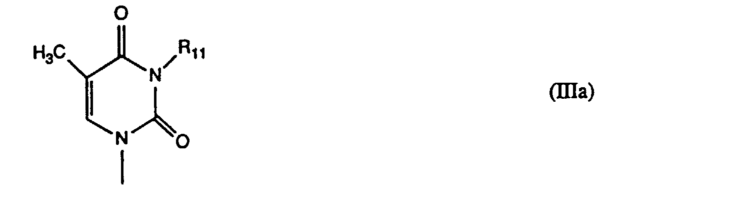 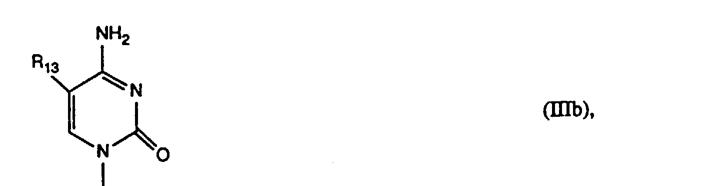 in which R₁₁ is H or C₁-C₄alkyl, and R₁₂ and R₁₃ independently of one another are H, OH, SH, NH₂, NHNH₂, NHOH, NHOalkyl having 1 to 12 C atoms, F, Cl, Br, alkyl or hydroxyalkyl or aminoalkyl or alkoxy or alkylthio, having 1 to 12 C atoms, the hydroxyl and amino groups being unsubstituted or substituted by a protective group, or R₁₂ and R₁₃ are phenyl, benzyl, primary amino having 1 to 20 C atoms or secondary amino having 2 to 30 C atoms, and the hydrogen atoms of the NH₂ group in formula IIIb are unsubstituted or substituted by C₁-C₆alkyl, benzoyl or benzyl, as well as the dihydro derivatives of the radicals of the formulae III, IIIa and IIIb.

17. A compound of the formula I according to claim 16, in which R₁₂ is H, C₁-C₆alkyl or C₁-C₆hydroxyalkyl, F, Cl, Br, NH₂, benzoylamino, mono- or di-C₁-C₆alkylamino.

18. A compound of the formula I according to claim 16, in which R₁₃ is H, C₁-C₆alkyl or C₁-C₆alkoxy or C₁-C₆hydroxyalkyl, F, Cl, Br, NH₂, benzoylamino, mono- or di-C₁-C₆alkylamino.

19. A compound of the formula I according to claim 17, in which R₁₂ is H, F, Cl, Br, NH₂, NHCH₃, N(CH₃)₂ or C₁-C₄alkyl.

20. A compound of the formula I according to claim 18, in which R₁₃ is H, C₁-C₄alkyl, NH₂, NHCH₃ or (CH₃)₂N.

21. A compound of the formula I according to claim 1, in which B as the radical of a pyrimidine analogue is derived from uracil, thymine, cytosine, 5-fluorouracil, 5-chlorouracil, 5-bromouracil, dihydrouracil, pseudouracil, 1-methylpseudouracil, 5-methyluracil, 3-methylcytosine and 5-methylcytosine.

22. A compound of the formula I according to claim 1, which is the α- or β-anomer of the formula IV, 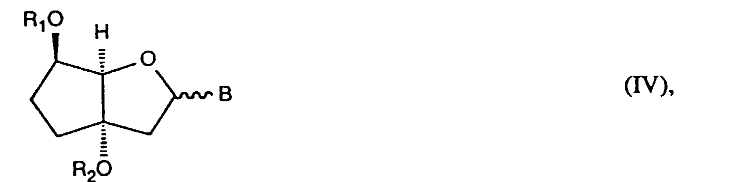 in which R₁, R₂ and B have the meanings given in claim 1.

23. A compound of the formula I according to claim 22, which is the β-anomer of the formula IVa, 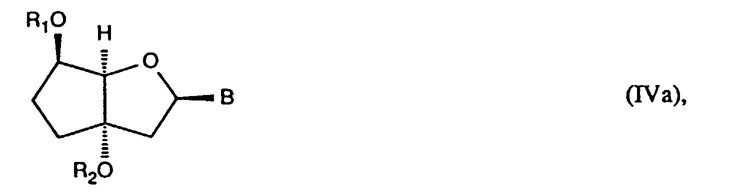 in which R₁, R₂ and B have the meanings given in claim 1.

24. A process for the preparation of a compound of the formulae I, IV or IVa according to one of claims 1 to 23, which comprises reacting a compound of the formula V in the form of a racemate or enantiomer thereof 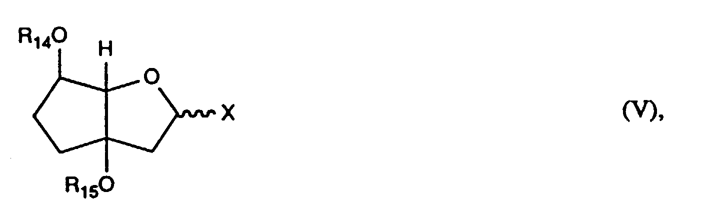 in which R₁₄ and R₁₅ are identical or different protective groups and X is a leaving group, with a purine, purine analogue or pyrimidine analogue, and, if desired, subsequently removing the protective groups.

25. A process according to claim 24, wherein the leaving group is halogen, C₁-C₆alkoxy, C₁-C₈acyloxy or R₁₆-SO₃, in which Rₗ₆ is C₁-C₆alkyl or C₁-C₆haloalkyl, or phenyl or benzyl each of which is unsubstituted or substituted by one to three halogen, C₁-C₄alkyl or C₁-C₄alkoxy.

26. A compound of the formula V or Va in the form of a racemate or enantiomer thereof, 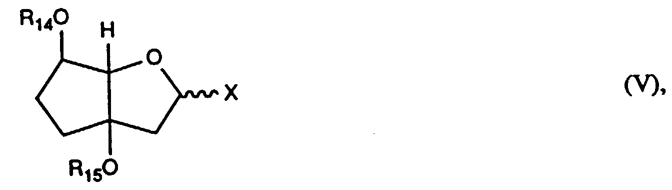 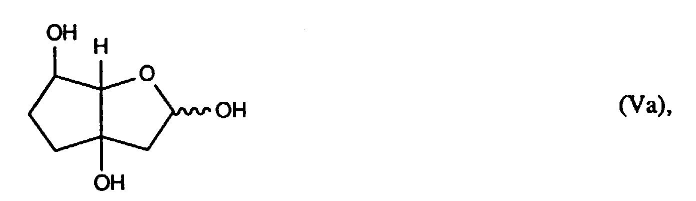 in which R₁₄ and R₁₅ are identical or different protective groups and X is a leaving group.

27. A compound of the formula V or Va according to claim 26, wherein the leaving group is halogen, C₁-C₆alkoxy, C₁-C₈acyloxy or R₁₆-SO₃, in which R₁₆ is C₁-C₆alkyl or C₁-C₆haloalkyl, or phenyl or benzyl, each of which is unsubstituted or substituted by one to three halogen, C₁-C₄alkyl or C₁-C₄alkoxy.

28. A compound of the formula V or Va according to claim 27, wherein the leaving group is CH₃CO(O)O.

29. A compound according to claim 26, which is an enantiomer of the formula VI or VIa, 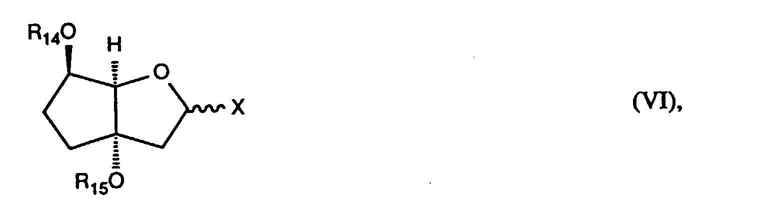 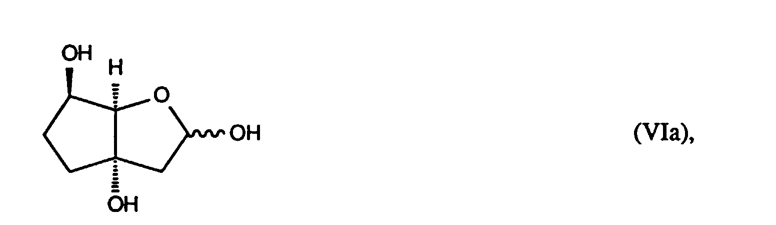 in which R₁₄, R₁₅ and X have the meanings given in claim 25.

30. Use of a compound of the formula I according to claim 1 in the form of a racemate or enantiomer for the preparation of oligonucleotides which comprise identical or different monomer units of compounds of the formula I or monomer units of other nucleosides, the oligonucleotides comprising 2 to 200 monomer units.

31. Use according to claim 30 for the preparation of oligonucleotides having 2 to 100 monomer units.

32. Use according to claim 30 for the preparation of oligonucleotides having 2 to 50 monomer units.

33. Use according to claim 30 for the preparation of oligonucleotides having 2 to 20 monomer units.

34. Use according to claim 30 for the preparation of oligonucleotides having identical monomer units of compounds of the formula I.

35. An oligonucleotide of the formula VII 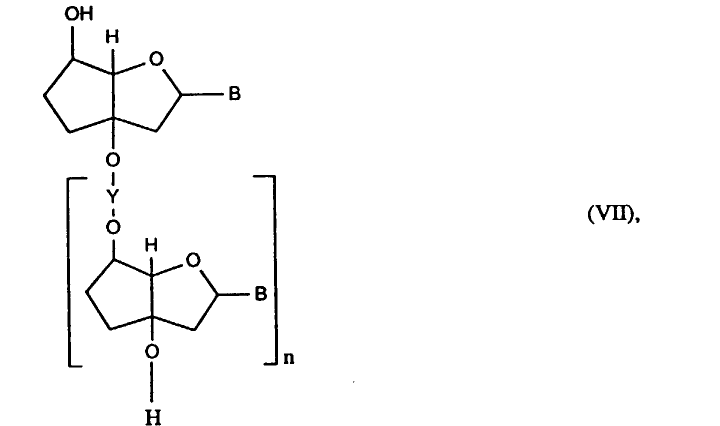 in which B is a purine or pyrimidine radical or an analogue thereof, n is a number from 2 to 200 and Y is a nucleotide bridging group.

36. An oligonucleotide of the formula VII according to claim 35, wherein the bridging group is -P(O)O-^{⊖}, -P(O)S^{⊖}-, -P(S)S^{⊖} -, -P(O)R₁₇-, -P(O)OR₁₈-, -P(O)NR₁₉R₂₀-, -CO- or -CON(R₁₈)₂-, in which R₁₇ is H or C₁-C₆alkyl and R₁₈ is C₁-C₆alkyl and R₁₉ and R₂₀ independently of one another have the meaning of R₁₇.

37. An oligonucleotide of the formula VII according to claim 35, wherein the bridging group is -P (O)O^{⊖}-.

38. An oligonucleotide of the formula VII according to claim 35, wherein n is a number from 2 to 100.

39. An oligonucleotide of the formula VII according to claim 35, wherein n is a number from 2 to 50.

40. An oligonucleotide of the formula VII according to claim 35, wherein n is a number from 2 to 20.

41. An oligonucleotide of the formula VII according to claim 35, in which B as a purine radical or an analogue thereof is a radical of the formulae II, IIa, IIb, IIc, IId or IIe, 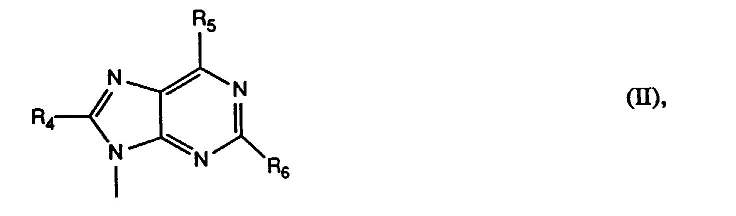 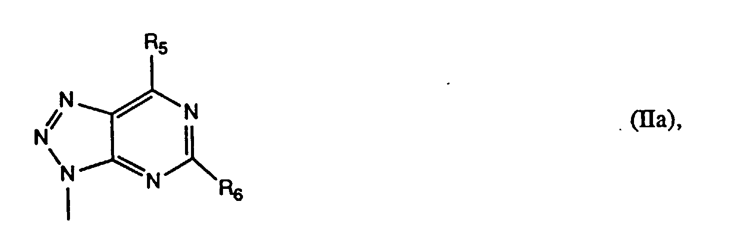 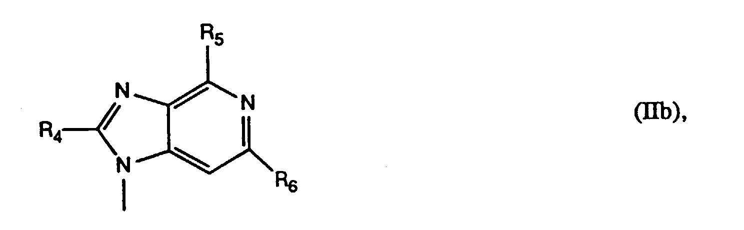 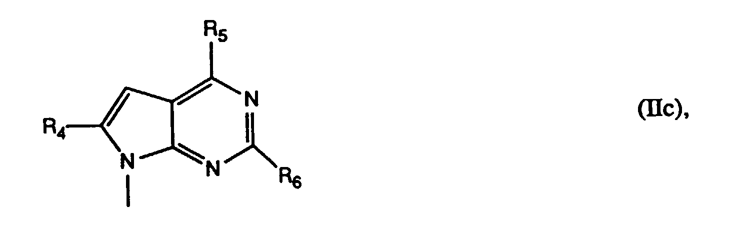 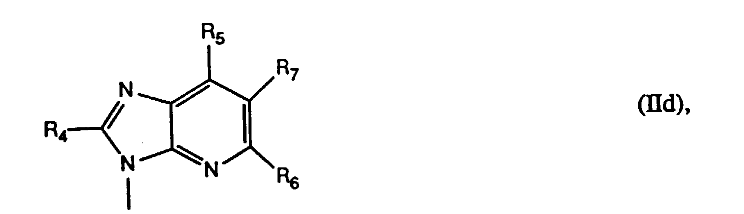 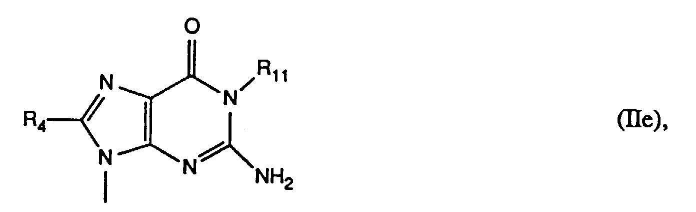 in which R₄ is H, Cl, Br or OH, and R₅, R₆ and R₇ independently of one another are H, OH, SH, NH₂, NHNH₂, NHOH, NHOalkyl having 1 to 12 C atoms, F, Cl, Br, alkyl or hydroxyalkyl or aminoalkyl or alkoxy or alkylthio having 1 to 12 C atoms, the hydroxyl and amino groups being unsubstituted or substituted by a protective group, or are phenyl, benzyl, primary amino having 1 to 20 C atoms or secondary amino having 2 to 30 C atoms, and R₁₁ is H or C₁-C₄alkyl.

42. An oligonucleotide of the formula VII according to claim 41, in which the protective group for hydroxyl and amino groups is C₁-C₈acyl.

43. An oligonucleotide of the formula VII according to claim 41, in which the primary amino has 1 to 12 C atoms and the secondary amino has 2 to 12 C atoms.

44. An oligonucleotide of the formula VII according to claim 41, in which the primary amino and secondary amino are radicals of the formula R₈R₉N in which R₈ is H or independently has the meaning of R₉, and R₉ is C₁-C₂₀alkyl, C₁-C₂₀aminoalkyl, C₁-C₂₀hydroxyalkyl; carboxyalkyl or carbalkoxyalkyl, the carbalkoxy group having 2 to 8 C atoms and the alkyl group having 1 to 6, preferably 1 to 4, C atoms; C₂-C₂₀alkenyl; phenyl, mono- or di-(C₁-C₄alkyl- or C₁-C₄alkoxy)phenyl, benzyl, mono- or di-(C₁-C₄alkyl- or C₁-C₄alkoxy)benzyl; or 1,2-, 1,3- or 1,4-imidazolyl-C₁-C₆alkyl, or R₈ and R₉ together are tetra- or pentamethylene, 3-oxa-1,5-pentylene, -CH₂-NR₁₀-CH₂CH₂- or -CH₂CH₂-NR₁₀-CH₂CH₂-, in which R₁₀ is H or C₁-C₄alkyl, the amino group in the aminoalkyl being unsubstituted or substituted by one or two C₁-C₄alkyl or C₁-C₄hydroxyalkyl groups and the hydroxyl group in the hydroxyalkyl is free or etherified with C₁-C₄alkyl.

45. An oligonucleotide of the formula VII according to claim 44, wherein the primary amino and secondary amino are methyl-, ethyl-, dimethyl-, diethyl-, allyl-, mono- or di-(hydroxyeth-2-yl)-, phenyl- and benzyl-, acetyl- and benzoylamino.

46. An oligonucleotide of the formula VII according to claim 41, in which R₄ in formulae II, IIb, IIc, IId and IIe is hydrogen.

47. An oligonucleotide of the formula VII according to claim 41, in which R₇ in formula IId is hydrogen.

48. An oligonucleotide of the formula VII according to claim 41, in which R₅ and R₆ in formulae II, IIa, IIb, IIc, IId and IIe independently of one another are H, F, Cl, Br, OH, SH, NH₂, NHOH, NHNH₂, methylamino, dimethylamino, benzoylamino, methoxy, ethoxy and methylthio.

49. An oligonucleotide of the formula VII according to claim 41, in which B is a purine radical or a radical of a purine analogue from the series comprising adenine, N-methyladenine, N-benzoyladenine, 2-methyladenine, 2-methylthioadenine, 2-aminoadenine, 3-carbaadenine, 7-carbaadenine, 1-carbaadenine, 6-hydroxypurine, 2-amino-6-chloropurine, 2-amino-6-methylthiopurine, 2-amino-6-hydroxypurine, 3-carba-6-chloropurine, guanine, 2-methylguanine.

50. An oligonucleotide of the formula VII according to claim 35, in which B in formula VII as an analogue pyrimidine radical is a uracil, thymine or cytosine radical of the formulae III, IIIa and IIIb, 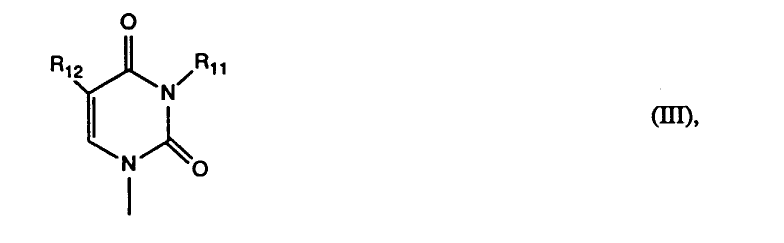 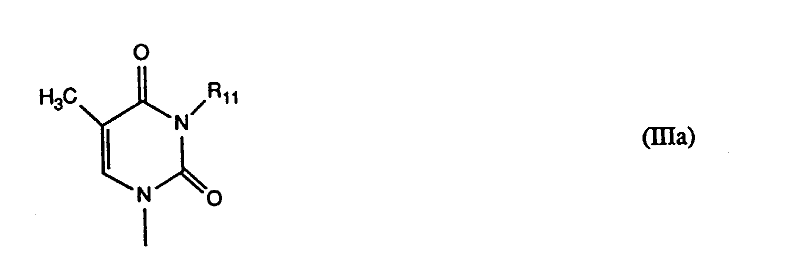 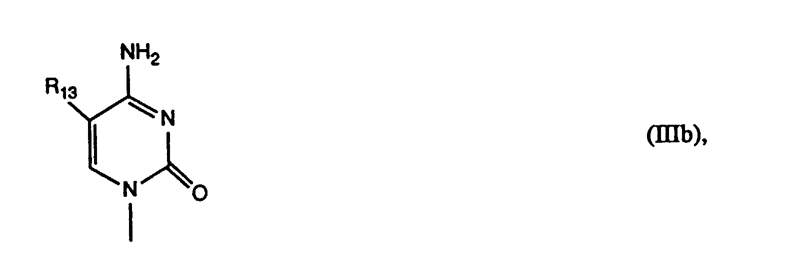 in which R₁₁ is H or C₁-C₄alkyl, and R₁₂ and R₁₃ independently of one another are H, OH, SH, NH₂, NHNH₂, NHOH, NHOalkyl having 1 to 12 C atoms, F, Cl, Br, alkyl or hydroxyalkyl or aminoalkyl or alkoxy or alkylthio having 1 to 12 C atoms, the hydroxyl and amino groups being unsubstituted or substituted by a protective group, or R₁₂ and R₁₃ are phenyl, benzyl, primary amino having 1 to 20 C atoms or secondary amino having 2 to 30 C atoms, and the hydrogen atoms of the NH₂ group in formula IIIb are unsubstituted or substituted by C₁-C₆alkyl, benzoyl or benzyl, as well as the dihydro derivatives of the radicals of the formulae III, IIIa and IIIb.

51. An oligonucleotide of the formula VII according to claim 50, in which R₁₂ is H, C₁-C₆alkyl or C₁-C₆hydroxyalkyl, F, Cl, Br, NH₂, benzoylamino, mono- or di-C₁-C₆alkylamino.

52. An oligonucleotide of the formula VII according to claim 50, in which R₁₃ is H, C₁-C₆alkyl or C₁-C₆alkoxy or C₁-C₆hydroxyalkyl, F, Cl, Br, NH₂, benzoylamino, mono- or di-C₁-C₆alkylamino.

53. An oligonucleotide of the formula VII according to claim 51, in which R₁₂ is H, F, C1, Br, NH₂, NHCH₃, N(CH₃)₂ or C₁-C₄alkyl.

54. An oligonucleotide of the formula VII according to claim 52, in which R₁₃ is H, C₁-C₄alkyl, NH₂, NHCH₃ or (CH₃)₂N.

55. An oligonucleotide of the formula VII according to claim 50, in which B as the radical of a pyrimidine analogue is derived from uracil, thymine, cytosine, 5-fluorouracil, 5-chlorouracil, 5-bromouracil, dihydrouracil, pseudouracil, 1-methylpseudouracil, 5-methyluracil, 3-methylcytosine and 5-methylcytosine.

56. An oligonucleotide of the formula VII according to claim 35, which is of the formula VIIa. 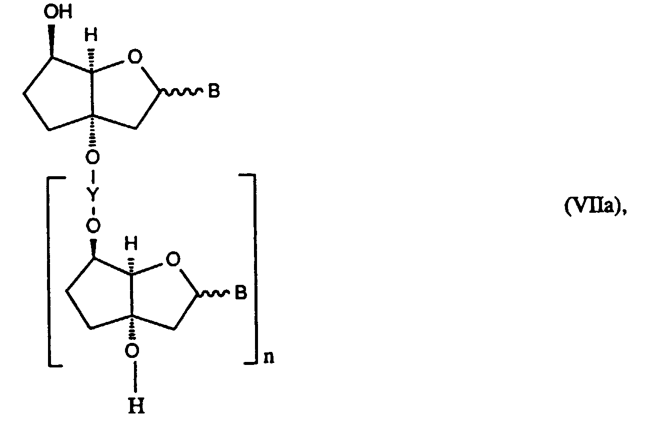 in which B, Y and n have the meanings given in claim 35.

57. An oligonucleotide of the formula VII according to claim 35, which is of the formula VIIb 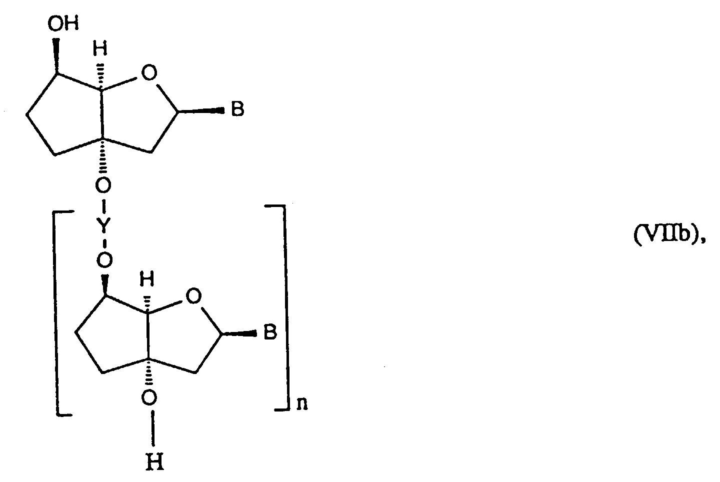 in which B, Y and n have the meanings given in claim 35.

58. An oligonucleotide of the formula VIIb according to claim 57, in which B is 9-adenyl and n is 10.

59. An oligonucleotide of the formula VIIb according to claim 57, in which B is cytosyl and n is 6.

60. An oligonucleotide of the formula VIIb according to claim 57, in which B is thymidyl and n is 10.

61. Use of an oligonucleotide of the formulae VII, VIIa or VIIb as a diagnostic for the detection of viral infections or genetically caused diseases.

62. A nucleoside of the formulae I, IV or IVa or of the oligonucleotides of the formulae VII, VIIa or VIIb for use in a therapeutic method for the treatment of diseases in warm-blooded species including humans by inactivation of nucleotide sequences in the body.

63. A pharmaceutical preparation comprising an effective amount of a nucleoside of formulae I, IV or VIa or of an oligonucleotide of formulae VII, VIIa or VIIb, as pure active ingredient or together with other active ingredients, a pharmaceutical excipient and, if desired, adjuncts.

64. A process for preparing a pharmaceutical preparation according to claim 63, which comprises adding a pharmaceutical excipient and, if desired, adjuncts to an effective amount of a nucleoside of the formulae I, IV or IVa according to any one of claims 1 to 23, or an oligonucleotide of the formulae VII, VIIa or VIIb according to any one of claims 35 to 60, alone or together with other active ingredients.

65. A process for the preparation of an oligonucleotide of the formulae VII, VIIa or VIIb according to any one of claims 35 to 60, which comprises employing a compound of the formula I, IV or IVa according to any one of claims 1 to 23 in the oligonucleotide synthesis.

66. A process according to claim 65, wherein the oligonucleotide has 2 to 100 monomer units.

67. A process according to claim 65, wherein the oligonucleotide has 2 to 50 monomer units.

68. A process according to claim 65, wherein the oligonucleotide has 2 to 20 monomer units.

69. A process for the preparation of a compound of the formula Va according to claim 26 or of the formula VIa according to claim 29, which comprises hydrolysing and cyclizing a compound of the formula H or of the formula J or the racemate 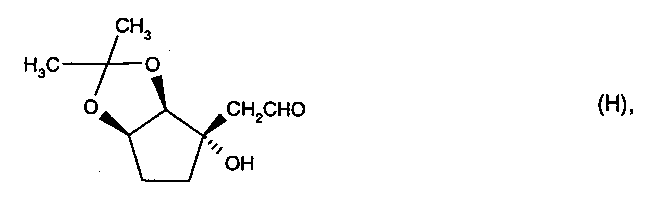 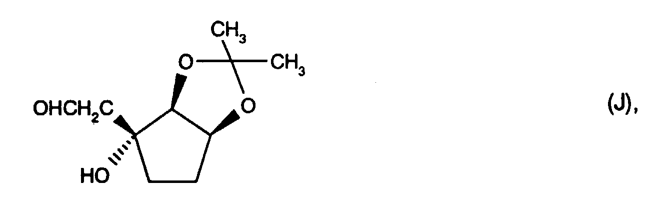 in the presence of aqueous acids or acidic ion exchangers in the presence of water.

70. A process for the preparation of a compound of the formula V according to any one of claims 26 to 28 or of the formula VI according to claim 29, which comprises replacing in a compound of the formula Va according to claim 26 or of the formula VIa according to claim 29 the anomeric hydroxyl group by a leaving group X and introducing the protective groups R₁₄, and R₁₅.

## Revendications

1. Composés de formule I, sous forme de leurs racémiques ou énantiomères, 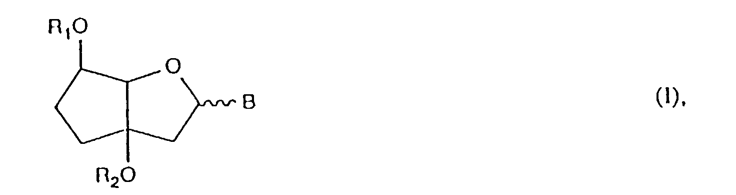 formule dans laquelle R₁ et R₂ représentent, idépendamment l'un de l'autre, un atome d'hydrogène ou un groupe protecteur, et B représente un reste de purine ou de pyrimidine ou un analogue de celui-ci.

2. Composés de formule I selon la revendication 1, dans lesquels R₁ et R₂ représentent chacun un atome d'hydrogène.

3. Composés de formule I selon la revendication 1, dans lesquels R₁ et R₂, indépendamment l'un de l'autre, représentent un groupe alkyle en C₁-C₈ linéaire ou ramifié, aralkyle en C₇-C₁₂, triphénylsilyle, alkyldiphénylsilyle, dialkylphénylsilyle ou trialkylsilyle ayant de 1 à 20 atomes de carbone dans les fragments alkyle, acyle en C₂-C₁₂, un groupe R₃-SO₂-, dans lequel R₃ représente un radical alkyle en C₁-C₁₂, cycloalkyle en C₅ ou C₆, phényle, benzyle, alkyl-(C₁-C₁₂)-phényle, alkyl(C₁-C₁₂)-benzyle ou un radical halogénophényle ou halogénobenzyle, ou représentent un groupe alcoxy(C₁-C₁₂)-carbonyle, phényloxycarbonyle, benzoyloxycarbonyle, méthyl- ou méthoxy- ou chlorophényloxycarbonyle ou -benzyloxycarbonyle.

4. Composés de formule I selon la revendication 3, dans lesquels R₁ et R₂, indépendamment l'un de l'autre, représentent un groupe alkyle en C₁-C₄ linéaire ou ramifié, aralkyle en C₇-C₁₂, trialkylsilyle ayant de 1 à 12 atomes de carbone dans les fragments alkyle, acyle en C₂-C₈, un groupe R₃-SO₂- dans lequel R₃ représente un radical alkyle en C₁-C₆, phényle, benzyle, alkyl(C₁-C₄)-phényle, alkyl(C₁-C₄)-benzyle ou halogénophényle ou halogénobenzyle, ou représentent un groupe alcoxy(C₁-C₈)-carbonyle, phénoxycarbonyle ou benzyloxycarbonyle.

5. Composés de formule I selon la revendication 1, dans lesquels R₁ et R₂ représentent des groupes protecteurs identiques.

6. Composés de formule I selon la revendication 1, dans lesquels R₁ et R₂ représentent les groupe méthyle, éthyle, n- et isopropyle, n-, iso- et tert-butyle; benzyle, méthylbenzyle, diméthylbenzyle, méthoxybenzyle, diméthoxybenzyle, bromobenzyle; diphénylméthyle, di(méthylphényl)méthyle, di(diméthylphényl)méthyle, di(méthoxyphényl)méthyle), di(diméthoxyphényl)méthyle, trityle, tri(méthylphényl)méthyle, tri(diméthylphényl)méthyle, tri(méthoxyphényl)méthyle, tri(diméthoxyphényl)méthyle; triméthylsilyle, triéthylsilyle, tri-n-propylsilyle, isopropyldiméthylsilyle, tert-butyl-diméthylsilyle, tert-butyldiphénylsilyle, n-octyl-diméthylsilyle, (1,1,2,2-tétraméthyléthyl)diméthylsilyle; acétyle, propionyle, butanoyle, pentanoyle, hexanoyle, benzoyle, méthylbenzoyle, méthoxybenzoyle, chlorobenzoyle et bromobenzoyle; méthyl-, éthyl-, propyl-, butyl-, phényl-, benzyl-, p-bromo-, p-méthoxy- et p-méthylphénylsulfonyle; méthoxy-, éthoxy-, n- ou isopropoxy- ou n-, iso- ou tert-butoxycarbonyle, ou phényloxycarbonyle, benzyloxycarbonyle, méthyl- ou méthoxyou chlorophényloxycarbonyle ou -benzyloxycarbonyle.

7. Composés de formule I selon la revendication 1, dans lesquels B, en tant que reste de purine ou analogue de I celui-ci, représente un reste de formule II, IIa, IIb, IIc, IId ou IIe, 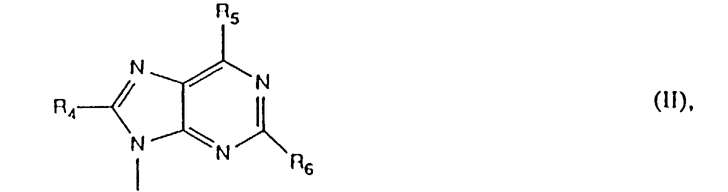 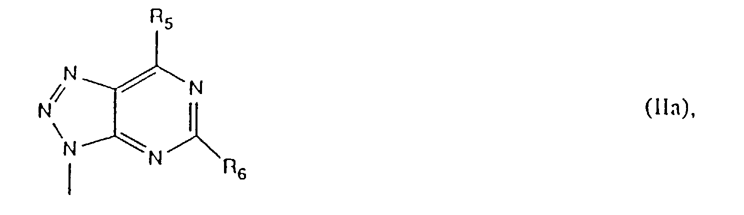 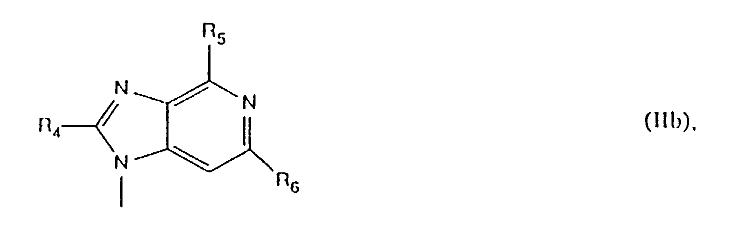 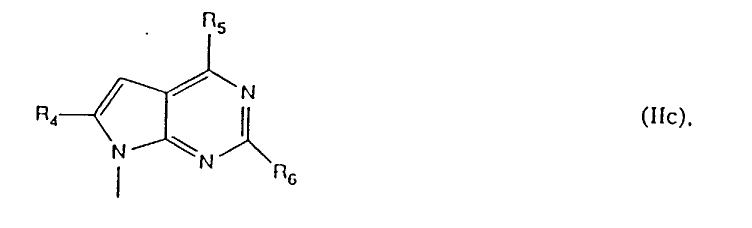 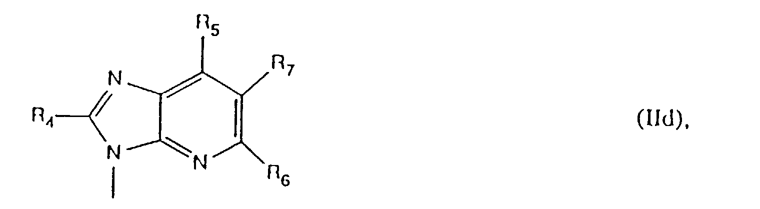 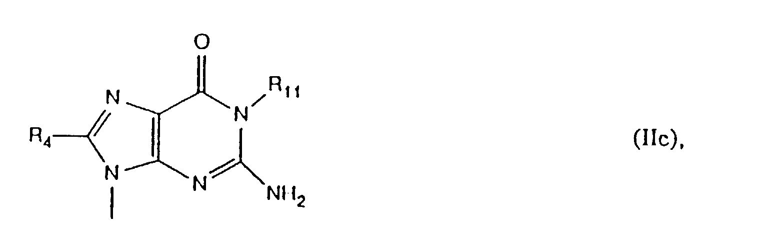 dans lesquelles R₄ représente H, Cl, Br ou OH, et R₅, R₆ et R₇, indépendamment les uns des autres, représentent H, OH, SH, NH₂, NHNH₂, NHOH, un groupe NHO-alkyle ayant de 1 à 12 atomes de carbone, F, Cl, Br, un groupe alkyle ou hydroxyalkyle ou aminoalkyle ou alcoxy ou alkylthio ayant de 1 à 12 atomes de carbone, les radicaux hydroxy- et amino étant non substitués ou substitués par un groupe protecteur, ou représentent un groupe phényle, benzyle, amino primaire ayant de 1 à 20 atomes de carbone ou amino secondaire ayant de 2 à 30 atomes de carbone, et R₁₁ représente H ou un groupe alkyle en C₁-C₄.

8. Composés de formule I selon la revendication 7, dans lesquels le groupe protecteur pour les groupes hydroxy et amino représente un groupe acyle en C₁-C₈.

9. Composés de formule I selon la revendication 7, dans lesquels le groupe amino primaire contient de 1 à 12 atomes de carbone et le groupe amino secondaire contient de 2 à 12 atomes de carbone.

10. Composés de formule I selon la revendication 7, dans lesquels, en ce qui concerne les groupes amino primaire et amino secondaire, il s'agit de radicaux de formule R₈R₉N, dans lesquels R₈ représente H ou a, indépendamment, la signification de R₉, et R₉ représente un groupe alkyle, aminoalkyle, hydroxyalkyle en C₁-C₂₀; un groupe carboxyalkyle ou carbalcoxyalkyle, le fragment carbalcoxy ayant de 2 à 8 atomes de carbone et le fragment alkyle ayant de 1 à 6, de préférence de 1 à 4 atomes de carbone; un groupe alcényle en C₂-C₂₀; un groupe phényle, mono- ou di[alkyl- ou alcoxy-(C₁-C₄)]-phényle, benzyle, mono- ou di[alkyl- ou alcoxy-(C₁-C₄)]-benzyle; ou un groupe 1,2-, 1,3- ou 1,4-imidazolyla1kyle(C₁-C₄), ou R₈ et R₉ représentent ensemble un groupe tétra- ou pentaméthylène, 3-oxa-1,5-pentylène, -CH₂-NR₁₀-CH₂CH₂ ou -CH₂CH₂-NR₁₀-CH₂CH₂-, R₁₀ représentant H ou un radical alkyle en C₁-C₄, le fragment amino dans le groupe aminoalkyle étant non substitué ou substitué par un ou deux radicaux alkyle ou hydroxyalkyle en C₁-C₄, et le radical hydroxy dans le groupe hydroxyalkyle étant éventuellement éthérifié par un groupe alkyle en C₁-C₄.

11. Composés de formule I selon la revendication 9, dans lesquels, en ce qui concerne les groupes amino primaire et amino secondaire, il s'agit des groupes méthyl-, éthyl-, diméthyl-, diéthyl-, allyl-, mono- ou di(1-hydroxy-éth-2-yl)-, phényl- et benzyl-, acétyl- et benzoylamino.

12. Composés de formule I selon la revendication 7, dans lesquels R₄ dans les formules II, IIb, IIc, IId et IIe représente un atome d'hydrogène.

13. Composés de formule I selon la revendication 7, dans lesquels R₇ dans la formule IId représente un atome d'hydrogène.

14. Composés de formule I selon la revendication 7, dans lesquels R₅ et R₆ dans les formules II, IIa, IIb, IIc, IId et IIe représentent, indépendamment l'un de l'autre, H, F, Cl, Br, OH, SH, NH₂, NHOH, NHNH₂, le groupe méthylamino, diméthylamino, benzoylamino, méthoxy, éthoxy ou méthylthio.

15. Composés de formule I selon la revendication 1, dans lesquels B est un reste de purine ou un reste d'un analogue de purine choisi parmi l'adénine, la N-méthyladénine, la N-benzoyladénine, la 2-méthyladénine, la 2-méthylthioadénine, la 2-aminoadénine, la 3-carbaadénine, la 7-carbaadénine, la 1-carbaadénine, la 6-hydroxypurine, la 2-amino6-chloro-purine, la 2-amino-6-méthylthiopurine, la 2-amino6-hydroxy-purine, la 3-carba-6-chloropurine, la guanine, la I 2-méthylguanine.

16. Composés de formule I selon la revendication 1, dans lesquels B dans la formule I, en tant que reste analogue de pyrimidine, représente un reste d'uracile, de thymine ou de cytosine de formules III, IIIa et IIIb, 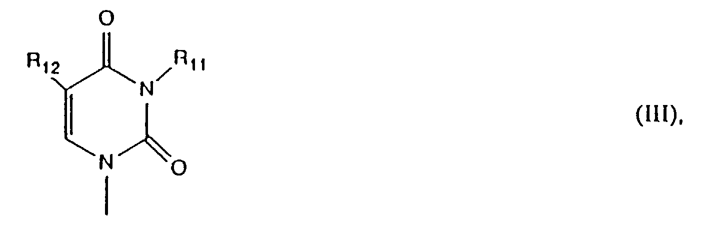 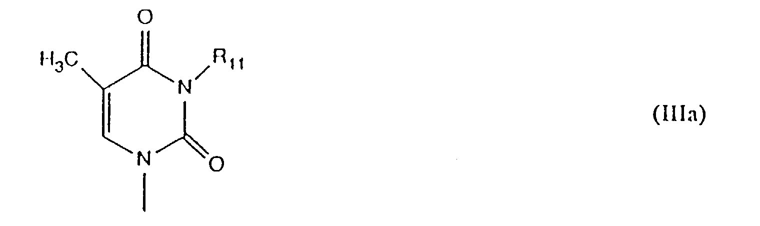 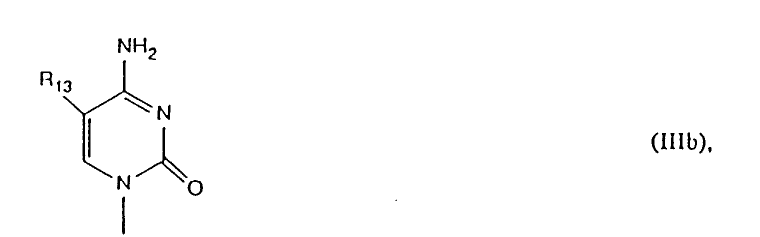 dans lesquelles R₁₁ représente H ou un groupe alkyle en C₁-C₄, et R₁₂ et R₁₃, indépendamment l'un de l'autre, représentent H, OH,SH, NH₂, NHNH₂, NHOH, un groupe NHO-alkyle ayant de 1 à 12 atomes de carbone, F, Cl, Br, un groupe alkyle ou hydroxyalkyle ou aminoalkyle ou alcoxy ou alkylthio ayant de 1 à 12 atomes de carbone, les radicaux hydroxy- et amino étant non substitués ou substitués par un groupe protecteur, ou représentent un groupe phényle, benzyle, amino primaire ayant de 1 à 20 atomes de carbone ou amino secondaire ayant de 2 à 30 atomes de carbone, et les atomes d'hydrogène du groupe NH₂ dans la formule IIIb sont non substitués ou substitués par un groupe alkyle en C₁-C₆, benzoyle ou benzyle, ainsi que les dérivés dihydro des restes de formules III, IIIa et IIIb.

17. Composés de formule I selon la revendication 16, dans lesquels R₁₂ représente H ou un groupe alkyle ou hydroxyalkyle en C₁-C₆, F, Cl, Br, NH₂, ou un groupe benzoylamino, mono- ou dialkyl(C₁-C₆)amino.

18. Composés de formule I selon la revendication 16, dans lesquels R₁₃ représente H ou un groupe alkyle ou alcoxy ou hydroxyalkyle en C₁-C₆, F, Cl, Br, NH₂, ou un groupe benzoylamino, mono- ou dialkyl(C₁-C₆)amino.

19. Composés de formule I selon la revendication 17, dans lesquels R₁₂ représente H, F, Cl, Br, NH₂, NHCH₃, N(CH₃)₂ ou un groupe alkyle en C₁-C₄.

20. Composés de formule I selon la revendication 18, dans lesquels R₁₃ représente H ou un groupe alkyle en C₁-C₄, NH₂, NHCH₃ ou (CH₃)₂N.

21. Composés de formule I selon la revendication 1, dans lesquels B, en tant que reste d'un analogue de pyrimidine, dérive de l'uracile, la thymine, la cytosine, le 5-fluoro-uracile, le 5-chloro-uracile, le 5-bromo-uracile, le dihydro-uracile, le pseudo-uracile, le 1-méthylpseudouracile, le 5-méthyluracile, la 3-méthylcytosine et la 5-méthylcytosine.

22. Composés de formule I selon la revendication 1, caractérisés en ce qu'ils correspondent aux anomères α et β de formule IV 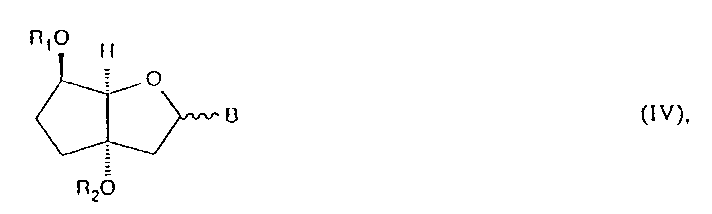 dans laquelle R₁, R₂ et B ont les significations données dans la revendication 1.

23. Composés de formule I selon la revendication 22, caractérisés en ce qu'ils correspondent aux anomères β de formule IVa 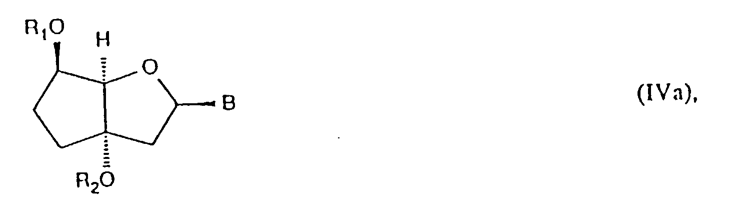 dans laquelle R₁, R₂ et B ont les significations données dans la revendication 1.

24. Procédé pour la préparation des composés de formule I, IV ou IVa selon l'une des revendications 1 à 23, caractérisé en ce que l'on fait réagir un composé de formule V, sous forme de ses racémiques ou énantiomères, 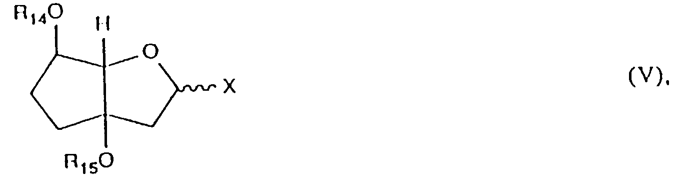 formule dans laquelle R₁₄ et R₁₅ représentent des groupes protecteurs identiques ou différents, et X représente un groupe partant, avec une purine, des analogues de purines ou des analogues de pyrimidines, et ensuite on élimine éventuellement les groupes protecteurs.

25. Procédé selon la revendication 24, caractérisé par le fait qu'en ce qui concerne le groupe partant, il s'agit d'un atome d'halogène ou d'un groupe alcoxy en C₁-C₆, acyloxy en C₁-C₈ ou d'un groupe R₁₆SO₃ dans lequel R₁₆ représente un radical alkyle ou halogénoalkyle en C₁-C₆, ou un radical phényle ou benzyle non susbtitué ou substitué par un à trois atomes d'halogène ou groupes alkyle ou alcoxy en C₁-C₄.

26. Composés de formules V et Va, sous forme de leurs racémiques ou énantiomères, 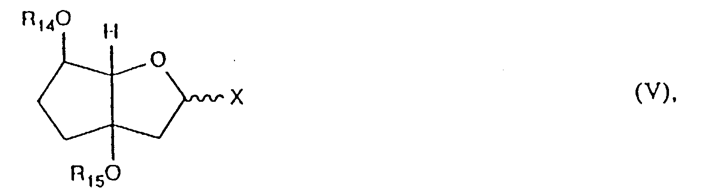 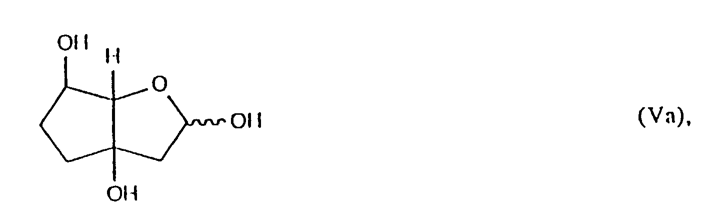 formules dans lesquelles R₁₄ et R₁₅ représentent des groupes protecteurs identiques ou différents, et X représente un groupe partant.

27. Composés de formules V et Va selon la revendication 26, dans lesquels, en ce qui concerne le groupe partant, il s'agit d'un atome d'halogène ou d'un groupe alcoxy en C₁-C₆, acyloxy en C₁-C₈ ou d'un groupe R₁₆SO₃ dans lequel R₁₆ représente un radical alkyle ou halogénoalkyle en C₁-C₆, ou un radical phényle ou benzyle non substitué ou substitué par un à trois atomes d'halogène ou groupes alkyle ou alcoxy en C₁-C₄.

28. Composés de formules V et Va selon la revendication 27, dans lesquels, en ce qui concerne le groupe partant, il s'agit du groupe CH₃C(O)O.

29. Composés selon la revendication 26, caractérisé en ce qu'ils correspondent aux énantiomères de formules VI et VIa, 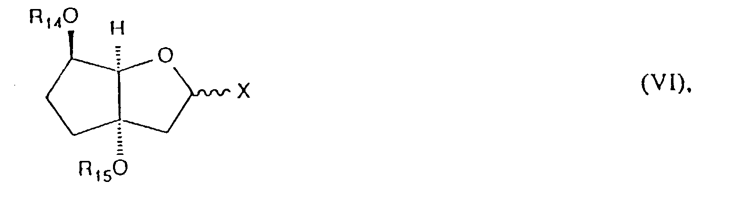 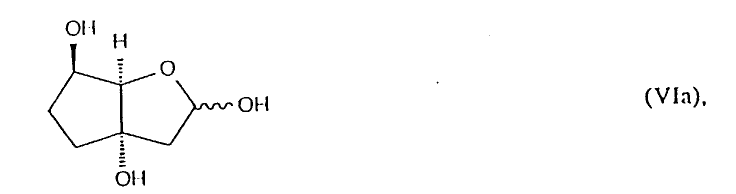 dans lesquelles R₁₄, R₁₅ et X ont les significations données dans la revendication 25.

30. Utilisation des composés de formule I selon la revendication 1, sous forme de racémiques ou d'énantiomères, pour la préparation d'oligonucléotides qui contiennent des unités monomères, identiques ou différentes, de composés de formule I ou des unités monomères, identiques ou différentes, d'autres nucléosides, les oligonucléotides contenant de 2 à 200 unités monomères.

31. Utilisation selon la revendication 30, pour la préparation d'oligonucléotides comportant de 2 à 100 unités monomères.

32. Utilisation selon la revendication 30, pour la préparation d'oligonucléotides comportant de 2 à 50 unités monomères.

33. Utilisation selon la revendication 30, pour la préparation d'oligonucléotides comportant de 2 à 20 unités monomères.

34. Utilisation selon la revendication 30, pour la préparation d'oligonucléotides comportant des unités monomères identiques de composés de formule I.

35. Oligonucléotides de formule VII 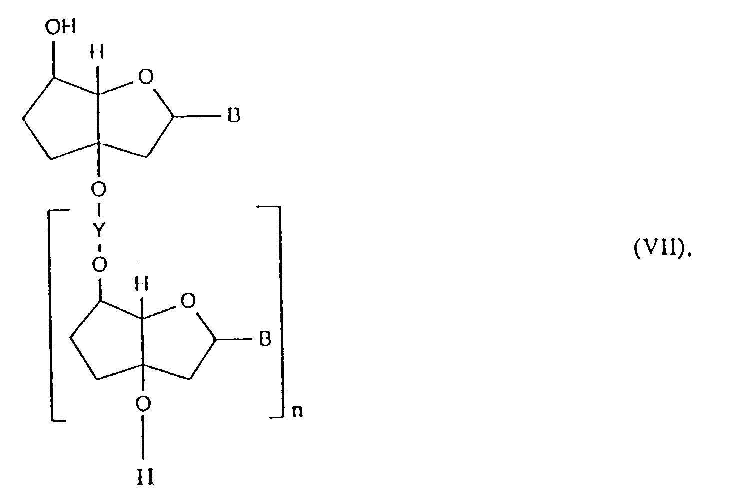 dans laquelle B représente un reste de purine ou de pyrimidine ou d'un analogue de celles-ci, n représente un nombre allant de 2 à 200, et Y représente un groupe pontant reliant les nucléotides.

36. Oligonucléotides de formule VII selon la revendication 35, caractérisés par le fait qu'en ce qui concerne le groupe pontant, il s'agit des groupes -P(O)O^{⊖}-, -P(O)S^{⊖}-, -P(S)S^{⊖}-, P(O)R₁₇-, P(O)OR₁₈-, P(O)NR₁₉R₂₀-, -CO- ou -CON(R₁₈)₂-, dans lesquels R₁₇ représente H ou un groupe alkyle en C₁-C₆, R₁₈ représente un groupe alkyle en C₁-C₆ et R₁₉ et R₂₀ ont indépendamment l'un de l'autre la signification de R₁₇.

37. Oligonucléotides de formule VII selon la revendication 35, caractérisés par le fait qu'en ce qui concerne le groupe pontant, il s'agit du groupe -P(O)O^{⊖}-.

38. Oligonucléotides de formule VII selon la revendication 35, caractérisés en ce que n représente un nombre allant de 2 à 100.

39. Oligonucléotides de formule VII selon la revendication 35, caractérisés en ce que n représente un nombre allant de 2 à 50.

40. Oligonucléotides de formule VII selon la revendication 35, caractérisés en ce que n représente un nombre allant de 2 à 20.

41. Oligonucléotides de formule VII selon la revendication 35, dans lesquels B, en tant que reste de purine ou analogue de celui-ci, représente un reste de formule II, IIa, IIb, IIc, IId ou IIe, 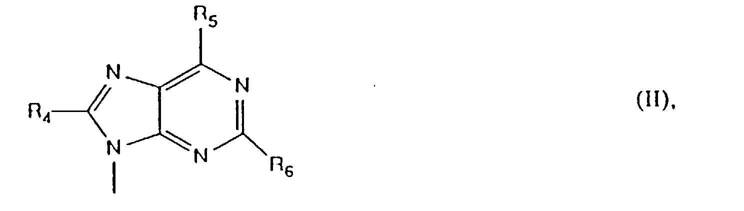 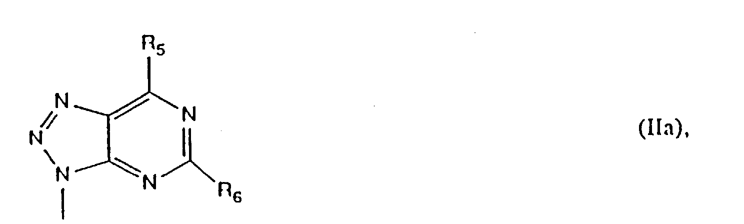 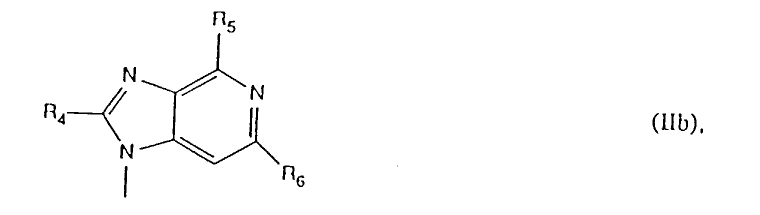 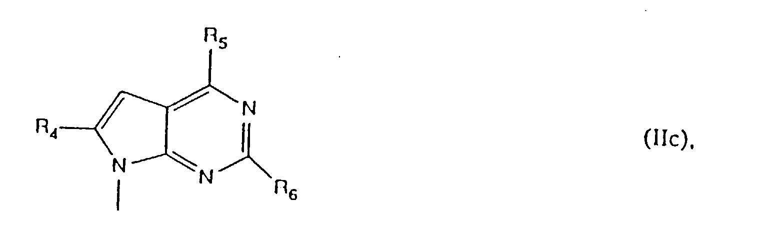 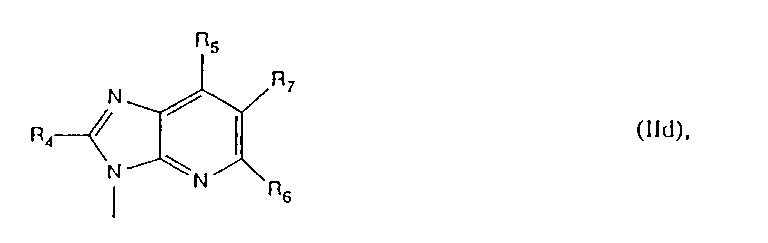 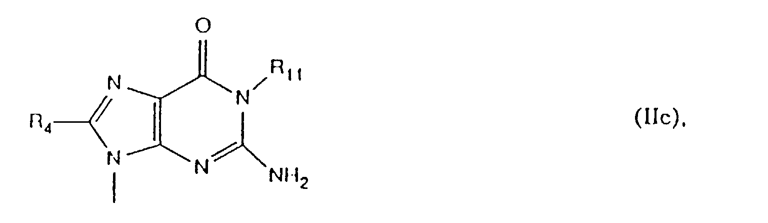 dans lesquelles R₄ représente H, Cl, Br ou OH, et R₅, R₆ et R₇, indépendamment les uns des autres, représentent H, OH, SH, NH₂, NHNH₂, NHOH, un groupe NHO-alkyle ayant de 1 à 12 atomes de carbone, F, Cl, Br, un groupe alkyle ou hydroxyalkyle ou aminoalkyle ou alcoxy ou alkylthio ayant de 1 à 12 atomes de carbone, les radicaux hydroxy- et amino étant non substitués ou substitués par un groupe protecteur, ou représentent un groupe phényle, benzyle, amino primaire ayant de 1 à 20 atomes de carbone ou amino secondaire ayant de 2 à 30 atomes de carbone, et R₁₁ représente H ou un groupe alkyle en C₁-C₄.

42. Oligonucléotides de formule VII selon la revendication 41, dans lesquels le groupe protecteur pour les groupes hydroxy et amino représente un groupe acyle en C₁-C₈.

43. Oligonucléotides de formule VII selon la revendication 41, dans lesquels le groupe amino primaire contient de 1 à 12 atomes de carbone et le groupe amino secondaire contient de 2 à 12 atomes de carbone.

44. Oligonucléotides de formule VII selon la revendication 41, dans lesquels, en ce qui concerne les groupes amino primaire et amino secondaire, il s'agit de radicaux de formule R₈R₉N, dans lesquels R₈ représente H ou a, indépendamment, la signification de R₉, et R₉ représente un groupe alkyle, aminoalkyle, hydroxyalkyle en C₁-C₂₀; un groupe carboxyalkyle ou carbalcoxyalkyle, le fragment carbalcoxy ayant de 2 à 8 atomes de carbone et le fragment alkyle ayant de 1 à 6, de préférence de 1 à 4 atomes de carbone; un groupe alcényle en C₂-C₂₀; un groupe phényle, mono- ou di-[alkyl- ou alcoxy(C₁-C₄)]-phényle, benzyle, mono- ou di-[alkyl- ou alcoxy(C₁-C₄))-benzyle; ou un groupe 1,2-, 1,3-ou 1,4-imidazolyl-alkyle(C₁-C₄) , ou R₈ et R₉ représentent ensemble un groupe tétra- ou pentaméthylène, 3-oxa-1,5-pentylène, -CH₂-NR₁₀-CH₂CH₂ ou -CH₂CH₂-NR₁₀-CH₂CH₂ -, R₁₀ représentant H ou un radical alkyle en C₁-C₄, le fragment amino dans le groupe aminoalkyle étant non substitué ou substitué par un ou deux radicaux alkyle ou hydroxyalkyle en C₁-C₄, et le radical hydroxy dans le groupe hydroxyalkyle étant éventuellement éthérifié par un groupe alkyle en C₁-C₄.

45. Oligonucléotides de formule VII selon la revendication 44, dans lesquels, en ce qui concerne les groupes amino primaire et amino secondaire, il s'agit des groupes méthyl-, éthyl-, diméthyl-, diéthyl-, allyl-, mono- ou di(1-hydroxy-éth-2-yl)-, phényl- et benzyl-, acétyl- et benzoylamino.

46. Oligonucléotides de formule VII selon la revendication 41, dans lesquels R₄ dans les formules II, IIb, IIc, IId et IIe représente un atome d'hydrogène.

47. Oligonucléotides de formule VII selon la revendication 41, dans lesquels R₇ dans la formule IId représente un atome d'hydrogène.

48. Oligonucléotides de formule VII selon la revendication 41, dans lesquels R₅ et R₆ dans les formules II, IIa, IIb, IIc, IId et IIe représentent, indépendamment l'un de l'autre, H, F, Cl, Br, OH, SH, NH₂, NHOH, NHNH₂, le groupe méthylamino, diméthylamino, benzoylamino, méthoxy, éthoxy ou méthylthio.

49. Oligonucléotides de formule VII selon la revendication 41, dans lesquels B est un reste de purine ou un reste d'un analogue de purine choisi parmi l'adénine, la N-méthyladénine, la N-benzoyladénine, la 2-méthyladénine, la 2-méthylthioadénine, la 2-aminoadénine, la 3-carbaadénine, la 7-carbaadénine, la 1-carbaadénine, la 6-hydroxypurine, la 2-amino-6-chloropurine, la 2-amino-6-méthylthiopurine, la 2-amino-6-hydroxypurine, la 3-carba-6-chloropurine, la guanine, la 2-méthylguanine.

50. Oligonucléotides de formule VII selon la revendication 35, dans lesquels B dans la formule VII, en tant que reste d'analogue de pyrimidine, représente un reste d'uracile, de thymine ou de cytosine de formules III, IIIa et IIIb, 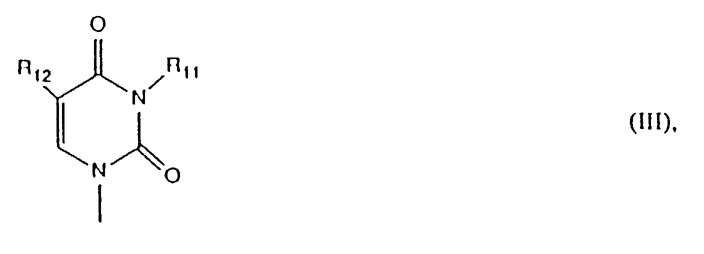 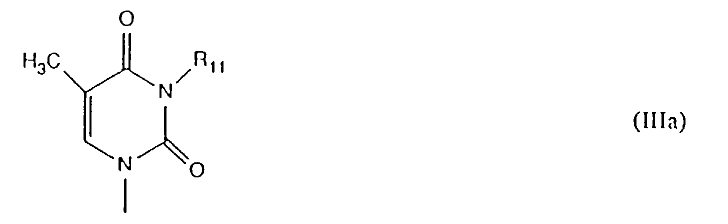 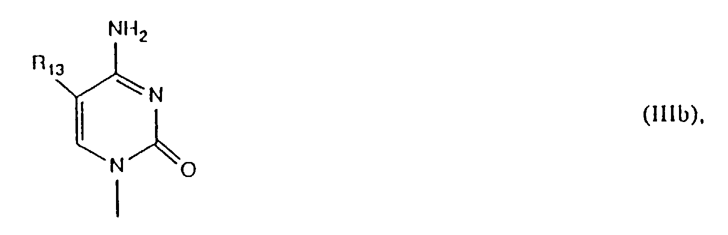 dans lesquelles R₁₁ représente H ou un groupe alkyle en C₁-C₄, et R₁₂ et R₁₃, indépendamment l'un de l'autre, représentent H, OH,SH, NH₂, NHNH₂, NHOH, un groupe NHO-alkyle ayant de 1 à 12 atomes de carbone, F, Cl, Br, un groupe alkyle ou hydroxyalkyle ou aminoalkyle ou alcoxy ou alkylthio ayant de 1 à 12 atomes de carbone, les radicaux hydroxy- et amino étant non substitués ou substitués par un groupe protecteur, ou représentent un groupe phényle, benzyle, amino primaire ayant de 1 à 20 atomes de carbone ou amino secondaire ayant de 2 à 30 atomes de carbone, et les atomes d'hydrogène du groupe NH₂ dans la formule IIIb sont non substitués ou substitués par un groupe alkyle en C₁-C₆, benzoyle ou benzyle, ainsi que les dérivés dihydro des restes de formules III, IIIa et IIIb.

51. Oligonucléotides formule VII selon la revendication 50, dans lesquels R₁₂ représente H ou un groupe alkyle ou hydroxyalkyle en C₁-C₆, F, Cl, Br, NH₂, ou un groupe benzoylamino, mono- ou dialkyl(C₁-C₆)amino.

52. Oligonucléotides de formule VII selon la revendication 50, dans lesquels R₁₃ représente H ou un groupe alkyle ou alcoxy ou hydroxyalkyle en C₁-C₆, F, Cl, Br, NH₂, ou un groupe benzoylamino, mono- ou dialkyl(C₁-C₆)amino.

53. Oligonucléotides de formule VII selon la revendication 51, dans lesquels R₁₂ représente H, F, Cl, Br, NH₂, NHCH₃, N(CH₃)₂ ou un groupe alkYle en C₁-C₄.

54. Oligonucléotides de formule VII selon la revendication 52, dans lesquels R₁₃ représente H ou un groupe alkyle en C₁-C₄, NH₂, NHCH₃ ou (CH₃)₂N.

55. Oligonucléotides de formule VII selon la revendication 50, dans lesquels B, en tant que reste d'un analogue de pyrimidine, dérive de l'uracile, la thymine, la cytosine, le 5-fluoro-uracile, le 5-chloro-uracile, le 5-bromo-uracile, le dihydro-uracile, le pseudo-uracile, le 1-méthylpseudo-uracile, le 5-méthyluracile, la 3-méthylcytosine et la 5-méthylcytosine.

56. Oligonucléotides de formule VII selon la revendication 35, caractérisés en ce qu'ils correspondent à la formule VIIa, 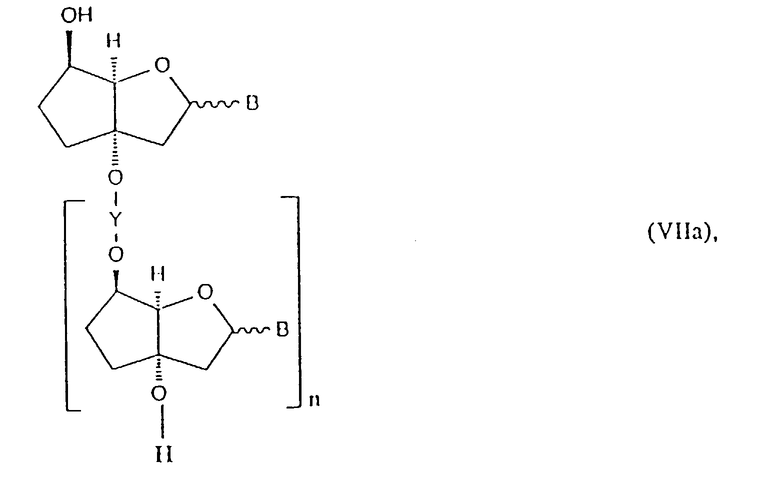 dans laquelle B, Y et n ont les significations données dans la revendication 35.

57. Oligonucléotides de formule VII selon la revendication 35, caractérisés en ce qu'ils correspondent à la formule VIIb, 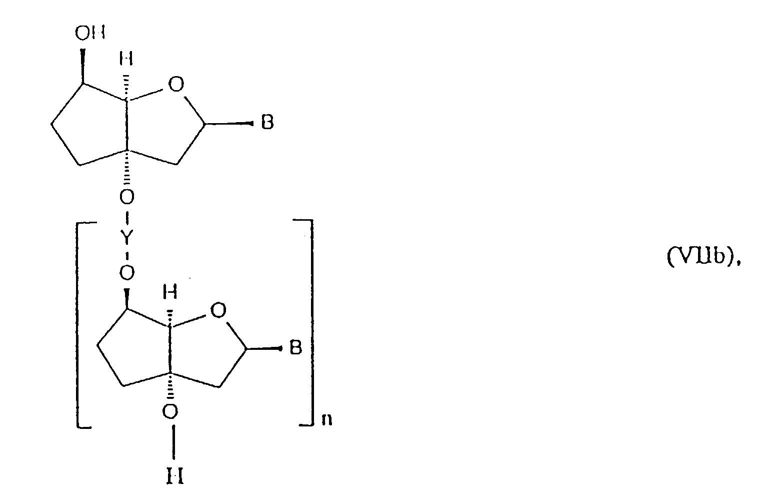 dans laquelle B, Y et n ont les significations données dans la revendication 35.

58. Oligonucléotides de formule VIIb selon la revendication 57, dans lesquels B représente le reste 9-adényle et n est égal à 10.

59. Oligonucléotides de formule VIIb selon la revendication 57, dans lesquels B représente le reste cytosyle et n est égal à 6.

60. Oligonucléotides de formule VIIb selon la revendication 57, dans lesquels B représente le reste thymidyle et n est égal à 10.

61. Utilisation des oligonucléotides de formule VII, VIIa ou VIIb, en tant qu'agents de diagnostic pour la détection d'infections virales ou de maladies d'origine génétique.

62. Nucléosides de formule I, IV ou VIa ou oligonucléotides de formule VII, VIIa ou VIIb, pour utilisation dans un procédé thérapeutique pour le traitement de maladies chez des animaux à sang chaud, l'homme y compris, par inactivation de séquences d'acide nucléique dans l'organisme.

63. Composition pharmaceutique, contenant une quantité efficace d'un nucléoside de formule I, IV ou VIa, ou d'un oligonucléotide de formule VII, VIIa ou VIIb, seuls ou conjointement avec d'autres substances actives, un véhicule pharmaceutique et éventuellement des adjuvants.

64. Procédé pour la préparation d'une composition pharmaceutique selon la revendication 63, caractérisé en ce que l'on ajoute un véhicule pharmaceutique et éventuellement des adjuvants à une quantité efficace d'un nucléoside de formule I, IV ou IVa selon l'une des revendications 1 à 23, ou d'un oligonucléotide de formule VII, VIIa ou VIIb selon l'une des revendications 35 à 60, seuls ou conjointement avec d'autres substances actives.

65. Procédé pour la préparation d'un oligonucléotide de formule VII, VIIa ou VIIb selon l'une des revendications 35 à 60, caractérisé en ce que l'on utilise dans la synthèse de l'oligonucléotide un composé de formule I, IV ou IVa selon l'une des revendications 1 à 23.

66. Procédé selon la revendication 65, caractérisé en ce que l'oligonucléotide comporte de 2 à 100 unités monomères.

67. Procédé selon la revendication 65, caractérisé en ce que l'oligonucléotide comporte de 2 à 50 unités monomères.

68. Procédé selon la revendication 65, caractérisé en ce que l'oligonucléotide comporte de 2 à 20 unités monomères.

69. Procédé pour la préparation d'un composé de formule Va selon la revendication 26 ou de formule VIa selon la revendication 29, caractérisé en ce qu'un composé de formule H ou de formule J ou le racémique 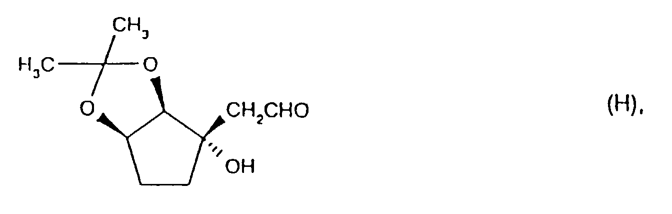 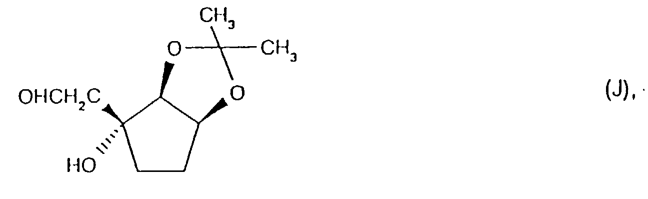 est hydrolysé et cyclisé en présence d'acides aqueux ou d'échangeurs d'ions acides en présence d'eau.

70. Procédé pour la préparation d'un composé de formule V selon l'une des revendications 26 à 28 ou de formule VI selon la revendication 29, caractérisé en ce que, dans un composé de formule Va selon la revendication 26 ou de formule VIa selon la revendication 29, on remplace le groupe hydroxy anomère par un groupe partant X, et on introduit les groupes protecteurs R₁₄ et R₁₅.
